(19) Europäisches Patentamt / European Patent Office / Office européen des brevets

(11) **EP 4 653 008 A1**

(12) **EUROPEAN PATENT APPLICATION**
published in accordance with Art. 153(4) EPC

(43) Date of publication:
26.11.2025　Bulletin 2025/48

(21) Application number: 24744420.1

(22) Date of filing: 24.01.2024

(51) International Patent Classification (IPC):
*A61K 36/9068* (2006.01)　　*A61P 37/02* (2006.01)
*A61P 31/16* (2006.01)　　*A61P 31/14* (2006.01)
*A61P 31/12* (2006.01)　　*A61P 29/00* (2006.01)
*A61P 11/02* (2006.01)　　*A61P 11/00* (2006.01)
*A61P 1/14* (2006.01)　　*A61P 1/12* (2006.01)
*A61P 1/08* (2006.01)

(52) Cooperative Patent Classification (CPC):
A61K 36/9068; A61K 47/69; A61P 1/00;
A61P 1/08; A61P 1/12; A61P 1/14; A61P 11/00;
A61P 11/02; A61P 29/00; A61P 31/12; A61P 31/14;
A61P 31/16; A61P 37/02; C11B 9/02

(86) International application number:
PCT/CN2024/073799

(87) International publication number:
WO 2024/153251 (25.07.2024 Gazette 2024/30)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority:　17.01.2023　CN 202310058976

(71) Applicant: Shijiazhuang Yiling Pharmaceutical
Co., Ltd.
Shijiazhuang, Hebei 050035 (CN)

(72) Inventors:
• WU, Yiling
Shijiazhuang, Hebei 050035 (CN)
• JIA, Zhenhua
Shijiazhuang, Hebei 050035 (CN)

(74) Representative: V.O.
P.O. Box 87930
2508 DH Den Haag (NL)

(54) **TRADITIONAL CHINESE MEDICINE COMPOSITION HAVING EFFICACY IN TREATING COLDS AND USE THEREOF**

(57)　A traditional Chinese medicine composition having efficacy in treating colds and a use thereof. The traditional Chinese medicine composition is prepared from perilla leaf, cabline potchouli, all-grass of haichow elsholtzia, bark of officinal magnolia, root of baikal skullcap, alum-processed pinellia ternata tuber, lonicera confusa, weeping forsythia capsule, Fu Ling, dried tangerine peel, and fresh ginger, and is capable of relieving exterior syndrome and harmonizing the middle, expelling uncleanliness and resolving dampness, and treating symptoms such as cold, fever, aversion to wind, stuffy and runny nose, headache and drowsiness, nausea and vomiting, diarrhea, heaviness in limbs, abdominal swelling and pain, decreased appetite and anorexia.

EP 4 653 008 A1

**Description**

This application claims the priority of Chinese Patent Application No. 202310058976.3, filed with the China National Intellectual Property Administration on January 17, 2023, and titled with "TRADITIONAL CHINESE MEDICINE COMPOSITION HAVING EFFICACY IN TREATING COLDS AND USE THEREOF", the disclosure of which is hereby incorporated by reference in its entirety.

**FIELD**

The present disclosure relates to the technical field of traditional Chinese medicine, in particular to a traditional Chinese medicine composition having efficacy in treating colds and use thereof.

**BACKGROUND**

As the most common acute respiratory tract infectious disease, colds manifest as symptoms such as nasal congestion, runny nose, headache, sneezing, fever, aversion to wind and cough, which are common triggers for diseases such as asthma and pneumonia, and can even lead to secondary bacterial infections, worsening of asthma, chronic obstructive pulmonary disease, and other serious complications. The symptoms of colds peak 2 to 3 days after infection in terms of severity. Patients usually recover after 5 to 7 days, but some symptoms may last up to 3 weeks or more. It has been reported that the number of people seeing a doctor each year due to colds reaches up to 2.7 billion. On average, adults suffer from 2 to 6 colds per year, and children suffer from 6 to 8 colds per year. The annual incidence rate among community residents in China is as high as 83.27%. According to data from the United States, the high incidence and frequency of colds have led to increased rates of absenteeism from work and school for population. Each year, the cost of over-the-counter medicines to relieve cold symptoms reaches $2 billion, and the cost of antimicrobial medicines is up to $2.27 billion. Further increases in medical costs due to the treatment of complications and worsening of the original disease aggravate the burden of disease treatment. Although the disease is common, its incidence and frequency are high and it is prone to cause severe clinical consequences, increasing the burden of disease. There is an urgent need for effective therapeutic drugs to improve clinical symptoms.

Common viruses that cause colds include rhinoviruses, coronaviruses, coxsackieviruses, parainfluenza viruses, respiratory syncytial viruses, and the like. Viruses replicate in the epithelial cells of the respiratory tract and in local lymphoid tissues, releasing various inflammatory mediators to induce an inflammation reaction that clinically manifests as a range of respiratory and systemic symptoms such as fever and pain. The symptoms of cold can vary slightly depending on the pathogenic virus. For example, coronaviruses are more likely to cause myalgia, headache, and general malaise, parainfluenza viruses and respiratory syncytial virus infections are likely to be manifested as fever, and coxsackieviruses and coronaviruses are also significant pathogens responsible for gastrointestinal symptoms. Viral replication in gastrointestinal tissues causes pathological changes such as degeneration and necrosis of mucosal cells, resulting in dysfunction of the digestive system, manifested by nausea, vomiting, abdominal distension, diarrhea, and the like. Among them, coxsackievirus infections are manifested as various symptoms of respiratory and digestive systems, including fever (97%), pharyngitis (85%), vomiting (56%), headache (49%), signs and symptoms of respiratory tract (44%), diarrhea (40%), and abdominal pain (33%). Researches show that, in addition to causing symptoms of cold, coronavirus infections also lead to vomiting, diarrhea, and abdominal pain in 57% of patients, and children are more likely than adults to experience gastrointestinal discomfort.

The above clinical syndrome caused by viral infection with symptoms of cold and digestive tract as the main manifestation is often commonly known as "gastrointestinal type cold" in China. ICD10 does not have a specific classification for this condition. There are currently no targeted treatments or uniform standards for efficacy evaluation. Clinical diagnosis is mainly based on symptoms of exogenous sensations and digestive tract combined with blood and stool routines. But it needs to be differentiated from viral gastroenteritis and the like. Viral gastroenteritis is primarily caused by viral infections affecting the intestinal tract, such as rotavirus, norovirus, enteric adenovirus, and astrovirus. The lesions are limited in location, with watery diarrhoea and abdominal pain as the initial or primary symptoms. Although both colds with gastrointestinal discomfort due to viral infections and viral gastroenteritis involve symptoms of digestive system, their pathogens are different and they are categorized as two different categories of diseases.

At present, the excessive use of antiviral drugs for colds has significantly increased the risk of related adverse reactions. Therefore, symptomatic treatments are mostly applied in clinical, for example, compound preparations having antipyretic-analgesic effect and effect of reducing nasal congestion and secretions such as acetaminophen and pseudoephedrine hydrochloride tablets, and decongestants having effect of alleviating nasal catarrh symptoms including nasal obstruction, rhinorrhea and the like. However, the above drugs have not paid enough attention to relieve the gastrointestinal symptoms associated with colds. Sometimes probiotics are given only to improve gastrointestinal function, montmorillonite powder and the like are given to stop diarrhea if it is severe, or drugs balancing water and electrolyte disorders (e.g. oral rehydration salts) are given to prevent dehydration. As can be seen from this, the clinical treatment usually uses the combination of multiple drugs targeting different symptoms, increasing the dosage, reducing patient compliance, and also raising safety risks in the combination use of drugs. Therefore, there is a need to develop comprehensive and effective therapeutic drugs for cold with symptoms of digestive system.

In traditional Chinese medicine, colds belong to the category of external contraction diseases. The earliest use of the term "cold" as a disease name is found in *Renzhai's Direct Guidance on Formulas* written by Yang Shiying from the Song Dynasty. Traditional Chinese medicine believes that colds are caused by external wind pathogen invading and affecting the Lung-Wei system. The *Diagnosis and Treatment Guidelines for Traditional Chinese Medicine on Common Cold* issued by Pulmonary Disease Branch of the China Association of Chinese Medicine in 2015 classifies this disease into: wind-cold pattern, wind-heat pattern, wind-dryness pattern, summerheat-dampness pattern, qi deficiency pattern and qi-yin deficiency pattern by pattern identification and syndrome differentiation. The *Expert Consensus on Diagnosis and Efficacy Evaluation for Gastrointestinal-Type Common Cold* issued by China Association of Traditional Chinese Medicine, Pulmonary Disease Branch of the China Association of Chinese Medicine, and Board of Specialty Committee of Digestive System Disease of World Federation of Chinese Medicine Societies classifies this disease into: cold due to external wind invasion with gastrointestinal disharmony and cold due to external summerheat-dampness invasion with gastrointestinal disharmony by pattern identification and syndrome differentiation. Therefore, wind pathogen is considered the primary causative factor of external contraction diseases, and dampness pathogen obstruction in the middle jiao is a critical contributor to gastrointestinal dysfunction. As early as the Spring and Autumn and Warring States periods, the *Yellow Emperor's Inner Canon* had categorized wind and dampness among six exogenous pathogenic factors. In the Eastern Han Dynasty, Zhang Zhongjing's *Treatise on Cold Damage and Miscellaneous Diseases* provided clear categorizations and documentation regarding external contraction diseases caused by wind stroke and dampness-caused diseases, in which "Taiyang wind stroke" and "dampness constitution predisposes to illness" was discussed in specific sections. These works laid the foundation for future generations to identify patterns involving external pathogens attacking the exterior and damaging the gastrointestinal system.

During the Jin and Tang dynasties, medical practitioners followed the theories in the *Inner Canon* and the *Treatise on Cold Damage and Miscellaneous Diseases,* and adopted the method of expelling pathogens through outward resolution for external contraction diseases due to both wind and dampness pathogens. In the Song, Jin, and Yuan dynasties, understanding of external contraction diseases due to wind and dampness pathogens deepened further. The *Taiping Holy Prescriptions for Universal Relief from the Pharmacy Service Bureau* compiled during the Song dynasty, includes the Huoxiang Zhengqi San, which remains a representative formula for treating external contraction diseases accompanied by nausea, vomiting, and diarrhea. By the Ming and Qing dynasties, Wu Jutong developed the Xin Jia Xiangru Yin based on the principle of combining pungent-warm and pungent-cool herbs, which has effects of releasing exterior heat stagnation and relieving interior dampness-turbidity, nausea and vomiting. Shi Shoutang developed the Huo Po Xia Ling Tang according to the principle that expelling pathogens via sweating is an external release, and expelling pathogens via urine/stool is also an external release, which can open the upper gate, establish branch pathway, and guide the downward movement of dampness to eliminate dampness and circulate qi, playing a role in clearing and draining dampness-heat from the upper, middle, and lower Sanjiao.

Thus it can be seen that colds accompanied by gastrointestinal discomfort can be treated according to the pattern of wind attacking the exterior and dampness obstruction in the middle jiao. Currently, Western medicine adopts drugs mainly targeting respiratory tract infections, offers only limited symptomatic treatment for gastrointestinal discomfort, leading to a poor efficacy. However, the traditional Chinese medicine has accumulated rich experience in diagnosing and treating such conditions and has developed a series of representative therapeutic formulas. Therefore, for concurrent respiratory and digestive disorders caused by impaired diffuse function of Lung-Wei and gastrointestinal disharmony due to wind and dampness invading the body, the development of a novel traditional Chinese medicine based on the diagnostic and therapeutic experiences of past medical practitioners may provide new approaches to treating this condition.

For colds, western medical treatment is mainly aimed at relieving respiratory and systemic symptoms due to viral infections, while paying less attention to possible symptoms of digestive tract, and mostly administering medications capable of improving intestinal function, stopping diarrhea, and correcting electrolyte disorders, which ignores the holistic concept of the disease. Therefore, the treatment effect on patients with cold, manifested as coexisting symptoms of respiratory and digestive systems, is not very satisfactory.

(1) For symptoms such as fever, sore throat and body aches due to cold, antipyretic and analgesic drugs are commonly used to reduce prostaglandin synthesis, so that peripheral vasodilatation, sweating and heat dissipation are achieved by the heat regulator center, the blocking impulses to nociceptive nerve endings is achieved, which plays an anti-inflammatory, antipyretic and analgesic effects. Representative drugs include acetaminophen, ibuprofen, etc. However, the overdose of acetaminophen carries the risk of liver damage, and ibuprofen has common adverse reactions of gastrointestinal system, including worsening of symptoms such as nausea, vomiting and indigestion due to irritation to gastrointestinal tract.

(2) For nasal congestion, runny nose and other common respiratory symptoms of colds, decongestant drugs are commonly used to relieve symptoms such as nasal congestion and runny nose due to cold by contracting the swollen nasal mucosa and sinus blood vessels of patients with colds. For example, pseudoephedrine, which selectively constricts blood vessels in the upper respiratory tract, is the most commonly used decongestant for patients with cold. However, other vasoconstrictor drugs such as ephedrine, if used in excess, can lead to side effects such as increased blood pressure and should be used with caution.

(3) For gastrointestinal dysfunction, probiotics drugs are commonly used to relieve gastrointestinal dysfunction symptoms caused by cold, such as loss of appetite and diarrhea. By repairing the damaged intestinal mucosa and eliminating inflammation, they promote the growth of intestinal probiotics and inhibit the harmful intestinal bacterial flora to restore the immune function and the normal physiological function of intestinal tract. Common drugs include triple viable probiotics containing streptococcus faecalis and clostridium butyricum and tetralogy of viable bifidobacterium, but their effect is limited to an adjunctive role.

(4) Antidiarrheal Drugs: Montmorillonite powder is mostly used for adults and children with acute and chronic diarrhea. Montmorillonite powder is a new type of mucosal protective agent for digestive tract and is in a form of particle powder. It can be evenly distributed on the gastrointestinal mucosal surface, has a certain fixed and inhibitory effect on intestinal viruses and toxins, and can improve the ability of the mucosal barrier to defense attack factors and mucosal repair ability by combining with mucus glycoproteins to promote rapid relief of symptoms, and reduce the occurrence of diarrhea. But a single drug treatment requires a long time to show the effect.

(5) For electrolyte disorders caused by dehydration, oral rehydration salts are used to regulate water, electrolyte and acid-base balance, and are suitable for mild or moderate dehydration caused by various kinds of diarrhea. For patients with diarrhea, the combination of conventional treatment as the basis with oral rehydration salts has synergistic effect on regulating the electrolyte balance of the body, which can stabilize the interior environment and promote recovery.

In summary, patients with cold identified as the pattern of wind attacking the exterior and dampness obstruction in the middle jiao are manifested as common respiratory and digestive tract discomforts in clinical, which have complex manifestations. Therefore, using a single antipyretic and analgesic drug fails to achieve satisfactory therapeutic effects. It is necessary to combine multiple drugs that have effects such as stopping diarrhea, correcting water and electrolyte disorders, and regulating intestinal function, leading to excessive and chaotic medication, ignoring the holistic concept of the disease treatment, and therefore leading to a poor patient compliance. These facts further indicate the obvious limitation of Western medicine in treating patients with cold identified as the pattern of wind attacking the exterior and dampness obstruction in the middle jiao. Therefore, there is an urgent need for a safe and effective traditional Chinese medicine with clinical characteristics, aiming at improving the treatment effect on colds with gastrointestinal discomfort.

## SUMMARY

The traditional Chinese medicine compositions of the present disclosure are obtained from three famous formulas, Huoxiang Zhengqi San documented in *Taiping Holy Prescriptions for Universal Relief from the Pharmacy Service Bureau* compiled during the Song dynasty, Huo Po Xia Ling Tang developed by Shoutang Shi during the Qin dynasty, and Xin Jia Xiangru Yin documented in *Detailed Analysis of Epidemic Warm Diseases* from Jutong Wu as an expert for warm disease, by changes of addition or subtraction of formulas. This was developed, according to pulmonary diseases researches under the guidance of Collateral Disease Theory, by Professor Yiling Wu (Academician of the Chinese Academy of Engineering) as the director of the National Key Laboratory of Collateral Disease Research and Innovative Chinese Medicine, through combining the following concepts: pathogenic factors typically follow the spatial progression pattern of Luo diseases (collateral disorders), entering the interior from the exterior through the exterior (superficial Yang collaterals)-middle (meridians)-interior (Yin collaterals between zang-organ and fu-organ) pathway, the lung and large intestine are interior-exteriorly related through their meridians, qi circulates in the lung, stomach, and large intestine meridians, and pathogenic invasion of the lung collaterals often affects functions of spleen, stomoach and intestine. The traditional Chinese medicine compositions of the present disclosure are useful in treating patients with cold identified as the pattern of wind attacking the exterior and dampness obstruction in the middle jiao. For this disease, proposed is that the invasion of exogenous wind pathogen and dampness is the root cause, and the disharmony of the spleen-stomach-intestine system is the core issue. Specifically, wind pathogens invade the Wei-defense and exterior, and dampness pathogens attached to wind pathogens permeate both the interior and exterior, causing dysfunction of lung collaterals. Their combined action obstructs qi movement and clear yang and further results in dysfunction of the spleen-stomach-intestine system. The core of treatment lies in dispersing and releasing wind, transforming dampness and repelling filth in combination of reinforcing the central body. Based on this, the treatment method of releasing the exterior, harmonizing the middle jiao, transforming dampness and repelling filth is established. For diseases caused by wind and dampness pathogens, pungent-aromatic herbs are used to release the exterior, exclude pathogens, harmonize the middle jiao and transform dampness. Also, light-clearing herbs are used to release the exterior skin of the body and expelling heat, and combined with qi-regulating, spleen-strengthening, dampness-drying and dampness-draining herbs to enhance the efficacy of harmonizing the middle jiao and transforming dampness. They can effectively alleviate symptoms such as fever, aversion to wind, nasal congestion, runny nose, headache and dizziness, nausea and vomiting, diarrhea, limb fatigue and heaviness, abdominal distension and pain, low food intake and poor appetite.

The formulation of the traditional Chinese medicine composition of the present invention integrates the formula formation perspective of using pungent, warm, aromatic, warm-drying and middle jiao-harmonizing herbs for dispersing superficial exterior pathogens, drying and resolving dampness and turbidity accumulated in the interior according to the Huoxiang Zhengqi San and Huo Po Xia Ling Tang, selects *Perillae folium, Pogostemonis cablin, Citri reticulatae pericarpium, Pinelliae rhizoma praeparatum cum alumine, Magnoliae officinalis cortex, Poria,* and *Zingiberis rhizoma recens* against wind and dampness pathogens, adopts *Moslae herba, Lonicerae flos,* and *Forsythiae fructus* documented in Xin Jia Xiangru Yin according to its formula formation perspective of combing pungent-warm and pungent-cold herbs, and incorporates *Scutellariae radix.* In the formula, *Perillae folium* is to release the exterior, dispel pathogens, regulate qi, and harmonize the middle jiao. *Pogostemonis cablin* is pungent-warm to disperse and releasing superficial pathogens, and is aromatic to repel filth, transform dampness and harmonize the middle jiao. *Moslae herba* has aromatic smell, cold taste, light and ascending characteristics. Its upward actions include dispersing Lung-qi to skin; and its downward actions include unblocking Sanjiao, inducing urination and draining dampness. *Pinelliae rhizoma praeparatum cum alumine* and *Magnoliae officinalis cortex* are used together to dry dampness, relieve fullness, and descend qi in the middle jiao, achieving effects of regulating qi movement, harmonizing the middle jiao, and arresting vomiting. *Citri reticulatae pericarpium* and *Poria* are used together to enhance the effect of transforming dampness and draining dampness and arresting vomiting and diarrhea, while strengthening the spleen and stomach to reinforce healthy qi in the middle jiao. *Zingiberis rhizoma recens* is included in the original formula of Huoxiang Zhengqi San to harmonize the spleen and stomach and arrest vomiting, serving in an assistant role. The combination of pungent-warm aromatic herbs with bitter-drying substances in this formula can externally release superficial pathogens, and transform interior dampness-turbidity, thereby unblocking qi movement, harmonizing the spleen and stomach, and naturally resolving both exterior and interior syndromes. Considering the tendency of wind and dampness to transform into heat and transmit, *Lonicerae flos* and *Forsythiae fructus* are used as a mutual reinforcement combination to clear and release heat from lung-meridian and Wei-defense, and expel pathogens to the exterior of the surface. Despite they are heat-clearing herbs, their light-ascending properties avoids the excessive bitter-cold actions that might impair spleen-stomach qi. *Scutellariae radix,* which is good at clearing dampness-heat in the upper and middle jiao, clears and releases dampness-turbidity and stagnant heat accumulated in the interior. Therefore, the formula combining herbs documented in three famous formulas by addition or subtraction can not only disperse superficial pathogens, but also harmonize the middle jiao, repel filth, and dry and transform dampness-turbidity accumulated in the interior to clear yang and normalize qi movement, thereby stabilizing the body's equilibrium.

*Perillae folium* and *Pogostemonis cablin* are used as sovereign medicines in the traditional Chinese medicine composition of the present disclosure.

*Perillae folium* is pungent and warm, and enters lung and spleen meridians. Its effects are to release the exterior, disperse pathogens, circulate qi and harmonize the middle jiao. This herb has properties of pungent, aromatic, light, ascending, dispersing, and potent fragrance. It can open the exterior skin and disperse lung qi to unblock the interstices by unblocking the upper jiao. For diseases caused by wind-dampness pathogens, it is not limited to the use of sweat-provoking and exterior-releasing herbs, the use of aromatic-dispersing herbs follows the principle of treating the upper jiao as a feather by no ascending no light herbs. In addition, this herb enters the spleen meridian and passes through the middle jiao, where it opens the chest and diaphragm in the middle, awakens the spleen and stomach, dissolve phlegmatic retention, release stagnation and unblock qi, thereby alleviating gastrointestinal symptoms such as anorexia, nausea and vomiting.

*Pogostemonis cablin* is pungent, slightly warm, and enters spleen, stomach, and lung meridians. Its effects are to transform dampness with its aromatic properties, repel filth, and harmonize the middle jiao. This herb is light-ascending and warm-dispersing. Its pungent-warm properties regulate qi through the lung meridian, and harmonize the middle jiao after entering spleen and stomach to reinforce Wei-qi and transform dampness with its aromatic properties, leading to unblocking the qi movement in the middle jiao. *Pogostemonis cablin* can open the middle jiao, and nourish lung collaterals to gradually fortify healthy qi. Two pungent and aromatic herbs, *Perillae folium* and *Pogostemonis cablin,* are used as sovereign medicines in this formula to play a role in dispersing externally contracted pathogens, overcoming dampness and repelling filth.

*Moslae herba, Magnoliae officinalis cortex, Scutellariae radix,* and *Pinelliae rhizoma praeparatum cum alumine* are used as minister medicines.

*Moslae herba* is pungent and lightly warm, and enters lung and stomach meridians. Its effects are to release the exterior, harmonize the middle jiao, and disperse and transform dampness pathogens. For the treatment of externally contracted diseases caused by wind and dampness pathogens, utilized is its role in inducing sweating and promoting urination. This herb is pungent-warm and aromatic. Its pungent-warm induces sweating to release superficial pathogens by penetrating muscular interstices and drains dampness retention by promoting urination, leading to an effect of alleviating the exterior and interior symptoms. It is combined with *Perillae folium* as sovereign medicine to enhance the effect of releasing the exterior and transforming dampness, and is compatible with *Pogostemonis cablin* to enhance the effect of harmonizing the middle jiao and draining dampness.

*Magnoliae officinalis cortex* is bitter, pungent, warm, and enters the spleen, stomach, lung, and large intestine meridians. Its effects are to move qi, transform dampness, unblock the middle jiao, and relieve fullness. This herb, as middle jiao-soothing, stagnation-dispersing, and stomach qi-stabilizing medicine, is capability of drying dampness with its warm property, clearing phlegm with its pungent property, and descending qi with its bitter property. For obstructed qi movement and the inability of clear yang to ascend due to dampness-turbidity, it plays a role in promoting digestion, transforming phlegm, removing dampness, dispersing distention, soothing organs that pertain to earth and metal. Combined with sovereign medicines and minister medicines, this herb circulates qi, stops vomiting, and unblocks qi movement among pungent-warm herbs.

*Scutellariae radix* is bitter and cold, and enters the lung, gallbladder, spleen, large intestine and small intestine meridians. Its effects are to clear heat and dry dampness. *Scutellariae radix* is particularly effective in clearing dampness-heat from the upper jiao and middle jiao, excelling in treating conditions where pathogens stagnate and transform into heat over time. Wind-dampness pathogens firstly invade the exterior. If not promptly resolved, they impair gastrointestinal reception and transmission functions, resulting in symptoms such as nausea, vomiting, acid reflux, diarrhea, and borborygmus. For the pattern due to dampness-turbidity accumulated in the interior transforming into heat, *Scutellariae radix* is essential for opening and resolving stagnation. In the formula, the addition of the cold herb into various pungent-warm herbs also follows Jutong Wu's principle of combining pungent-warm with pungent-cool herbs in formula.

*Pinelliae rhizoma praeparatum cum alumine* is pungent and warm, and enters the spleen, stomach and lung meridians. Its effects are to dry dampness, harmonize the stomach, descend counterflow and stop vomiting. It can strengthen and tonify the spleen, dry dampness and transport water, and is especially effective in drying and transforming dampness-turbidity in the middle jiao. It acts to open the appetite, fortify the spleen, stop vomiting, and eliminate phlegm fullness in the chest. It utilizes its pungent-warm and diffusing properties to unblock and regulate qi movement.

The aforementioned four herbs are used as minister herbs. Among them, *Moslae herba* with its pungent-warm and aromatic properties, can disperse and transform exterior pathogens to resolve exterior syndromes. The combination of *Moslae herba* with *Magnoliae officinalis cortex* can dry and eliminate dampness-turbidity in the middle jiao to treat vomiting and diarrhea, and paired with *Scutellariae radix* with strong heat-clearing property, they can warm and disperse dampness-turbidity and clear stagnant heat. *Pinelliae rhizoma praeparatum cum alumine* is pungent and warm, and enters the lung and stomach. It can descend counterflow and harmonize the stomach. *Magnoliae officinalis cortex* is bitter, pungent and warm, and can dry dampness, relieve fullness, regulate qi and unblock the middle jiao. The combination of the two can enhance effects of unblocking and descending qi movement, harmonizing the middle jiao and stopping vomiting.

The assistant herbs include *Lonicerae flos, Forsythiae fructus, Poria* and *Citri reticulatae pericarpium.*

*Lonicerae flos* is sweet and cold, and enters the lung, heart, and stomach meridians. Its effects are to clear and release wind pathogens, and expelling pathogens to the exterior. *Lonicerae flos* and *Lonicerae japonicae flos* are recorded to be the same substance in historical documents. In the 2020 edition of the Chinese Pharmacopoeia, the two are described identically in terms of their properties including flavor, meridian affinity, functions, indications, administration, and dosage, indicating that they share pharmacological properties and medicinal characteristics. This herb is effective in dispersing the external pathogens to the exterior. Combined with *Forsythiae fructus, Forsythiae fructus* and *Lonicerae flos* are used with mutual reinforcement to clear heat stagnation in the lungs and release heat pathogens by guiding them to the surface of muscle without hindering the qi movement in the middle jiao.

*Forsythiae fructus* is bitter and slightly cold, and enters the lung, heart, small intestine meridians. Its effects are to release the exterior and clear the stagnant heat in the interior. It disperses and expels pathogens through the upper jiao with its light-ascending property, prevents external pathogens from transforming into heat inside the body with its slightly cold property, while avoiding excessive cold that might inhibit clear yang. Combined with *Scutellariae radix,* they can release the interior heat and expel heat pathogens outward from the interior.

*Poria* is sweet, bland and neutral, and enters the heart, lung, spleen and kidney meridians. Its effects are to strengthen the spleen, harmonize the middle jiao, drain dampness and stop diarrhea. It can alleviate symptoms related to spleen and stomach such as nausea, vomiting and the loss of appetite caused by dampness-turbidity stagnated in the interior. *Poria* has moderate properties, and can tonify without causing stagnation and drain without being harsh. This herb can reinforce healthy qi and eliminate pathogenic factors to reinforce the deficiency of the middle jiao and enhance the effect of draining dampness.

*Citri reticulatae pericarpium* is bitter, pungent and warm, and enters the lung and spleen meridians. Its functions include regulating qi, strengthening the spleen, drying dampness, and stopping vomiting. It is a good herb for treating disruption of qi's ascending and descending movement caused by stagnant heat in the lung and spleen. For digestive symptoms such as nausea, loss of appetite and diarrhea caused by impaired transformation and transportation functions of the spleen and stomach due to dampness stagnation, *Citri reticulatae pericarpium* can also descend qi and stop vomiting. Its bitter property enables draining and drying, pungent property enables dispersing, and warm property enables harmonizing. Therefore, it is useful in various diseases. Its function of regulating qi and drying dampness is often utilized for tonifying in combination with tonifying herbs, reducing in combination with reducing herbs, ascending in combination with ascending herbs, and descending in combination with descending herbs. The spleen is considered the mother of yuan-primordial qi, and the lung governs the intake of qi. Thus, *Citri reticulatae pericarpium* acts on qi movement in the two meridians and plays its function of tonifying, reducing, ascending or descending according to the herbs it is paired with.

The aforementioned four herbs are used as assistant herbs. *Lonicerae flos* is sweet and cold and has an aromatic flavor. It can clear pathogens and expel them to the exterior. *Forsythiae fructus* is bitter and slightly cold, and it can clear the heat in the interior. The pungent-cool flavor of *Forsythiae fructus* and *Lonicerae flos* reaches the exterior of the lung meridian, acting only exteriorly without affecting the middle jiao, and thus they are often used. *Forsythiae fructus* and *Lonicerae flos* are used with mutual reinforcement to expel the heat and release the exterior and also clear the interior heart by descending and dispersing with its light property. *Citri reticulatae pericarpium* is combined with *Magnoliae officinalis cortex* to enhance the effect of regulating qi and harmonizing the middle jiao, is combined with *Pinelliae rhizoma praeparatum cum alumine* to play a role in harmonizing the middle jiao and stopping vomiting, and is combined with *Poria* to promote transforming dampness and stopping diarrhea while reinforcing the healthy qi in the middle jiao.

*Zingiberis rhizoma recens* is used as the guide herb.

*Zingiberis rhizoma recens* is pungent and slightly warm, and enters the lung, spleen and stomach meridians. Its effects are to release the exterior, disperse pathogens, stop vomiting and harmonize the middle jiao. *Zingiberis rhizoma recens* can remove dampness, warm the middle jiao, and tonify the spleen and stomach. If spleen and stomach have healthy qi with a mild movement, dampness is naturally eliminated. *Zingiberis rhizoma recens* as the guide herb works in harmony with the entire formula.

This formula prominently employs pungent aromatic herbs to disperse pathogens, release the exterior, transform dampness, and harmonize the middle jiao. The formula can reinforce central qi, and prevent lingering turbidity pathogens from spreading and transforming. Due to its pungent flavor, with its mobile and dispersing nature, it is particularly effective in dispersing pathogens and promoting qi movement. As Tianshi Ye said "freeing meridians with pungent", the extensive use of pungent-aromatic herbs in this formula can smooth the Qi movement in the lung collaterals and support the recovery of lung function. Bitter-flavored herbs are supplemented to enhance effects of drying and transforming damp-turbidity. The combination of pungent-warm exterior-releasing herbs with pungent-cool heat-clearing herbs not only disperses dampness but also clears interior stagnant heat, achieving a dual effect of exterior dispersion and interior clearance without excessive dispersion that could lead to complications. The entire formula works to expel pathogens from the body surface and the lung-defense while resolving dampness-turbidity accumulated in the middle and lower jiao. For wind-dampness pathogens, it employs warming and dispersing to resolve them at the exterior, meanwhile, by entering the interior, it either expels pathogens from the interior to exterior or guide and drain pathogens downward. The formula comprehensively addresses both exterior and interior aspects, uses warm and cool herbs, supports healthy qi, and expels pathogens, and thereby achieving the effects of releasing the exterior, harmonizing the middle jiao, repelling faith, and transforming dampness.

The entire formula combines both dispersing and draining actions while harmonizing the middle jiao and repelling faith. The combination of various herbs is used to remove exterior and interior pathogens, by releasing the exterior for pathogens in the upper jiao (Lung-Wei), and by drying, transforming and draining for pathogens in the middle and lower jiao (gastrointestinal tract). This prescription comes from renowned traditional Chinese medicine formulas, and is designed to have a high degree of coherence among theory, methodology, formula, and herb, ensuring logical alignment with the disease's etiology, pathogenesis, therapeutic principles, formula-designing and herb-selecting, making the prescription rational. Its effects are to release the exterior, harmonize the middle jiao, repel faith and transform dampness. It is a traditional Chinese medicine preparation developed on the basis of clinical experience, and has specific efficacy and safety, filling a gap in the treatment of colds primarily caused by wind-dampness pathogens, an area where existing Chinese patent herbal medicines have been lacking.

Pharmacological studies suggest that the pharmaceutical composition of the present invention has a significant inhibitory effect on coxsackievirus B3 (CVB3), influenza A virus H3N2, influenza B virus (IBV), respiratory syncytial virus (RSV), enterovirus EV71, coronavirus HCoV-229E, parainfluenza virus (PIV), rotavirus (RV) *in vitro.* It also has the effects of regulating the balance of immune cells and reducing the inflammation reaction of local tissues. The pharmaceutical composition of the present invention also has obvious inhibition effect on gastric emptying and intestinal motility, antidiarrhoeal, antipyretic, analgesic, anti-inflammatory effects, and can relieve virus-caused clinical symptoms.

The clinical study in a small scale launched by the inventor including 90 patients with colds (identified as the pattern of wind attacking the exterior and dampness obstruction in the middle jiao) suggests that the clinical recovery rate of the patients treated with the pharmaceutical compositions of the present invention is significantly higher compared to the control group, and shows good efficacy trends in shortening the course of the disease and relieving symptoms. In summary, the pharmaceutical composition of the present invention can produce a good therapeutic benefit for patients with colds (identified as the pattern of wind attacking the exterior and dampness obstruction in the middle jiao) in clinical trials.

In order to achieve the above inventive purpose, the present invention adopts the following technical solutions.

Provided is a traditional Chinese medicine composition for use in the treatment of a cold, comprising the following components in parts by weight: 230 to 340 parts of *Perillae folium,* 230 to 340 parts of *Pogostemonis cablin,* 230 to 340 parts of *Moslae herba,* 230 to 340 parts of *Magnoliae officinalis cortex,* 230 to 340 parts of *Scutellariae radix,* 170 to 260 parts of *Pinelliae rhizoma praeparatum cum alumine,* 285 to 430 parts of *Lonicerae flos,* 230 to 340 parts of *Forsythiae fructus,* 230 to 340 parts of *Poria,* 230 to 340 parts of *Citri reticulatae pericarpium,* and 115 to 170 parts of *Zingiberis rhizoma recens.*

Further, the traditional Chinese medicine composition for use in the treatment of a cold comprises the following components in parts by weight: 230 parts of *Perillae folium,* 340 parts of *Pogostemonis cablin,* 230 parts of *Moslae herba,* 340 parts of *Magnoliae officinalis cortex,* 230 parts of *Scutellariae radix,* 260 parts of *Pinelliae rhizoma praeparatum cum alumine,* 285 parts of *Lonicerae flos,* 340 parts of *Forsythiae fructus,* 230 parts of *Poria,* 340 parts of *Citri reticulatae pericarpium,* and 115 parts of *Zingiberis rhizoma recens.*

Further, the traditional Chinese medicine composition for use in the treatment of a cold comprises the following components in parts by weight: 340 parts of *Perillae folium,* 230 parts of *Pogostemonis cablin,* 340 parts of *Moslae herba,* 230 parts of *Magnoliae officinalis cortex,* 340 parts of *Scutellariae radix,* 170 parts of *Pinelliae rhizoma praeparatum cum alumine,* 430 parts of *Lonicerae flos,* 230 parts of *Forsythiae fructus,* 340 parts of *Poria,* 230 parts of *Citri reticulatae pericarpium,* and 170 parts of *Zingiberis rhizoma recens.*

Further, the traditional Chinese medicine composition for use in the treatment of a cold comprises the following components in parts by weight: 300 parts of *Perillae folium,* 300 parts of *Pogostemonis cablin,* 300 parts of *Moslae herba,* 300 parts of *Magnoliae officinalis cortex,* 300 parts of *Scutellariae radix,* 230 parts of *Pinelliae rhizoma praeparatum cum alumine,* 380 parts of *Lonicerae flos,* 300 parts of *Forsythiae fructus,* 300 parts of *Poria,* 300 parts of *Citri reticulatae pericarpium,* and 150 parts of *Zingiberis rhizoma recens.*

Further, the traditional Chinese medicine composition for use in the treatment of a cold comprises the following components in parts by weight: 286 parts of *Perillae folium,* 286 parts of *Pogostemonis cablin,* 286 parts of *Moslae herba,* 286 parts of *Magnoliae officinalis cortex,* 286 parts of *Scutellariae radix,* 214 parts of *Pinelliae rhizoma praeparatum cum alumine,* 357 parts of *Lonicerae flos,* 286 parts of *Forsythiae fructus,* 286 parts of *Poria,* 286 parts of *Citri reticulatae pericarpium,* and 143 parts of *Zingiberis rhizoma recens.*

Further, the traditional Chinese medicine composition for use in the treatment of a cold comprises the following components in parts by weight: 250 parts of *Perillae folium,* 250 parts of *Pogostemonis cablin,* 250 parts of *Moslae herba,* 250 parts of *Magnoliae officinalis cortex,* 250 parts of *Scutellariae radix,* 200 parts of *Pinelliae rhizoma praeparatum cum alumine,* 320 parts of *Lonicerae flos,* 250 parts of *Forsythiae fructus,* 250 parts of *Poria,* 250 parts of *Citri reticulatae pericarpium,* and 125 parts of *Zingiberis rhizoma recens.*

Further, the traditional Chinese medicine composition of the present invention is useful in treating at least one of influenza, common cold or gastrointestinal type cold.

The present disclosure provides use of the traditional Chinese medicine composition above in the manufacture of a medicament against H3N2, IBV, RSV, CVB3, EV71, HCoV-229E, PIV or RV virus.

The present disclosure provides use of the traditional Chinese medicine composition above in the manufacture of a medicament for inhibiting replication of coxsackievirus B3 in large intestine tissues, regulating balance of immune cells, and reducing inflammation reaction of local tissues.

The present disclosure provides use of the traditional Chinese medicine composition above in the manufacture of a medicament for reducing degree of colonic lesions, increasing ratio of lymphocytes CD4+/CD8+ in blood, decreasing level of TNF-$\alpha$ and elevating level of IFN-$\gamma$ in large intestine tissues of model animals.

The present disclosure provides use of the traditional Chinese medicine composition above in the manufacture of a medicament for inhibiting gastric emptying and intestinal motility effect.

The present disclosure provides use of the traditional Chinese medicine composition above in the manufacture of a medicament for treating diarrhea.

The present disclosure provides use of the traditional Chinese medicine composition above in the manufacture of a medicament for relieving pain.

The present disclosure provides use of the traditional Chinese medicine composition above in the manufacture of a medicament against inflammation.

Further, the traditional Chinese medicine composition of the present invention is useful in treating a gastrointestinal type cold manifested as fever with aversion to wind, headache and dizziness, nausea and vomiting, diarrhea, nasal congestion and runny nose, limb fatigue and heaviness, abdominal distention and pain, or low food intake and poor appetite.

Further, provided is a method for producing the traditional Chinese medicine composition of the present invention, comprising producing an active ingredient of the traditional Chinese medicine composition by steps of:

(1) weighing *Perillae folium, Pogostemonis cablin, Moslae herba, Citri reticulatae pericarpium*, and *Zingiberis rhizoma recens* according to a prescribed amount, extracting a volatile oil via distillation by water vapor, collecting the volatile oil for later use, and filtering an aqueous solution obtained after the distillation for later use;

(2) weighing *Pogostemonis cablin, Scutellariae radix, Pinelliae rhizoma praeparatum cum alumine, Lonicerae flos, Forsythiae fructus,* and *Poria* according to a prescribed amount, adding water, extracting, filtering to obtain a filtrate, concentrating the filtrate and the aqueous solution obtained after the distillation of step (1) under reduced pressure, performing spray drying, and collecting spray powder for later use;

wherein the active ingredient of the traditional Chinese medicine composition comprises the volatile oil obtained in step (1) and the extract obtained in step (2).

Further, the present invention provides a medicament comprising the aforementioned traditional Chinese medicine composition or the traditional Chinese medicine composition produced by the aforementioned method.

The medicament of the present invention is in a dosage form of capsule, tablet, granule, injection, pill, powder or oral liquid.

In order to realize the above dosage forms, pharmaceutically acceptable excipients should be added when preparing these dosage forms, such as fillers, disintegrants, lubricants, suspending agents, binders, sweeteners, flavoring agents, preservatives, bases, and the like. The fillers include starch, pregelatinized starch, lactose, mannitol, chitin, microcrystalline cellulose, sucrose, and the like. The disintegrants include starch, pregelatinized starch, microcrystalline cellulose, sodium carboxymethyl starch, cross-linked polyvinylpyrrolidone, low-substituted hydroxypropyl cellulose, cross-linked sodium carboxymethyl cellulose and the like. The lubricants include magnesium stearate, sodium lauryl sulfate, talc, silicon dioxide and the like. The suspending agents include polyvinylpyrrolidone, microcrystalline cellulose, sucrose, agar, hydroxypropyl methylcellulose and the like. The binders include starch mucilage, polyvinylpyrrolidone, hydroxypropyl methylcellulose and the like. The sweeteners include sodium saccharin, aspartame, sucrose, sodium cyclamate, glycyrrhetinic acid and the like. The flavoring agents include sweeteners and various flavors. The preservatives include parabens, benzoic acid, sodium benzoate, sorbic acid and salts thereof, benzalkonium bromide, chlorhexidine acetate, eucalyptus oil and the like. The bases include PEG6000, PEG4000, insect wax and the like.

Further, the granule of the traditional Chinese medicine composition of the present invention is produced by steps of:

(1) weighing *Perillae folium, Pogostemonis cablin, Moslae herba, Citri reticulatae pericarpium*, and *Zingiberis rhizoma recens* according to a prescribed amount, extracting a volatile oil via distillation by water vapor, collecting the volatile oil for later use, and filtering an aqueous solution obtained after the distillation to obtain a volatile oil extract for later use;

(2) weighing *Pogostemonis cablin, Scutellariae radix, Pinelliae rhizoma praeparatum cum alumine, Lonicerae flos, Forsythiae fructus,* and *Poria* according to a prescribed amount, adding water, decocting, filtering to obtain a filtrate, combining the filtrate and the volatile oil extract obtained in step (1), concentrating, filtering and drying to obtain dry paste powder for later use;

(3) encapsulating the volatile oil obtained in step (1) in betacyclodextrin to obtain a volatile oil inclusion substance; and

(4) adding an appropriate amount of an excipient to the dry paste powder obtained in step (2) and the volatile oil inclusion substance obtained in step (3) for granulation, and producing the granule according to a conventional method.

Further, the granule of the traditional Chinese medicine composition of the present invention is produced by the following method:

(1) weighing *Perillae folium, Pogostemonis cablin, Moslae herba, Citri reticulatae pericarpium,* and *Zingiberis rhizoma recens* according to a prescribed amount, adding 8 to 12 times the amount of water, extracting a volatile oil via distillation by water vapor for 6 to 10 h, collecting the volatile oil for later use, and filtering an aqueous solution obtained after the distillation to obtain a volatile oil extract for later use;

(2) weighing *Pogostemonis cablin, Scutellariae radix, Pinelliae rhizoma praeparatum cum alumine, Lonicerae flos,*

*Forsythiae fructus,* and *Poria* according to a prescribed amount, adding 8 to 12 times the amount of water, decocting twice for 1 to 2 h each time, filtering to obtain a filtrate, combining the filtrate and the volatile oil extract obtained in step (1), concentrating under reduced pressure, and performing spray drying to obtain dry paste powder for later use;

(3) encapsulating the volatile oil obtained in step (1) in betacyclodextrin to obtain a volatile oil inclusion substance, drying, and crushing to obtain dry powder for later use; and

(4) subjecting the dry paste powder obtained in step (2) and the dry powder of the volatile oil inclusion substance obtained in step (3) to dry granulation, and producing the granule according to a conventional method.

Further, the granule of the traditional Chinese medicine composition of the present invention is produced by the following method:

(1) weighing *Perillae folium, Pogostemonis cablin, Moslae herba, Citri reticulatae pericarpium,* and *Zingiberis rhizoma recens* according to a prescribed amount, adding 10 times the amount of water, extracting a volatile oil via distillation by water vapor for 8 h, collecting the volatile oil for later use, and filtering an aqueous solution obtained after the distillation to obtain a volatile oil extract for later use;

(2) weighing *Pogostemonis cablin, Scutellariae radix, Pinelliae rhizoma praeparatum cum alumine, Lonicerae flos, Forsythiae fructus,* and *Poria* according to a prescribed amount, adding 10 times the amount of water, decocting twice for 1.5 h each time, filtering to obtain a filtrate, combining the filtrate and the volatile oil extract obtained in step (1), concentrating under reduced pressure, and performing spray drying to obtain dry paste powder for later use;

(3) encapsulating the volatile oil obtained in step (1) in betacyclodextrin to obtain a volatile oil inclusion substance, drying, and crushing to obtain dry powder for later use; and

(4) subjecting the dry paste powder obtained in step (2) and the dry powder of the volatile oil inclusion substance obtained in step (3) to dry granulation, and producing the granule according to a conventional method.

Further, the tablet of the traditional Chinese medicine composition of the present invention is produced by steps of:

(1) weighing *Perillae folium, Pogostemonis cablin, Moslae herba, Citri reticulatae pericarpium,* and *Zingiberis rhizoma recens* according to a prescribed amount, adding 10 times the amount of water, extracting a volatile oil via distillation by water vapor for 8 h, collecting the volatile oil for later use, and filtering an aqueous solution obtained after the distillation to obtain a volatile oil extract for later use;

(2) weighing *Pogostemonis cablin, Scutellariae radix, Pinelliae rhizoma praeparatum cum alumine, Lonicerae flos, Forsythiae fructus,* and *Poria* according to a prescribed amount, adding 10 times the amount of water, decocting twice for 1.5 h each time, filtering to obtain a filtrate, combining the filtrate and the volatile oil extract obtained in step (1), concentrating under reduced pressure, and performing spray drying to obtain dry paste powder for later use;

(3) encapsulating the volatile oil obtained in step (1) in betacyclodextrin to obtain a volatile oil inclusion substance, drying, and crushing to obtain dry powder for later use; and

(4) subjecting the dry paste powder obtained in step (2) and the dry powder of the volatile oil inclusion substance obtained in step (3) to dry granulation, and producing the tablet according to a conventional method.

Further, the capsule of the traditional Chinese medicine composition of the present invention is produced by the following method:

(1) weighing *Perillae folium, Pogostemonis cablin, Moslae herba, Citri reticulatae pericarpium,* and *Zingiberis rhizoma recens* according to a prescribed amount, adding 10 times the amount of water, extracting a volatile oil via distillation by water vapor for 8 h, collecting the volatile oil for later use, and filtering an aqueous solution obtained after the distillation to obtain a volatile oil extract for later use;

(2) weighing *Pogostemonis cablin, Scutellariae radix, Pinelliae rhizoma praeparatum cum alumine, Lonicerae flos, Forsythiae fructus,* and *Poria* according to a prescribed amount, adding 10 times the amount of water, decocting twice for 1.5 h each time, filtering to obtain a filtrate, combining the filtrate and the volatile oil extract obtained in step (1), concentrating under reduced pressure, and performing spray drying to obtain dry paste powder for later use;

(3) encapsulating the volatile oil obtained in step (1) in betacyclodextrin to obtain a volatile oil inclusion substance, drying, and crushing to obtain dry powder for later use; and

(4) subjecting the dry paste powder obtained in step (2) and the dry powder of the volatile oil inclusion substance obtained in step (3) to dry granulation, and producing the capsule according to a conventional method.

Further, the present invention further provides a method for treating a disease, comprising administering the aforementioned traditional Chinese medicine composition, the traditional Chinese medicine composition produced by the aforementioned method, or the aforementioned medicament.

The treating includes at least one of treating a cold, inhibiting gastric emptying and intestinal motility, treating diarrhea, relieving pain and/or anti-inflammation effect.

The cold includes influenza, common cold or gastrointestinal type cold.

The gastrointestinal type cold is manifested as fever with aversion to wind, headache and dizziness, nausea and vomiting, diarrhea, nasal congestion and runny nose, limb fatigue and heaviness, abdominal distention and pain, or low food intake and poor appetite.

The treating a cold includes resisting H3N2, IBV, RSV, CVB3, EV71, HCoV-229E, PIV or RV virus.

The treating a cold includes at least one of:

inhibiting replication of coxsackievirus B3 in large intestine tissues, regulating balance of immune cells, and reducing inflammation reaction of local tissues; and

reducing degree of colonic lesions, increasing ratio of lymphocytes CD4+/CD8+ in blood, decreasing level of TNF-$\alpha$ and elevating level of IFN-$\gamma$ in large intestine tissues of model animals.

The method for producing an active ingredient of the traditional Chinese medicine composition provided by the present invention is simple, easy to operate, safe and environmentally friendly, and capable of retaining the active ingredient in the traditional Chinese medicine to the greatest extent to ensure the efficacy of the medicine.

## DETAILED DESCRIPTION

In order to illustrate the objectives, technical solutions and advantages of the present disclosure more clearly, the present disclosure will be further described in detail below in conjunction with the examples. It should be understood that the specific examples described herein are only used to explain the present disclosure, rather than limit the present disclosure.

In order to further illustrate the traditional Chinese medicine composition provided by examples of the present invention having efficacy in treating colds, the following examples are provided for further exemplary illustration.

Example 1

Prescription:

286 g of *Perillae folium,* 286 g of *Pogostemonis cablin,* 286 g of *Moslae herba,* 286 g of *Magnoliae officinalis cortex,* 286 g of *Scutellariae radix,* 214 g of *Pinelliae rhizoma praeparatum cum alumine,* 357 g of *Lonicerae flos,* 286 g of *Forsythiae fructus,* 286 g of *Poria,* 286 g of *Citri reticulatae pericarpium,* and 143 g of *Zingiberis rhizoma recens.*

Preparation

Used were 11 components described above. To *Perillae folium, Pogostemonis cablin, Moslae herba, Citri reticulatae pericarpium,* and *Zingiberis rhizoma recens,* 10 times the amount of water was added. A volatile oil was extracted via distillation by water vapor for 8 h. The volatile oil was collected for later use. An aqueous solution obtained after the distillation was filtered for later use. Other 6 components including *Magnoliae officinalis cortex* were added with 10 times the amount of water, decocted twice for 1.5 h each time, and filtered to obtain a filtrate. The filtrate and the aqueous solution obtained after the distillation were concentrated under reduced pressure and subjected to spray drying to collect spray powder for later use. The volatile oil was encapsulated in 10 times the amount of betacyclodextrin to obtain an inclusion substance. The inclusion substance was dried and crushed. The spray powder, the inclusion substance, sucralose, and maltodextrin were mixed well and prepared into 1000 g of granules by dry granulation to obtain the final product.

Example 2

Prescription:

340 g of *Perillae folium,* 230 g of *Pogostemonis cablin,* 340 g of *Moslae herba,* 230 g of *Magnoliae officinalis cortex,* 340 g of *Scutellariae radix,* 170 g of *Pinelliae rhizoma praeparatum cum alumine,* 430 g of *Lonicerae flos,* 230 g of *Forsythiae fructus,* 340 g of *Poria,* 230 g of *Citri reticulatae pericarpium,* and 170 g of *Zingiberis rhizoma recens.*

Preparation

Used were 11 components described above. To *Perillae folium, Pogostemonis cablin, Moslae herba, Citri reticulatae pericarpium,* and *Zingiberis rhizoma recens,* 8 times the amount of water was added. A volatile oil was extracted via distillation by water vapor for 10 h. The volatile oil was collected for later use. An aqueous solution obtained after the distillation was filtered for later use. Other 6 components including *Magnoliae officinalis cortex* were added with 8 times the amount of water, decocted twice for 2 h each time, and filtered to obtain a filtrate. The filtrate and the aqueous solution obtained after the distillation were concentrated under reduced pressure and subjected to spray drying to collect spray powder for later use. The volatile oil was encapsulated in 10 times the amount of betacyclodextrin to obtain an inclusion substance. The inclusion substance was dried and crushed. The spray powder, the inclusion substance, sucralose, and maltodextrin were mixed well and prepared into 1000 g of granules by dry granulation. The tablet was prepared according to a conventional method to obtain the final product.

Example 3

Prescription:

230 g of *Perillae folium,* 340 g of *Pogostemonis cablin,* 230 g of *Moslae herba,* 340 g of *Magnoliae officinalis cortex,* 230 g of *Scutellariae radix,* 260 g of *Pinelliae rhizoma praeparatum cum alumine,* 285 g of *Lonicerae flos,* 340 g of *Forsythiae fructus,* 230 g of *Poria,* 340 g of *Citri reticulatae pericarpium,* and 115 g of *Zingiberis rhizoma recens.*

Preparation

Used were 11 components described above. To *Perillae folium, Pogostemonis cablin, Moslae herba, Citri reticulatae pericarpium, and Zingiberis rhizoma recens,* 12 times the amount of water was added. A volatile oil was extracted via distillation by water vapor for 10 h. The volatile oil was collected for later use. An aqueous solution obtained after the distillation was filtered for later use. Other 6 components including *Magnoliae officinalis cortex* were added with 8 times the amount of water, decocted twice for 1 h each time, and filtered to obtain a filtrate. The filtrate and the aqueous solution obtained after the distillation were concentrated under reduced pressure and subjected to spray drying to collect spray powder for later use. The volatile oil was encapsulated in 10 times the amount of betacyclodextrin to obtain an inclusion substance. The inclusion substance was dried and crushed. The spray powder, the inclusion substance, sucralose, and maltodextrin were mixed well and prepared into 1000 g of granules by dry granulation. The capsule was prepared according to a conventional method to obtain the final product.

Example 4

Prescription:

300 g of *Perillae folium,* 300 g of *Pogostemonis cablin,* 300 g of *Moslae herba,* 300 g of *Magnoliae officinalis cortex,* 300 g of *Scutellariae radix,* 230 g of *Pinelliae rhizoma praeparatum cum alumine,* 380 g of *Lonicerae flos,* 300 g of *Forsythiae fructus,* 300 g of *Poria,* 300 g of *Citri reticulatae pericarpium,* and 150 g of *Zingiberis rhizoma recens.*

Preparation

Used were 11 components described above. To *Perillae folium, Pogostemonis cablin, Moslae herba, Citri reticulatae pericarpium, and Zingiberis rhizoma recens,* 10 times the amount of water was added. A volatile oil was extracted via distillation by water vapor for 8 h. The volatile oil was collected for later use. An aqueous solution obtained after the distillation was filtered for later use. Other 6 components including *Magnoliae officinalis cortex* were added with 10 times the amount of water, decocted twice for 1.5 h each time, and filtered to obtain a filtrate. The filtrate and the aqueous solution obtained after the distillation were concentrated under reduced pressure and subjected to spray drying to collect spray powder for later use. The volatile oil was encapsulated in 10 times the amount of betacyclodextrin to obtain an inclusion substance. The inclusion substance was dried and crushed. The spray powder, the inclusion substance, sucralose, and maltodextrin were mixed well and prepared into the pill according to a conventional method to obtain the final product.

Example 5

Prescription:

250 g of *Perillae folium,* 250 g of *Pogostemonis cablin,* 250 g of *Moslae herba,* 250 g of *Magnoliae officinalis cortex,* 250 g of *Scutellariae radix,* 200 g of *Pinelliae rhizoma praeparatum cum alumine,* 320 g of *Lonicerae flos,* 250 g of *Forsythiae fructus,* 250 g of *Poria,* 250 g of *Citri reticulatae pericarpium,* and 125 g of *Zingiberis rhizoma recens.*

Preparation

Used were 11 components described above. To *Perillae folium, Pogostemonis cablin, Moslae herba, Citri reticulatae pericarpium, and Zingiberis rhizoma recens,* 10 times the amount of water was added. A volatile oil was extracted via distillation by water vapor for 8 h. The volatile oil was collected for later use. An aqueous solution obtained after the distillation was filtered for later use. Other 6 components including *Magnoliae officinalis cortex* were added with 10 times the amount of water, decocted twice for 1.5 h each time, and filtered to obtain a filtrate. The filtrate and the aqueous solution obtained after the distillation were concentrated under reduced pressure, and prepared into the oral liquid according to the conventional method to obtain the final product.

In order to further illustrate the properties of the traditional Chinese medicine composition having efficacy in treating colds provided by the examples of the present invention, the granules obtained in the example were tested for efficacy, as shown below.

Test Example 1

In order to elaborate the safety and *in vitro* antiviral effects of the traditional Chinese medicine composition of the present invention, the granules prepared in Example 1 (hereinafter referred to as the traditional Chinese medicine composition of the present invention) were subjected to a toxicity test and an *in vitro* antiviral assay.

1. Study purpose

This assay investigated the *in vitro* antiviral effects of the pharmaceutical composition of the present invention on influenza A virus H3N2, influenza B virus (IBV), respiratory syncytial virus (RSV), rhinovirus (Rhv), coxsackievirus B3 (CVB3), enterovirus EV71, coronavirus HCoV-229E, parainfluenza virus (PIV), and rotavirus (RV), and provided a reference for its clinical studies.

2 Experimental materials

2.1 Test products

Tested was the pharmaceutical composition of the present invention provided by Shijiazhuang Yiling Pharmaceutical Co., Ltd. Batch number: 211101. Content: 4.5 g of crude drug/g drug powder. Expiration date: 2023.11.09. Administration and dosage: oral administration, 0.5 g of crude drug/kg.

2.2 Positive control product

Oseltamivir Acid (batch number: 18345) manufactured by MedChem Express. Period of validity: valid for 6 months (stored at -80°C) or for 1 month (stored at -20°C) after preparation. It was used as a positive control drug against influenza A virus H3N2 and influenza B virus (IBV). Ribavirin Spray (400 mg/bottle) manufactured by Penglai Nuokang Pharmaceutical Co, Ltd. Batch number: 210720. Expiration date: 2023.07.19. It was used as the positive control drug against respiratory syncytial virus (RSV), rhinovirus (Rhv), coxsackievirus B3 (CVB3), enterovirus EV71, rotavirus (RV), and parainfluenza virus (PIV). Recombinant human interferon $\alpha$2b gel manufactured by Zhaokao Pharmaceutical (Hefei) Co., Ltd. Specification: $1.0 \times 10^5$ IU/g, 5g/gel. Batch number: 20210821. Expiration date: 2023.02.27. It was used as the positive drug against coronavirus HCoV-229E.

### 2.3 Virus strains

Influenza A virus H3N2 (strain A/California/2/2014, number: ATCC VR-1938) was purchased from American Type Culture Collection (ATCC). Influenza B virus (IBV, B/Virginia/ATCC5/2012, number: ATCC VR-1807) was purchased from ATCC. Rhinovirus (Rhv, 130-63[V-185-001-021], number: ATCC VR-1187), respiratory syncytial virus (RSV, Long, number: ATCC VR-26PQ), coxsackievirus (CVB3, Nancy, number: ATCC VR-30), enterovirus (EV71, H, number: ATCC VR-1432), coronavirus (HCoV-229E, 229E, number: ATCC VR-740), parainfluenza virus (PIV, ATCC-2011-5, number: ATCC VR-1782), and rotavirus (RV, Hu/Australia/10-25-10/77/L, number: ATCC VR-2104) were all obtained from ATCC. These viruses were cultured and stored in our company's biosafety level-II laboratory, which was registered under the filing number: Laboratory Approval Document No. B010 (2021) issued by Changsha Health Commission.

### 2.4 Cell lines

African green monkey kidney (Vero) cells, Madin-Darby canine kidney (MDCK) cells, human laryngeal epidermoid carcinoma (HEp-2) cells, human fetal lung fibroblast (MRC-5) cells, and Lilly laboratories culture-monkey kidney 2 (LLC-MK2) cells were obtained from Wuhan Pricella Biotechnology Co., Ltd. Human cervical cancer (H1HeLa) cells were obtained from Beijing Beina Chuanglian Biotech Institute.

### 2.5 Main reagents

The main reagents are shown in Table 1 below.

Table 1 List of reagents

| Name | Specification | Manufacturer | Batch number |
|---|---|---|---|
| Basic medium (DMEM) | 500 mL | Gibco | 8121512/6121622 |
| Fetal bovine serum (FBS) | 100 mL | Sciencell, USA | 2300015 |
| Cell counting reagent (CCK-8) | 5 mL | GLPBIO | GK1001 |
| Phosphate buffer ($1 \times$PBS) | 500 mL | Solarbio | 20211115 |
| Dimethyl sulfoxide (DMSO) | 100 mL | BeyoSIme | ST038 |
| Trypsin (Trypsin TPCK-treated) | 100 mg | Sangon Biotech (Shanghai) Co., Ltd. | G810BD0313 |

### 2.6 Main instruments

BSCIIB2-1101 Biosafety Cabinet was produced by Shanghai Ruiyang Purification Equipment Co., Ltd., YXQ-50A Vertical Pressure Steam Sterilizer was produced by Shanghai Boxun Medical Biological Instrument Corp, 3111 $CO_2$ Incubator was produced by Thermo Fisher (USA), CJ-1F Medical Purified Workbench was produced by Suzhou Fengshi Laboratory Equipment Co., Ltd., SW-CJ-1FD Clean Bench was produced by Suzhou Antai Airtech Co., Ltd (Sujing Group), and DMi8 Inverted Microscope was produced by Leica (Germany).

### 3 Test contents

### 3.1 Preparation of medium

### 3.1.1 Preparation of complete cell medium

DMEM medium was added with FBS with a final concentration of 10%, mixed well, and stored at 2-8°C, for later use.

### 3.1.2 Preparation of virus maintenance solution

Serum-free DMEM medium (for culturing Rhv and PIV viruses), DMEM medium + 2 $\mu$g/mL TPCK-treated trypsin (for culturing H3N2 and IBV viruses), DMEM medium containing 2% FBS (for culturing RSV, CVB3, EV71 and HCoV-229E viruses) and DMEM medium + 1 $\mu$g/mL trypsin (for culturing RV virus).

3.2 Cell culture

African green monkey kidney (Vero) cells, Madin-Darby canine kidney (MDCK) cells, human laryngeal epidermoid carcinoma (HEp-2) cells, human fetal lung fibroblast (MRC-5) cells, Lilly laboratories culture-monkey kidney 2 (LLC-MK2) cells, and human cervical cancer cell (H1HeLa) are all adherent cells, and were cultured in DMEM medium containing 10% FBS. The culture method was as follows. The cells that grew into a single layer adhering to the wall were taken, and the medium was discarded. The cells were washed twice with 1×PBS, then added with 1 mL of 0.25% Trypsin-EDTA for digestion, left to stand for 2-5 min in an incubator, and observed under a microscope. After the cells became rounded and were about to be detached, 3 mL of complete medium was added to terminate the digestion, and a suspension of single cells was obtained by gently pipetting. The suspension of single cells was transferred to the EP tube, and centrifuged at 1000 rpm for 5 min. The supernatant was discarded. The obtained cells were resuspended in an appropriate amount of fresh complete medium, inoculated into new culture flasks according to a certain proportion, and cultured in a 37°C, 5% $CO_2$ incubator. According to the growth state, the cells were passaged every 2-3 days.

3.3 Cytotoxic effects of pharmaceutical composition of the present invention

(1) Test Grouping: The appropriate starting concentration of the pharmaceutical composition was selected for test grouping according to the references. All the pharmaceutical compositions were diluted with serum-free DMEM medium. For the cell control group, only serum-free DMEM medium was used. For the test group, the pharmaceutical composition of the present invention was tested at concentrations of 1000, 333.33, 111.11, 100, 33.33, and 11.11 µg/mL. For the positive control drug group, oseltamivir acid as a positive drug was set up at concentrations of 1000, 500, 250, 125, 62.5, and 31.25 µM. Ribavirin as a positive drug was set up at final concentrations of 1000, 333.33, 111.11, 100, 33.33, and 11.11 µg/mL. Recombinant human interferon α2b gel as a positive drug was set up at final concentrations of 1000, 333.33, 111.11, 100, 33.33, and 11.11 IU/mL. The blank control group contained only serum-free DMEM medium and no cells. Three replicate wells were set up for each concentration in each group.

(2) Cell Plating: Cells were prepared into a suspension single cells ($1×10^4$ cells/mL), seeded to a 96-well cell culture plate (100 µL/well), and incubated overnight in a 37°C, 5% $CO_2$ incubator.

(3) Addition of Drug and Test: The culture medium was discarded and the cells were washed twice with 1×PBS. For each test group, 100 µL of media containing different concentrations of drug were added to each well and cells were cultured in a 37°C, 5% $CO_2$ cell incubator for 72 h. 10 µL of CCK8 was added to each well. After cells were incubated in the incubator for 1-4 h, the $OD_{450\,nm}$ value was read using a microplate reader. The inhibition rate of cells and the half maximal inhibitory concentration ($IC_{50}$) of the tested drugs on cells were calculated.

Inhibition rate (%) = (average $OD_{450\,nm}$ value of the cell in control group - average $OD_{450nm}$ value of the cell in drug group)/(average $OD_{450nm}$ value of the cell in control group)×100%.

3.4 Amplification of viruses

Host cells MDCK, H1HeLa, HEp-2, LLC-MK2, MRC-5 and Vero were inoculated into culture flasks respectively. When the cell confluence reached 70-80%, the medium was discarded, and the cells were washed twice with 1×PBS. The viral solution (1 mL) was then added to the T25 flask. The cells were incubated in an incubator, and the culture plate was gently shaken every 15-30 min to ensure that the virus were adsorbed evenly on the cell surface. After 2 h, the viral solution was discarded and the cells were washed twice with 1×PBS for removal of free viruses. The cells were added with appropriate amount of virus maintenance solution and incubated in an incubator. The specific incubation conditions are shown in Table 2. The cells were observed daily under an inverted microscope for morphological changes. When 80%-100% of the cells became swollen and rounded, with increased intercellular spaces, nuclear shrinkage or rupture, and partial or complete cell detachment in severe cases, the culture was terminated. The cells were then lysed using a repeated freeze-thaw method. After centrifugation at 3000 rpm for 10 min, the supernatant was collected, aliquoted into freezing tubes, and either used for the next step of the test or stored under an ultra-low temperature for later use.

Table 2 Culture conditions for viruses and host cells

| Virus | Host cell | Culture condition |
|---|---|---|
| influenza A virus (H3N2) | MDCK | 35°C, 5% $CO_2$ |

(continued)

| Virus | Host cell | Culture condition |
|---|---|---|
| influenza B virus (IBV) | MDCK | 35°C, 5% $CO_2$ |
| coronavirus (HCoV-229E) | MRC-5 | 33°C, 5% $CO_2$ |
| respiratory syncytial virus (RSV) | HEp-2 | 37°C, 5% $CO_2$ |
| Coxsackievirus B3 (CVB3) | LLC-MK2 | 33°C-37°C, 5% $CO_2$ |
| enterovirus (EV71) | LLC-MK2 | 33°C-37°C, 5% $CO_2$ |
| parainfluenza virus (PIV) | LLC-MK2 | 33°C-37°C, 5% $CO_2$ |
| rotavirus (RV) | Vero | 37°C, 5% $CO_2$ |
| rhinovirus (Rhv) | H1HeLa | 33°C, 5% $CO_2$ |

3.5 Determination of 50% tissue culture infective dose ($TCID_{50}$) of virus

The viral solution collected in 3.3 was subjected to $TCID_{50}$ assay, and the specific method was as follows.

(1) Cell Plating: Vero, MDCK, HEp-2, MRC-5, LLC-MK2 and H1HeLa cells were separately prepared into a suspension of single-cells ($1\times10^4$ cells/mL), inoculated into a 96-well cell culture plate at 100 $\mu$L per well, and incubated overnight in a 37°C, 5% $CO_2$ incubator.

(2) Inoculation of Virus: After the cells had grown to monolayer adherent cells, the original virus solution was serially diluted with DMEM medium at a 10-fold ratio ($10^{-1}$, $10^{-2}$, $10^{-3}$, $10^{-4}$, $10^{-5}$ and $10^{-6}$) to obtain 6 concentrations. For each concentration, 8 replicate wells and 2 replicate control wells containing cells were set up. The original medium in the 96-well plate was discarded, and the plate was washed twice with $1\times$PBS. Subsequently, 100 $\mu$L of the corresponding concentration of viral dilution was added to each well.

(3) Culture and Observation: After the inoculation of viruses was completed, the 96-well cell culture plate was incubated in an incubator with corresponding culture conditions for 2 h. The culture plate was taken and gently shaken every 30 min to promote virus adsorption. After 2 h of adsorption was completed, the medium in each well was replaced with 100 $\mu$L of virus maintenance solution, and the incubation was continued. The cytopathic effect (CPE) was observed daily under an inverted microscope, and the number of wells in which cells showed CPE was recorded. The endpoint was defined as the highest dilution at which no CPE was observed. The $TCID_{50}$ was calculated using the Reed-Muench formula:

$TCID_{50}$ = logarithm of the highest virus dilution with CPE above 50% + proportionate distance value $\times$ logarithm of the dilution factor

Wherein proportionate distance value = (percentage of wells with CPE above 50% - 50%) / (percentage of wells with CPE above 50% - percentage of wells with CPE below 50%).

3.6 *In vitro* antiviral effect of the pharmaceutical combination of the present invention

(1) Test Grouping: Concentrations were set up based on the comprehensive results of cytotoxicity assays and $OD_{450nm}$, values, and literature reports on the antiviral effects of positive control drugs. The concentration without significant toxic effects on cells under microscopic observation was selected as the starting concentration for the *in vitro* antiviral test. Wherein the test substance (the pharmaceutical composition of the present invention) was serially diluted at a 2-fold ratio, the oseltamivir acid as the positive control drug was serially diluted at a 4-fold ratio, and other drugs were serially diluted at a 2-fold ratio. All of them were diluted into 5 concentrations, with 4 replicate wells set up for each concentration. A virus titer of 100 $TCID_{50}$ was used to infect the cells. The grouping was as follows: virus + drug group, in which the drug group included the test substance group and the positive control drug group; the virus control group (with virus and no drug), and the cell control group (with virus maintenance solution only). See Table 3 below for detail.

(2) Cell Plating: Cells were prepared into a single cell suspension ($1\times10^4$ cells/mL), seeded into a 96-well cell culture

plate at 100 $\mu$L per well, and incubated overnight in a 37°C, 5% $CO_2$ incubator.

(3) Addition of Drug and Observation: The medium was discarded, and the cells were washed twice with 1×PBS. Then, 50 $\mu$L of virus solution of 100 $TCID_{50}$ was added to each well for 2 h of adsorption. The virus solution was discarded. The cells were washed twice with 1×PBS, added with 100 $\mu$L of medium containing different concentrations of drugs (according to the above group settings), respectively, and continued to be cultured.

(4) The cytopathic effect (CPE) was observed. The culture was terminated when the cells with CPE in the virus control group reached approximately 75%. To each well, 10 $\mu$L of CCK-8 was added, followed by 1-4 h of incubation. The $OD_{450nm}$ value of the cells was read using a microplate reader. The inhibition rates of virus by different concentrations of the test substance and their median effective concentration ($EC_{50}$) against virus were calculated as follows.

Virus inhibition rate (%) = [(average $OD_{450nm}$ value of drug-treated group (drug + virus) - average $OD_{450nm}$ value of virus control group) / (average $OD_{450nm}$ value of cell control group-average $OD_{450nm}$ value of virus control group)] × 100%

Therapeutic index (TI) = $IC_{50}$ of cytotoxicity / $EC_{50}$ of *in vitro* antivirus

Table 3 Concentrations of antiviral activity *in vitro* and the amount of viral infection

| Name of virus | Amount of viral infection for antiviral assay *in vitro* (100TCID$_{50}$) | Concentration of drug | | | |
| --- | --- | --- | --- | --- | --- |
| | | The pharmaceutical composition of the present invention ($\mu$g/mL) | Oseltamivir Acid (nM) | Ribavirin Spray ($\mu$g/mL) | Recombinant human interferon $\alpha$2b gel (IU/mL) |
| influenza A virus (H3N2) | $10^{-2.29}$/0.1mL | 200, 100, 50, 25, 12.5 | 2000, 500, 125, 31.25, 7.8125 | / | / |
| influenza B virus (IBV) | $10^{-2.23}$/0.1mL | 200, 100, 50, 25, 12.5 | 2000, 500, 125, 31.25, 7.8125 | / | / |
| coronavirus (HCoV-229E) | $10^{-1.50}$0.1mL | 200, 100, 50, 25, 12.5 | / | / | 1000, 500, 250, 125, 62.5 |
| respiratory syncytial virus (RSV) | $10^{-1-29}$/0.1mL | 1000, 500, 250, 125, 62.5 | / | 10, 5, 2.5, 1.25, 0.625 | / |
| Coxsackievirus B3 (CVB3) | $10^{-2.19}$/0.1mL | 100, 50, 25, 12.5, 6.25 | / | 500, 250, 125, 62.5, 31.25 | / |
| enterovirus (EV71) | $10^{-1.40}$/0.1mL | 100, 50, 25, 12.5, 6.25 | / | 100, 50, 25, 12.5, 6.25 | / |
| parainfluenza virus (PIV) | $10^{-0.76}$/0.1mL | 100, 50, 25, 12.5, 6.25 | / | 100, 50, 25, 12.5, 6.25 | / |
| rotavirus (RV) | $10^{-0.88}$/0.1mL | 100, 50, 25, 12.5, 6.25 | / | 100, 50, 25, 12.5, 6.25 | / |
| rhinovirus (Rhv) | $10^{-0.46}$/0.1mL | 10, 5, 2.5, 1.25, 0.625 | / | 30, 15, 7.5, 3.75, 1.875 | / |

4 Data processing and information analysis

All data in the summary report of this test were rounded off according to the principle of rounding. Values that were not evenly divisible were retained to two decimal places. Excel 2010 was used to statistically analyze the raw data and calculate the inhibition rate according to the corresponding formula. Subsequently, the GraphPad Prism 8.0.1 software was utilized to perform curve fitting based on the inhibition rate to determine the $IC_{50}$ or $EC_{50}$ values.

5 Test results

5.1 Results of cytotoxicity assay of the pharmaceutical composition of the present invention

As shown in Table 4, the $IC_{50}$ value of the *in vitro* cytotoxicity of the test substance (pharmaceutical composition of the present invention) on MDCK cells was 321.20 μg/mL, and other results were greater than 1000 μg/mL. For subsequent tests, the concentration of the drug that showed no obvious cytotoxic effects on cells under microscopic observation was selected as the starting concentration for the *in vitro* antiviral assays.

Table 4 Summary of $IC_{50}$ of the *in vitro* cytotoxicity of the pharmaceutical composition of the present invention (n=3)

| Cell name | $IC_{50}$ | | | |
| --- | --- | --- | --- | --- |
| | the pharmaceutical composition of the present invention (μg/mL) | Ribavirin (μg/mL) | Oseltamivir acid (μM) | Recombinant human interferon α2b gel (IU/mL) |
| MDCK | 321.20 | / | >1000 | / |
| LLC-MK2 | >1000 | >1000 | / | / |
| MRC-5 | >1000 | / | / | >1000 |
| HEp-2 | >1000 | >1000 | / | / |
| H1HeLa | >1000 | >1000 | 1 | / |
| Vero | >1000 | >1000 | / | / |

5.2 Results of viral titer detection

The results of the viral titer detection are shown in Table 5, and a viral infective dose of 100 $TCID_{50}$ was selected for subsequent *in vitro* antiviral assays.

Table 5 Summary of 50% tissue culture infective dose ($TCID_{50}$) of virus on cell (n=8)

| Virus name | $TCID_{50}$ |
| --- | --- |
| H3N2 | $10^{-4.29}$/0.1mL |
| IBV | $10^{-4.23}$/0.1mL |
| Rhv | $10^{-2.46}$/0.1mL |
| RSV | $10^{-3.29}$/0.1mL |
| CVB3 | $10^{-4.19}$/0.1mL |
| EV71 | $10^{-3.40}$/0.1mL |
| HCoV-229E | $10^{-3.50}$0.1mL |
| PIV | $10^{-2.76}$/0.1mL |
| RV | $10^{-2.88}$/0.1mL |

5.3 Results of in vitro antiviral assays of the pharmaceutical composition of the present invention

The cells in the cell control group showed normal morphology. In the virus control group, cell particles increased, cells became rounded in shape and most of them were detached. The pharmaceutical composition of the present invention can significantly inhibit virus-induced cytopathic effects. The inhibition rate of virus by the pharmaceutical composition gradually increased with increasing drug concentrations. As shown in Table 6, the *in vitro* antiviral $EC_{50}$ values of the test substance (the pharmaceutical composition of the present invention) against influenza A virus (H3N2), influenza B virus (IBV), respiratory syncytial virus (RSV), Coxsackievirus B3 (CVB3), enterovirus (EV71), and coronavirus (HCoV-229E) were 71.83, 52.02, 134.60, 30.50, 5.99 and 189.80 μg/mL, respectively. The *in vitro* antiviral $EC_{50}$ values against parainfluenza virus (PIV) and rotavirus (RV) were greater than 100 μg/mL. The therapeutic index (TI) of the pharmaceutical composition of the present invention against the above viruses were 4.47, 6.17, greater than 7.43, greater than 32.79, greater than 166.94, greater than 5.27, greater than 10 and greater than 10, respectively. In the *in vitro* antiviral assay against rhinovirus (Rhv), there was no significant difference in the cell survival rate between the drug-treated group and the virus control group, indicating that the pharmaceutical composition of the present invention had no significant inhibitory effect on rhinovirus (Rhv) *in vitro.*

Table 6 Summary of EC$_{50}$ results of *in vitro* antiviral assay (n=4)

| Virus name | EC$_{50}$ | | | | Therapeutic index (TI) |
| --- | --- | --- | --- | --- | --- |
| | the pharmaceutical composition of the present invention ($\mu$g/mL) | Oseltamivir acid (nM) | Recombinant human interferon $\alpha$2b gel (IU/mL) | Ribavirin ($\mu$g/mL) | |
| H3N2 | 71.83 | 931 | / | / | 4.47 |
| IBV | 52.02 | 80.64 | / | / | 6.17 |
| Rhv | >10 | / | / | >30 | / |
| RSV | 134.60 | / | / | >10 | >7.43 |
| CVB3 | 30.50 | / | / | 101.90 | >32.79 |
| EV71 | 5.99 | / | / | 6.03 | >166.94 |
| HCoV-229 E | 189.80 | / | 410.20 | / | >5.27 |
| PIV | >100 | / | / | 20.94 | >10 |
| RV | >100 | / | / | 20.64 | >10 |

6 Conclusion and Evaluation

The result showed that, after influenza A virus (H3N2), influenza B virus (IBV), rhinovirus (Rhv), respiratory syncytial virus (RSV), Coxsackievirus B3 (CVB3), enterovirus (EV71), coronavirus (HCoV-229E), parainfluenza virus (PIV) and rotavirus (RV) separately infected host cells for 2 h, the pharmaceutical composition of the present invention was administered at different concentrations. After a period of culture, the virus control groups showed cytopathic effects and the normal control group showed normal cell morphology. After the treatment with the pharmaceutical composition of the present invention, the number of cells with normal morphology significantly increased. Changes in cell morphology included shrinkage, overlapping, or detachment. Within the safe concentration range, the inhibition rate against virus increased with higher drug concentrations. These results indicated the pharmaceutical composition of the present invention can inhibit influenza A virus (H3N2), influenza B virus (IBV), respiratory syncytial virus (RSV), Coxsackievirus B3 (CVB3), enterovirus (EV71), coronavirus (HCoV-229E), parainfluenza virus (PIV) and rotavirus (RV) *in vitro*. However, in the test where the pharmaceutical composition of the present invention within the tested concentration range treated rhinovirus (Rhv)-infected H1HeLa cells, there was no significant difference in cell survival rate between the group treated with the pharmaceutical composition of the present invention and the virus control group, indicating that the pharmaceutical composition of the present invention had no obvious inhibitory effect on rhinovirus *in vitro.*

Conclusion: The pharmaceutical composition of the present invention had strong inhibitory effects *in vitro* on influenza A virus (H3N2), influenza B virus (IBV), respiratory syncytial virus (RSV), Coxsackievirus B3 (CVB3), enterovirus (EV71), coronavirus (HCoV-229E), parainfluenza virus (PIV), and rotavirus (RV), with EC$_{50}$ values of 71.83 $\mu$g/mL, 52.02 $\mu$g/mL, 134.60 $\mu$g/mL, 30.50 $\mu$g/mL, 5.99 $\mu$g/mL, 189.80 $\mu$g/mL, greater than 100 $\mu$g/mL, and greater than 100 $\mu$g/mL, respectively. Its therapeutic index (TI) was 4.47, 6.17, greater than 7.43, greater than 32.79, greater than 166.94, greater than 5.27, greater than 10, and greater than 10, respectively.

Test Example 2

Coxsackievirus B3 is a common infectious enterovirus found in the human intestinal tract. It mainly invades immunocompromised perinatal infants. It can replicate extensively in the gastrointestinal tract after infection, causing gastrointestinal disorder, which is mainly manifested as recurrent diarrhea. If it is not treated promptly and the condition worsens, the virus may invade the bloodstream through the gastrointestinal system, leading to various human diseases, including myocarditis, herpangina, aseptic meningitis, pancreatitis, Sicca syndrome, hand-foot-and-mouth disease, and even neonatal sudden death. To date, there are no specific drugs or vaccines available for the treatment or prevention of Coxsackievirus B3 infections. Therefore, in this study, young SD rats were used as research subjects, and they were infected with Coxsackievirus B3 by intraperitoneal injection. Huo Xiang Zheng Qi capsule and ribavirin granules were selected as positive controls to evaluate the efficacy of the pharmaceutical composition of the present invention against Coxsackievirus B3 infection *in vivo,* and to provide experimental bases for its clinical research.

In order to elucidate the activity of the traditional Chinese medicine composition of the present invention for the treatment of colds, the granules prepared in Example 1 (hereinafter referred to as the traditional Chinese medicine composition of the present invention) were used for the following pharmacodynamics tests.

1 Study purpose

In this study, young SD rats were injected intraperitoneally with Coxsackievirus B3 to establish *in vivo* infection model, and Huo Xiang Zheng Qi capsule and ribavirin granules were selected as positive controls to evaluate the efficacy of the pharmaceutical composition of the present invention against Coxsackievirus B3 *in vivo,* and to provide experimental basis for its clinical use.

2 Experimental materials

2.1 Test products

The pharmaceutical composition of the present invention was provided by Shijiazhuang Yiling Pharmaceutical Co., Ltd. Batch number: 211101. Content: 4.5 g of crude drug/g drug powder. Expiration date: tentatively set until 2023.11.09. Administration and dosage: oral administration, 0.5 g of crude drug/kg.

2.2 Positive control product

The details of Huo Xiang Zheng Qi capsules provided by Shijiazhuang Yiling Pharmaceutical Co., Ltd. were as follows. Specification: 0.3g/capsule. Batch Number: 210501. Expiration date: April 2023. Manufacturer: Jiangxi Yuanjian Pharmaceutical Co. Ltd. Administration and dosage: oral administration, 4 capsules twice daily. Functions and indications: releasing the exterior, transforming dampness, regulating qi, and harmonizing the middle jiao. It is useful for cold due to external wind invasion and internal dampness stagnation, manifested as headache and dizziness, tightness and fullness in the chest and diagram, abdominal distension and pain, vomiting, and diarrhea.

Ribavirin granules. Batch Number: 200904. Expiration date: 2023.07. Manufacturer: Sichuan Baili Pharmaceutical Co. Ltd. Indications: This product is suitable for respiratory syncytial virus-caused viral pneumonia and bronchitis and skin herpes virus infection. Administration and dosage: For viral respiratory tract infection: For adults, 0.15 g at a time, three times daily, for 7 consecutive . For skin herpes simplex virus infection: For adults, 0.3 g at a time, 3-4 times daily, for 7 consecutive days.

2.3 Experimental Animals

39 (4 litters) young SD rats, with female rats, 7 days old, SPF grade, without restriction to male and female, were used to establish the Coxsackievirus B3 (CVB3) infection model of young SD rats, which were purchased from Changsha Tianqin Biotechnology Co., Ltd. (Production License Number: SCXK (Hunan) 2019-0014), with animal quality certificate number of 430726221100014858. 350 (35 litters) young SD rats, with female rats, 7 days old, SPF grade, without restriction to male and female, were used to evaluate the effects of the pharmaceutical composition of the present invention on the Coxsackievirus B3 (CVB3) infection model of young SD rats, which were purchased from Hunan Slack Jingda Laboratory Animals Co., Ltd. (Production License Number: SCXK (Hunan) 2019-0004), with animal quality certificate number of 430727221101001215. All animals were housed in Biosafety Level 2 (ABSL-II) facility, located in Zone C in a laboratory animal barrier environment. The Laboratory Animal Certificate Number was SYXK (Hunan) 2020-0015. The Laboratory Registration Number was Laboratory Approval Document No. B010 (2021) issued by Changsha Health Commission. The Ethical Approval Number was IACUC-2022(2)003-1.

2.4 Virus

Coxsackievirus B3 (CVB3), with the number: ATCC VR-30, provided by the American Type Culture Collection (ATCC) was cultured and amplified in the pathogenic microorganism laboratory (BSL-II, Laboratory Registration Number: Laboratory Approval Document No. B010 (2021) issued by Changsha Health Commission) of this center.

2.5 Main Reagents

The rat CD4 antibody, with the batch number: 554838, was manufactured by BD Pharmingen. The rat CD8 antibody, with the batch number: 200609, was manufactured by Biolegend. The rat CD11b antibody, with the batch number: 17-0112-82, was manufactured by e-Bioscience. The rat CD3 antibody, with the batch number: 2220944, was manufactured by Thermo Fisher. The rat CD16/CD32 antibody, with the batch number: 24222285, was manufactured by Thermo Fisher. The Rb mAb to NCAM1 (CD56) antibody, with the batch number: Ab237380, was manufactured by Abcam. The high glucose DMEM medium (specification: 500 mL/bottle), was manufactured by Gibco, with the batch number: 8121512. The fetal bovine serum (specification: 100 mL/bottle) was manufactured by Sciencell, USA, with the batch number: 2300015. Rat Interleukin-1β (IL-1β) ELISA Kit, Rat Interleukin-10 (IL-10) ELISA Kit, Rat Tumor Necrosis Factor Alpha (TNF-α) ELISA Kit and Rat Gamma Interferon (IFN-γ) ELISA Kit, with the batch number: 202205 were manufactured by Jiangsu Meimian Industrial Co., Ltd. Expiration date: 2022.11.

2.6 Main Instruments

Used instruments included BCR-MI02-72-C11-PPSU mouse independent ventilation cage system (manufacturer: Shandong Xinhua Medical Instrument Co., Ltd.), BSC1300-II-B2 biological safety cabinet (manufacturer: Shandong Xinhua Medical Instrument Co., Ltd.), CJ-1F medical purification bench (manufacturer: Suzhou Fengshi Laboratory Equipment Co., Ltd.), Model 3111 $CO_2$ Incubator (Manufacturer: Thermo Fisher, USA), DMIL Inverted Microscope (Manufacturer: Leica, Germany), YXQ-50A Vertical Pressure Steam Sterilizer (Manufacturer: Shanghai Boxun Medical Biological Instrument Co., Ltd.), TD4 Benchtop Low-speed Centrifuge (Manufacturer: Hunan Kaida Industrial Development Co., Ltd.), DFC 420C Camera for Pathology Imaging (Manufacturer: Leica, Germany), Spectra Max i3x Multi-Mode Microplate Reader (Manufacturer: MeiGu Photonics, Austria), Nucleic Acid Micro-detector (Manufacturer: Thermo Fisher, USA), Beckman: A00-1-1102 Flow Cytometer, and C6 Flow Cytometer (Manufacturer: BD, USA).

3 Study Content

3.1 Measurement of virus virulence

3.1.1 Cell recovery

The freezing tube was taken from the -80°C refrigerator or liquid nitrogen, and quickly placed into warm water at about 37°C, during which the freezing tube was constantly shaken to make it completely thawed. The liquid in the tube was removed into a centrifuge tube and centrifuged at 1000 rpm for 5 min, and the supernatant was discarded. An appropriate amount of complete culture solution containing 10% FBS was aspirated and mixed well by pipetting with a pipette. The cells were inoculated into the cell culture flask, added with an appropriate amount of complete culture solution, and cultured in a 37°C, 5% $CO_2$ incubator. The cap of the flask was loosened, leaving a gap, in order to facilitate gas exchange and maintain a stable pH environment.

3.1.2 Virus amplification

The freezing tube was taken from the -80°C refrigerator or liquid nitrogen, and quickly placed into warm water at about 37°C, during which the freezing tube was constantly shaken to make it completely thawed. The liquid in the tube was added to the well cultured cells, and the medium was replenished to just cover cells for 1-2 h of virus adsorption, during which, the culture flask was gently shaken every 15 min for uniform virus adsorption. After 2h of virus adsorption, an appropriate amount of virus growth solution was added, and the cells were placed and cultured in a 5% $CO_2$ incubator at 33-37°C.

3.1.3 $TCID_{50}$ Assay

The virus amplified in 3.1.2 was subjected to $TCID_{50}$ assay, and the specific method was as follows.

(1) Cell Plating: An appropriate amount of cells were prepared into a suspension of single cells ($1 \times 10^4$ cells/mL), seeded into a 96-well plate at 100 μL per well, and incubated overnight in a 37°C, 5% $CO_2$ incubator.

(2) Virus inoculation: After the cells grew to a monolayer of adherent cells, the original virus solution was diluted with DMEM medium at a 10-fold ratio to perform serial dilution from $10^{-1}$ to $10^{-6}$, and 8 replicate wells were set up for each dilution. The original medium in the 96-well plate was discarded and the cells were washed twice with $1 \times$ PBS solution. Then 100 μL of virus dilution solution with the corresponding concentration was added to each well. 8 wells containing cells without inoculating the virus were also set up as negative controls.

(3) Culture and Observation: After inoculation of viruses, the 96-well cell culture plate was incubated in an incubator with corresponding culture conditions for 2 h. The culture plate was taken and gently shaken every 30 min to promote virus adsorption. After 2 h of adsorption, the medium in each well was replaced with 100 μL of virus maintenance solution, and the incubation was continued. The cytopathic effect was observed daily under a microscope, and the number of wells in which cells showed CPE was recorded. The endpoint was defined as the highest dilution at which no CPE was observed. The $TCID_{50}$ was calculated according to the Reed-Muench formula.

Reed-Muench formula: $TCID_{50}$ = logarithm of the highest virus dilution with >50% CPE + proportional distance value $\times$ logarithm of the dilution factor Proportional distance value = (Percentage of >50% CPE - 50%) / (percent of >50% CPE - percent of <50% CPE).

3.2 Establishment of coxsackievirus B3 infection model of young SD rats

Thirty-seven quarantine-qualified young SD rats (4 litters), without restriction to male and female, 7 days old, were randomly grouped into the original solution group (6 rats), $10^{-1}$ group (6 rats), $10^{-2}$ group (6 rats), $10^{-3}$ group (6 rats), $10^{-4}$ group (4 rats), $10^{-5}$ group (5 rats) and $10^{-6}$ group (4 rats) according to body weight. The animals in each group were injected intraperitoneally with the corresponding concentration of CVB3 at 0.2 mL per rat. After the inoculation, the animals in each group were observed for death incident. 7 days after the virus inoculation, the animals in each group were scarified by cervical dislocation. Heart, liver, spleen, lung, brain, kidney, and gastrointestinal tissues were collected, fixed in 10% formaldehyde, embedded in paraffin, sectioned, and stained with HE, and the pathological changes of the tissues were observed under the microscope.

3.3 Effect of the pharmaceutical composition of the present invention on coxsackievirus B3 infection model of young SD rats

3.3.1 Grouping, modeling and administration

350 quarantine-qualified young SD rats (35 litters), without restriction to male and female, 7 days old, were selected and grouped into normal control group, model control group, Huo Xiang Zheng Qi capsule group (0.32 g/kg), ribavirin granule group (120.7 mg/kg), and low-, medium-, and high-dose groups of the pharmaceutical composition of the present invention (2.0, 4.0, and 8.0 g crude drug/kg) according to the female weight per litter. Each group consisted of 50 animals. Animals in each group were injected intraperitoneally with the original $CVB_3$ solution (titer $TCID_{50}=10^{-3.34}/100$ $\mu L$) at 0.2 mL/rat, and rats in the normal control group were injected intraperitoneally with an equivalent amount of 0.1 mmol/L phosphate buffer. Before the administration daily, the pharmaceutical composition of the present invention was prepared into solutions of different concentrations using pure water. Two hours after the virus infection, the animals in each group were orally administered with the corresponding concentration of test substance via gavage at a dose of 10 mL/kg, once a day, for 7 consecutive days. An equal volume of pure water was administered orally to the normal control group and the model control group.

3.3.2 Observation indicators

3.3.2.1 Measurement of viral load in tissue

On Day 4 (D4, 3 days after the administration) and Day 8 (D8, the next day after the last administration), 6 young rats were selected from each group and scarified by cervical dislocation, respectively. Through dissection, stomach, small intestine and large intestine tissues were separately collected and placed in a homogenizer. RNA extraction solution was added at a ratio of tissue (stomach, small intestine or large intestine) mass (mg): RNA extraction solution ($\mu L$) = 1:2. Homogenization was carried out using a high-speed homogenizer to prepare a tissue suspension. The suspension was centrifuged at 14000 rpm for 10 min at 4°C and the resulting supernatant was collected. RNA was extracted using the RNA extraction solution, and the extracted RNA was reverse transcribed into c-DNA. The fluorescence intensity of c-DNA obtained by reverse transcription was measured by using absolute quantitative PCR, and the fluorescence intensity of c-DNA was used to reflect the load of coxsackievirus $B_3$ in the tissues of the stomach, small intestine, and large intestine.

3.3.2.2 Histopathological examination

On Day 4 (D4, 3 days after the administration) and Day 8 (D8, the next day after the last administration), 10 young rats were selected from each group and scarified by cervical dislocation, respectively. Through dissection, stomach, small intestine and large intestine tissues were separately collected, fixed in 10% neutral formalin solution by perfusion, and stained with HE, and the histopathological changes of stomach, small intestine and large intestine were observed under the light microscope. If abnormalities were observed, the lesion characteristics were recorded and classified as mild (+), moderate (++), or severe (+++) according to the degree of lesion. If no obvious abnormalities were found, "NSL" (no significant lesion) was recorded.

3.3.2.3 Detection of lymphocyte in tissue

On Day 4 (D4, 3 days after the administration) and Day 8 (D8, the next day after the last administration), 6 young rats were selected from each group and subjected to blood collection from the heart. The lymphocyte (CD4$^+$ and CD8$^+$) level in blood was measured by flow cytometry.

3.3.2.4 Measurement of IL-1$\beta$, TNF-$\alpha$, IFN-$\gamma$ and IL-10 factors in tissues

On Day 4 (D4, 3 days after the administration) and Day 8 (D8, the next day after the last administration), 8 young rats were selected from each group and scarified by cervical dislocation, respectively. Through dissection, stomach, small intestine and large intestine tissues were separately collected, added with 0.9% sodium chloride injection according to the tissue (stomach, small intestine or large intestine) mass (g): 0.9% sodium chloride injection (mL) = 1:9, homogenized by grinding, and centrifuged at 3000 rpm for 10 min. The resulting supernatant was collected, and the contents of IL-1$\beta$, TNF-$\alpha$, IFN-$\gamma$ and IL-10 in stomach, small intestine and large intestine tissues were measured by using a rat ELISA kit.

3.3.3 Dosage design

The clinically intended dosage of the pharmaceutical composition of the present invention was 0.5 g of crude drug/kg/day. Assumed that the weight of infants was 10 kg, the body surface area was calculated as 0.466m$^2$ according to lgS=0.8762+0.698lgW. Assumed that the weight of young rats was 10 g, the body surface area was calculated as 0.00375m$^2$. The dose of the pharmaceutical composition of the present invention was calculated according to the animal dose conversion formula: $D_{young\ rat} = D_{infant} \times (W_{infant} \times S_{young\ rat})/(W_{young\ rat} \times S_{infant})$ = 0.5 g of crude drug/kg $\times$ (10$\times$0.00375)/(0.01$\times$0.466) = approximately 4.0 g of crude drug/kg. The low-dose and high-dose groups were set at 0.5 times and 2 times the equivalent dose, resulting in doses of 2.0 g of crude drug/kg and 8.0 g of crude drug/kg, respectively.

The clinical dosage of Huo Xiang Zheng Qi capsule was 4 capsules twice daily, and its specification was 0.3 g/capsule. Assumed that the weight of infants was 10 kg, the body surface area was calculated as 0.466m$^2$ according to lgS=0.8762+0.698lgW. Assumed that the weight of young rats was 10 g, the body surface area was calculated as 0.00375m$^2$. According to the animal dose conversion formula: $D_{young\ rat}$ - $D_{infant} \times (W_{infant} \times S_{young\ rat}) / (W_{young\ rat} \times S_{infant})$ = 0.04 g/kg $\times$ (10$\times$0.00375) / (0.01$\times$0.466) = approximately 0.32 g/kg, the equivalent dose for rats (0.32g/kg) was used as the administration dose of Huo Xiang Zheng Qi capsule in this test.

The clinical dosage of ribavirin granules for children was 10-15 mg/kg/day, 3 doses a day, for 3 days. Assumed that the weight of infants was 10 kg, the body surface area was calculated as 0.466m$^2$ according to lgS=0.8762+0.698lgW. Supposed that the weight of young rats was 10 g, the body surface area was calculated as 0.00375m$^2$. According to the animal dose conversion formula: $D_{young\ rat} = D_{infant} \times (W_{infant} \times S_{young\ rat})/(W_{young\ rat} \times S_{infant})$ = 15 mg/kg $\times$ (10$\times$0.00375)/(0.01$\times$0.466) = 120.7 mg/kg, the equivalent dose for rats (120.7 mg/kg) was used as the administration dose of ribavirin granules in this test. The details of the dose design are shown in Table 7.

Table 7 Grouping and dose design

| Group | Dose (g crude drug/kg ) | Dose (g drug powder/kg ) | Concentration (g drug powder/mL) | Administration volume (mL/kg) | Multiples relative to the clinicall y intended dose. |
|---|---|---|---|---|---|
| Normal control group | - | - | - | 10 | - |
| Model control group | - | - | - | 10 | - |
| Huo Xiang Zheng Qi capsule group | 0.32 g/kg | - | 0.032 | 10 | 1 |
| Ribavirin granule group | 120.7 mg/kg | - | - | 10 | 1 |

(continued)

| Group | | Dose (g crude drug/kg ) | Dose (g drug powder/kg ) | Concentration (g drug powder/mL) | Administration volume (mL/kg) | Multiples relative to the clinically intended dose. |
|---|---|---|---|---|---|---|
| The pharmaceutical composition of the present invention | Low-dose group | 2.0 | 0.4 | 0.04 | 10 | 0.5 |
| | Medium-dose group | 4.0 | 0.9 | 0.09 | 10 | 1 |
| | High-dose group | 8.0 | 1.8 | 0.18 | 10 | 2 |

Note: Each g of drug powder contains 4.5 g of crude drug.

3.4 Data processing and statistical analysis

The experimental data was processed by removing outliers using the G-test, followed by rounding valid numbers according to rounding rules. The statistical analysis was conducted in accordance with the SOP. The software used for statistical analysis was SPSS 22.0. Measurement data was presented as mean $\pm$ standard deviation ($\bar{x}\pm s$). Levene's test was used to assess normality and homogeneity of variance. If there was no statistical significance (P > 0.05), the statistical analysis was performed by one-way ANOVA. If ANOVA indicated statistical significance (P $\leq$ 0.05), the comparative analysis was performed by LSD test (parametric method). If the variances were not homogeneous (P $\leq$ 0.05), the Kruskal-Wallis test was applied. If the Kruskal-Wallis test indicated statistical significance (P $\leq$ 0.05), the comparative analysis was performed by Dunnett's Test (non-parametric method). Frequency data were comparatively analyzed using rank-sum tests. The threshold for statistical significance was set at $\alpha$ = 0.05, wherein P $\leq$ 0.05 indicates a statistical significance and P $\leq$ 0.01 indicates a high statistical significance for difference tested.

4 Experimental results

4.1 Measurement of virus virulence

The result of virus titer detection was $TCID_{50}$= $10^{-3.34}$/100 μL.

4.2 Establishment of coxsackievirus infection model of young rats

As shown in Table 8, coxsackievirus infection via intraperitoneal injection mainly caused edema in the submucosa of the large intestine of young rats, accompanied by a small amount of inflammatory cell infiltration. Other tissues (stomach and small intestine) showed varying degrees of pathological changes, but these changes were relatively mild and did not exhibit a clear correlation with the infectious dose of $CVB_3$. No virus-related pathological changes were observed in the rest of the tissues (heart, liver, kidneys, brain and spleen). Therefore, the subsequent formal test used the coxsackievirus original solution to infect young SD rats by intraperitoneal injection. The stomach and intestinal tissues (mainly large intestine) were collected and subjected to detection of relevant indicators to reflect the infection status of the animals and the efficacy of the test substances.

Table 8 Results of pathological changes of coxsackievirus infection model of young SD rats

| Group | Vacuolar degeneration of epithelium of gastric mucosa | Vacuolar degeneration of epithelium of small intestinal mucosa (duodenum, ileum, jejunum) | Submucosal edema of large intestine, minimal inflammatory cell infiltration (colon, cecum, rectum) | Vacuolar degeneration of hepatocytes | Other organs (heart, kidney, brain and spleen) |
|---|---|---|---|---|---|
| Original solution group | 6/6 | 1/6 | 6/6 | 0/6 | 0/6 |

(continued)

| Group | Vacuolar degeneration of epithelium of gastric mucosa | Vacuolar degeneration of epithelium of small intestinal mucosa (duodenum, ileum, jejunum) | Submucosal edema of large intestine, minimal inflammatory cell infiltration (colon, cecum, rectum) | Vacuolar degeneration of hepatocytes | Other organs (heart, kidney, brain and spleen) |
|---|---|---|---|---|---|
| $10^{-1}$ group | 4/6 | 3/6 | 5/6 | 1/6 | 0/6 |
| $10^{-2}$ group | 5/6 | 0/6 | 2/6 | 1/6 | 0/6 |
| $10^{-3}$ group | 6/6 | 1/6 | 2/6 | 1/6 | 0/6 |
| $10^{-4}$ group | 2/4 | 0/4 | 2/4 | 0/4 | 0/4 |
| $10^{-5}$ group | 4/5 | 0/5 | 3/5 | 0/5 | 0/5 |
| $10^{-6}$ group | 4/4 | 0/4 | 3/4 | 2/4 | 0/4 |

4.3 Effect of the pharmaceutical compositions of the present invention on coxsackievirus $B_3$ infection model of young SD rats

4.3.1 Effects on viral load in tissue

As shown in Table 9, 3 days after the administration, the viral loads in the stomach, small intestine and large intestine tissues of rats in the model control group were significantly increased ($P \le 0.01$) compared to the normal control group. Compared to the model control group, the viral loads in the large intestine tissues in the medium- and high-dose groups of the pharmaceutical composition of the present invention were all significantly reduced ($P \le 0.05$), and the viral loads in the large intestine tissues in the Huo Xiang Zheng Qi capsule group and the ribavirin granule group were all significantly reduced ($P \le 0.05$ or $P \le 0.01$).

As shown in Table 10, on the next day after the last administration, compared to the normal control group, the viral loads in the stomach, small intestine and large intestine tissues of the rats in the model control group were all significantly increased ($P \le 0.05$ or $P \le 0.01$), and the viral loads in the stomach, small intestine and large intestine tissues of rats in other administration groups only showed a tendency to decrease, with no statistical difference.

Table 9 Effect of pharmaceutical composition of the present invention on viral load in gastrointestinal tissues of coxsackievirus infection model of young rats-3 days after administration ($\bar{x}\pm s$, n=6)

| Group | Dose (g crude drug/kg) | Stomach (Copies/mg) | Small intestine (Copies/mg) | Large intestine (Copies/mg) |
|---|---|---|---|---|
| Normal control group | - | 0.00±0.00 | 0.00±0.00 | 0.00±0.00 |
| Model control group | - | **118.09±84.74++** | **1063.60±847.12++** | **7958.44±8859.7 8++** |
| Huo Xiang Zheng Qi capsule group | 0.32 g/kg | 125.89±83.57 | 550.43±266.62 | **2137.71±2013.3 2*** |
| Ribavirin granule group | 120.7 mg/kg | 49.19±11.47 | 947.76±811.44 | **515.92±226.55**** |
| Low-dose group of the pharmaceutical composition of the present invention | 2.0 | 336.29±290.90 | 3492.44±4408.29 | 6050.77±5232.5 7 |
| Medium-dose group of the pharmaceutical composition of the present invention | 4.0 | 243.96±198.65 | 978.75±806.44 | **2648.59±2060.5 4** |

(continued)

| Group | Dose (g crude drug/kg) | Stomach (Copies/mg) | Small intestine (Copies/mg) | Large intestine (Copies/mg) |
|---|---|---|---|---|
| High-dose group of the pharmaceutical composition of the present invention | 8.0 | 157.22±181.02 | 1843.91±1680.04 | **1974.74±2377.2 6*** |

Note: Compared to normal control group, ++ indicates P ≤ 0.01; compared to the model control group, * indicates P ≤ 0.05, and ** indicates P ≤ 0.01.

Table 10 Effect of pharmaceutical composition of the present invention on viral load in gastrointestinal tissues of coxsackievirus infection model of young rats-the next day after last administration ($\bar{x}\pm s$, n=6)

| Group | Dose (g crude drug/kg) | Stomach (Copies/mg) | Small intestine (Copies/mg) | Large intestine (Copies/mg) |
|---|---|---|---|---|
| Normal control group | | 0.00±0.00 | 0.00±0.00 | 0.00±0.00 |
| Model control group | | **38.25±38.65++** | **295.96±108.99++** | **15183.24±14535.79+** |
| Huo Xiang Zheng Qi capsule group | 0.32g/kg | 62.43±55.39 | 153.92±184.75 | 2972.94±2620.10 |
| Ribavirin granule group | 120.7mg/kg | 104.75±74.10 | 308.03±198.76 | 16346.45±20084.56 |
| Low-dose group of the pharmaceutical composition of the present invention | 2.0 | 31.15±29.36 | 145.88±227.63 | 75883.08±98306.02 |
| Medium-dose group of the pharmaceutical composition of the present invention | 4.0 | 31.15±19.91 | 255.62±200.91 | 6523.10±5665.94 |
| High-dose group of the pharmaceutical composition of the present invention | 8.0 | 45.02±28.79 | 262.28±91.56 | 5402.97±2208.13 |

Note: ++ indicates P ≤ 0.01 and + indicates P ≤ 0.05 compared to normal control group.

4.3.2 Effects on histopathological changes

As shown in Table 11, 3 days after administration, the degree of lesions in colon, cecum and rectum tissues of the animals in the model control group was significantly aggravated compared to the normal control group (P ≤ 0.05). The degree of lesions in the colon tissue of the animals both in the medium-dose and high-dose groups of the pharmaceutical composition of the present invention was significantly reduced compared to the model control group (P ≤ 0.05).

On the next day after the last administration, the degree of lesions in the colon tissue of animals in the model control group was significantly aggravated compared to the normal control group (P ≤ 0.01). Compared to the model control group, the degree of lesions in the colon tissue of the animals in the high-dose group of the pharmaceutical composition of the present invention was significantly reduced (P ≤ 0.05), and the degree of lesions in the colon tissue of the animals in the Huo Xiang Zheng Qi capsule group was significantly reduced (P ≤ 0.05).

Although there were different degrees of gastric epithelial vacuolization lesions in the stomach tissues of animals in each group, gastric epithelial vacuolization was also observed in animals in the normal control group, it was considered to be a background change, which had no correlation with pathological changes caused by viruses and administration interventions.

Table 11 Effect of the pharmaceutical composition of the present invention on pathological changes of gastrointestinal tract in coxsackievirus infection model of young rats (n=10)

| Time | Group | Dose (g crude drug/kg) | Stomach | | | | Duodenum and jejunum | | | | Ileum and colon | | | | Cecum and rectum | | | |
|---|---|---|---|---|---|---|---|---|---|---|---|---|---|---|---|---|---|---|
| | | | - | + | ++ | P value | - | + | ++ | P value | - | + | ++ | P value | - | + | ++ | P value |
| 3 days after administration | Normal control group | - | 0 | 1 | 9 | - | 9 | 1 | 0 | - | 8 | 1 | 1 | - | 10 | 0 | 0 | - |
| | Model control group | - | 0 | 5 | 5 | >0.05 | 9 | 1 | 0 | >0.05 | 2 | 8 | 0 | ≤0.05 | 6 | 4 | 0 | ≤0.05 |
| | Huo Xiang Zheng Qi capsule group | 0.32 g/kg | 1 | 9 | 0 | ≤0.01 | 9 | 0 | 1 | >0.05 | 6 | 4 | 0 | >0.05 | 7 | 3 | 0 | >0.05 |
| | Ribavirin granule group | 120.7 mg/kg | 0 | 9 | 1 | >0.05 | 10 | 0 | 0 | >0.05 | 5 | 2 | 3 | >0.05 | 7 | 3 | 0 | >0.05 |
| | Low-dose group of the pharmaceutical composition of the present invention | 2.0 | 0 | 0 | 10 | ≤0.05 | 10 | 0 | 0 | >0.05 | 6 | 4 | 0 | >0.05 | 4 | 6 | 0 | >0.05 |
| | Medium-dose group of the pharmaceutical composition of the present invention | 4.0 | 1 | 5 | 4 | >0.05 | 10 | 0 | 0 | >0.05 | 7 | 3 | 0 | ≤0.05 | 6 | 4 | 0 | >0.05 |
| | High-dose group of the pharmaceutical composition of the present invention | 8.0 | 0 | 7 | 3 | >0.05 | 10 | 0 | 0 | >0.05 | 7 | 3 | 0 | ≤0.05 | 5 | 4 | 1 | >0.05 |
| On the next day after the last administration | Normal control group | - | 0 | 0 | 10 | | 10 | 0 | 0 | - | 10 | 0 | 0 | | 9 | 1 | 0 | |
| | Model control group | - | 0 | 3 | 7 | >0.05 | 9 | 0 | 1 | >0.05 | 4 | 6 | 0 | ≤0.01 | 5 | 5 | 0 | >0.05 |
| | Huo Xiang Zheng Qi capsule group | 0.32 g/kg | 0 | 7 | 3 | >0.05 | 10 | 0 | 0 | >0.05 | 9 | 1 | 0 | ≤0.05 | 5 | 5 | 0 | >0.05 |
| | Ribavirin granule group | 120.7 mg/kg | 5 | 5 | 0 | ≤0.01 | 10 | 0 | 0 | >0.05 | 5 | 2 | 3 | >0.05 | 3 | 5 | 2 | >0.05 |

| Time | Group | Dose (g crude drug/kg) | Stomach | | | | Duodenum and jejunum | | | | Ileum and colon | | | | Cecum and rectum | | | |
|---|---|---|---|---|---|---|---|---|---|---|---|---|---|---|---|---|---|---|
| | | | - | + | + + | P value | - | + | + + | P value | - | + | + + | P value | - | + | + + | P value |
| | Low-dose group of the pharmaceutical composition of the present invention | 2.0 | 0 | 0 | 1 0 | >0. 05 | 1 0 | 0 | 0 | >0. 05 | 7 | 3 | 0 | >0. 05 | 3 | 7 | 0 | >0 .0 5 |
| | Medium-dose group of the pharmaceutical composition of the present invention | 4.0 | **2** | **6** | **2** | ≤ **0.0 5** | 1 0 | 0 | 0 | >0. 05 | 6 | 4 | 0 | >0. 05 | 5 | 5 | 0 | >0 .0 5 |
| | High-dose group of the pharmaceutical composition of the present invention | 8.0 | **3** | **5** | **2** | ≤ **0.0 5** | 1 0 | 0 | 0 | >0. 05 | **1 0** | **0** | **0** | ≤ **0.0 1** | 4 | 6 | 0 | >0 .0 5 |

Note: + indicates $P \leq 0.05$, and ++ indicates $P \leq 0.01$ compared to normal control group. * indicates $P \leq 0.05$, and ** indicates $P \leq 0.01$ compared to model control group.

EP 4 653 008 A1

4.3.3 Effects on lymphocytes

As shown in Table 12, 3 days after the administration, the model control group showed a significant increase in the proportion of CD8[+] lymphocytes (P ≤ 0.01), a significant decrease in the ratio of CD4[+]/CD8[+] lymphocytes (P ≤ 0.01), and a tendency to decrease but with no statistically significant difference in the proportion of CD4[+] lymphocytes in the blood of animals compared to the normal control group. Compared to the model control group, the ratios of CD4[+]/CD8[+] lymphocytes in the blood of animals in the ribavirin granule group and Huo Xiang Zheng Qi capsule group were significantly increased (P ≤ 0.05 or P ≤ 0.01), the ratios of CD4[+]/CD8[+] lymphocytes in the blood of animals in the low-dose and medium-dose groups of the pharmaceutical composition of the present invention were significantly increased (P ≤ 0.05), the proportion of CD8[+] lymphocytes in the blood of animals in the high-dose group of the pharmaceutical composition of the present invention was significantly decreased (P ≤ 0.01), and the ratio of CD4[+]/CD8[+] lymphocytes in the high-dose group was significantly increased (P ≤ 0.01). On the next day after the last administration, the ratio of CD4[+]/CD8[+] lymphocytes in the blood of animals in the middle-dose group of the pharmaceutical composition of the present invention was significantly increased (P ≤ 0.05) compared to the model control group, and there was no significant difference in the other groups.

Table 12 Effect of the pharmaceutical composition of the present invention on lymphocytes in coxsackievirus infection model of young rats ($\bar{x}\pm s$, n=6)

| Group | Dose g crude drug/kg | % lymphocyte (3 days after administration) | | | % lymphocyte on the next day after the last administration | | |
|---|---|---|---|---|---|---|---|
| | | CD4+ | CD8+ | CD4+/CD8+ | CD4+ | CD8+ | CD4+/CD8+ |
| Normal control group | - | 28.82±3.51 | 13.49±2.84 | 2.18±0.28 | 18.59±3.29 | 9.50±1.16 | 1.95±0.24 |
| Model control group | - | 23.95±11.29 | **19.97±4.46++** | **1.16±0.32++** | 19.45±6.50 | 11.80±4.29 | 1.70±0.45 |
| Huo Xiang Zheng Qi capsule group | 0.32g/kg | 29.17±3.76 | 17.19±2.27 | **1.74±0.39*** | 18.64±5.63 | 9.70±1.10 | 1.91±0.49 |
| Ribavirin granule group | 120.7mg/kg | 33.31±8.66 | 20.52±5.91 | **1.65±0.18**** | 22.69±4.04 | 11.40±2.18 | 2.03±0.37 |
| Low-dose group of the pharmaceutical composition of the present invention | 2.0 | 32.98±5.65 | 21.42±3.54 | **1.59±0.43*** | 22.00±6.52 | 11.17±3.04 | 2.01±0.42 |
| Medium-dose group of the pharmaceutical composition of the present invention | 4.0 | 27.64±10.48 | 15.49±5.56 | **1.81±0.37*** | 19.93±4.50 | 8.52±1.91 | **2.37±0.35*** |
| High-dose group of the pharmaceutical composition of the present invention | 8.0 | 26.31±3.56 | **14.21±1.77**** | **1.87±0.28**** | 20.99±6.78 | 10.00±2.49 | 2.11±0.53 |

Note: ++ indicates P ≤ 0.01 compared to normal control group. * indicates P ≤ 0.05, and ** indicates P ≤ 0.01 compared to model control group.

4.3.4 Effects on cytokines in tissues

4.3.4.1 Effects on TNF-$\alpha$ and IFN-$\gamma$

As shown in Tables 13 and 14, 3 days after the administration, compared to the normal control group, the level of TNF-$\alpha$ factor in the large intestine tissue of rats in the model control group was significantly increased (P $\leq$ 0.01), and the level of IFN-$\gamma$ factor was significantly decreased (P $\leq$ 0.05). Compared to the model control group, the levels of TNF-$\alpha$ factor in the large intestine tissue of rats in the ribavirin granule group, and the low-, medium- and high- dose groups of the pharmaceutical composition of the present invention were all significantly decreased (P $\leq$ 0.05 or P $\leq$ 0.01), the levels of IFN-$\gamma$ factor in the large intestine tissue of rats in the middle- and high- dose groups of the pharmaceutical composition of the present invention were significantly increased (P $\leq$ 0.05), and the levels of TNF-$\alpha$ factor in the stomach tissue of rats in the low- and high- dose groups of the pharmaceutical composition of the present invention were significantly increased (P $\leq$ 0.05 or P $\leq$ 0.01). On the next day after the last administration, compared to the normal control group, the level of TNF-$\alpha$ factor in the large intestine tissue of rats in the model control group was significantly increased (P $\leq$ 0.01) and the level of IFN-$\gamma$ factor was significantly decreased (P $\leq$ 0.05). Compared to the model control group, the levels of TNF-$\alpha$ factor in the large intestine tissue of rats in the ribavirin granule group, the low-, medium- and high- dose groups of the pharmaceutical composition of the present invention were all significantly decreased (P $\leq$ 0.01), the levels of IFN-$\gamma$ factor in the large intestine tissue of rats in the Huo Xiang Zheng Qi capsule group, the ribavirin granule group, the low-, medium- and high- dose groups of the pharmaceutical composition of the present invention were all significantly increased (P $\leq$ 0.05 or P $\leq$ 0.01), and the levels of IFN-$\gamma$ factor in the stomach tissue of rats in the ribavirin granule group and the low-dose group of the pharmaceutical composition of the present invention were significantly decreased (P $\leq$ 0.05).

Table 13 Effect of the pharmaceutical composition of the present invention on TNF-$\alpha$ of coxsackievirus infection model of young rats ($\bar{x}\pm s$, n=8)

| Group | Dose (g crude drug/kg) | Stomach (pg/mg) | | Small intestine (pg/mg) | | Large intestine (pg/mg) | |
|---|---|---|---|---|---|---|---|
| | | D4 | D8 | D4 | D8 | D4 | D8 |
| Normal control group | - | 2290±81 | 2558±208 | 2827±239 | 3078±115 | 2648±148 | 2576±126 |
| Model control group | - | 2295±157 | 2468+177 | 2777±236 | 2899±292 | **2829±98++** | **2806±96++** |
| Huo Xiang Zheng Qi capsule group | 0.32 g/kg | 2350±214 | 2559±244 | 2841±326 | 2864±207 | 2860±70 | 2722±114 |
| Ribavirin granule group | 120.7 mg/kg | 2288±148 | 2731±96 | 2806±158 | 2698±145 | **2557±100\*\*** | **2491±151\*\*** |
| Low-dose group of the pharmaceutical composition of the present invention | 2 | **2560±77\*\*** | 2584±87 | 2725±188 | 2856±119 | **2602±110\*\*** | **2598±124\*\*** |
| Medium-dose group of the pharmaceutical composition of the present invention | 4 | 2275±217 | 2841±277 | 2755±240 | 2752±264 | **2714±80\*** | **2538±243\*\*** |
| High-dose group of the pharmaceutical composition of the present invention | 8 | **2502±159\*** | 2814±55 | 2838±172 | 2963±135 | **2556±87\*\*** | **2594±184\*\*** |

Note: ++ indicates P $\leq$ 0.01 compared to normal control group. * indicates P $\leq$ 0.05, and ** indicates P $\leq$ 0.01 compared to model control group.

Table 14 Effect of the pharmaceutical composition of the present invention on IFN-$\gamma$ of coxsackievirus infection model of young rats ($\bar{x}\pm s$, n=8)

| Group | Dose (g crude drug/kg) | Stomach (pg/mg) | | Small intestine (pg/mg) | | Large intestine (pg/mg) | |
|---|---|---|---|---|---|---|---|
| | | D4 | D8 | D4 | D8 | D4 | D8 |
| Normal control group | | 17395$\pm$902 | 19055$\pm$1032 | 16191$\pm$898 | 17533$\pm$788 | 19019$\pm$1037 | 19046$\pm$1269 |
| Model control group | | 17744$\pm$1481 | 18203$\pm$1213 | 16559$\pm$978 | 17093$\pm$1511 | **17502$\pm$645[+]** | **16779$\pm$2078[+]** |
| Huo Xiang Zheng Qi capsule group | 0.32g/kg | 18271$\pm$1455 | 19213$\pm$353 | 16063$\pm$1331 | 17900$\pm$735 | 18599$\pm$1735 | **19251$\pm$839[*]** |
| Ribavirin granule group | 120.7mg/kg | 18235$\pm$816 | **16481$\pm$1093[*]** | 16295$\pm$865 | 17443$\pm$1296 | 18364$\pm$1203 | **19728$\pm$926[**]** |
| Low-dose group of the pharmaceutical composition of the present invention | 2 | 18473$\pm$1220 | **16465$\pm$2041[*]** | 16348$\pm$1587 | 18273$\pm$1378 | 18039+1357 | **20695$\pm$1661[**]** |
| Medium-dose group of the pharmaceutical composition of the present invention | 4 | 18463$\pm$1708 | 17286$\pm$1373 | 16255$\pm$1710 | 17444$\pm$1336 | **18802$\pm$1206[*]** | **20786$\pm$2306[**]** |
| High-dose group of the pharmaceutical composition of the present invention | 8 | 18502$\pm$440 | 17850$\pm$797 | 17007$\pm$739 | 17988$\pm$1346 | **18884$\pm$930[*]** | **21131$\pm$797[**]** |

Note: + indicates P $\leq$ 0.05 compared to normal control group. * indicates P $\leq$ 0.05, and ** indicates P $\leq$ 0.01 compared to model control group.

EP 4 653 008 A1

4.3.4.2 Effect on IL-10 and IL-1β in tissues

As shown in Tables 15 and 16, 3 days after the administration, the level of IL-1β factor in the small intestine tissue of rats in the high-dose group of the pharmaceutical composition of the present invention was significantly increased compared to the model control group (P ≤ 0.05). On the next day after the last administration, compared to the model control group, the levels of IL-10 and IL-1β factors in the stomach tissue of rats in the high-dose group of the pharmaceutical composition of the present invention were significantly increased (P ≤ 0.05 or P ≤ 0.01), and the levels of IL-10 and IL-1β factors in the small intestinal tissue of rats in the low-dose group of the pharmaceutical composition of the present invention were significantly increased (P ≤ 0.05 or P ≤ 0.01), the level of IL-1β factor in the small intestinal tissue of rats in the medium-dose group of the pharmaceutical composition of the present invention was significantly increased (P ≤ 0.05), and the level of IL-10 factor in the large intestinal tissue of rats in the middle-dose group of the pharmaceutical composition of the present invention was significantly increased (P ≤ 0.05).

Table 15 Effect of the pharmaceutical composition of the present invention on IL-10 of coxsackievirus infection model of young rats ($\bar{x}\pm s$, n=8)

| Group | Dose (g crude drug/kg) | Stomach (pg/mg) | | Small intestine (pg/mg) | | Large intestine (pg/mg) | |
|---|---|---|---|---|---|---|---|
| | | D4 | D8 | D4 | D8 | D4 | D8 |
| Normal control group | - | 545±27 | 536±3 6 | 477±41 | 497±2 3 | 478±11 | 510±45 |
| Model control group | - | 512±30 | 523±3 0 | 479±32 | 480±4 0 | 495±14 | 485±25 |
| Huo Xiang Zheng Qi capsule group | 0.32g/kg | 525±41 | 543±1 4 | 483±38 | 502±3 3 | 479±28 | 522±38 |
| Ribavirin granule group | 120.7mg/kg | 509±37 | 509±1 9 | 473±23 | 485±2 7 | 497±28 | 496±50 |
| Low-dose group of the pharmaceutical composition of the present invention | 2.0 | 541±26 | 495±2 3 | 464±32 | **527±2 8**[*] | 483±28 | 534±38 |
| Medium-dose group of the pharmaceutical composition of the present invention | 4.0 | 534±41 | 524±4 3 | 466±39 | 515±5 5 | 488±41 | **545±60**[*] |
| High-dose group of the pharmaceutical composition of the present invention | 8.0 | 535±22 | **566±3 7**[**] | 495±26 | 459±2 8 | 511±24 | 523±74 |

Note: * indicates P ≤ 0.05, and ** indicates P ≤ 0.01 compared to model control group.

Table 16 Effect of the pharmaceutical composition of the present invention on IL-1β of coxsackievirus infection model of young rats ($\bar{x}\pm s$, n=8)

| Group | Dose (g crude drug/kg) | Stomach (pg/mg) | | Small intestine (pg/mg) | | Large intestine (pg/mg) | |
|---|---|---|---|---|---|---|---|
| | | D4 | D8 | D4 | D8 | D4 | D8 |
| Normal control group | - | 281±23 | 304±39 | 318±24 | 342±7 | 307±17 | 336±30 |
| Model control group | - | 285±11 | 292±14 | 311±11 | 323±24 | 311±20 | 310±22 |
| Huo Xiang Zheng Qi capsule group | 0.32 g/kg | 291±23 | 304±16 | 313±24 | 332±19 | 304±29 | 323±19 |
| Ribavirin granule group | 120.7 mg/kg | 291±20 | 308±24 | 318±23 | 340±21 | 317±18 | 320±34 |
| Low-dose group of the pharmaceutical composition of the present invention | 2 | 300±25 | 302±25 | 312±17 | **377±25**[**] | 301±25 | 344±20 |

(continued)

| Group | Dose (g crude drug/kg) | Stomach (pg/mg) | | Small intestine (pg/mg) | | Large intestine (pg/mg) | |
|---|---|---|---|---|---|---|---|
| | | D4 | D8 | D4 | D8 | D4 | D8 |
| Medium-dose group of the pharmaceutical composition of the present invention | 4 | $286\pm22$ | $299\pm29$ | $317\pm19$ | $\mathbf{351\pm22}$[*] | $303\pm24$ | $337\pm46$ |
| High-dose group of the pharmaceutical composition of the present invention | 8 | $304\pm14$ | $\mathbf{351\pm22}$[*] | $\mathbf{331\pm10}$[*] | $344\pm27$ | $311\pm22$ | $353\pm27$ |

Note: * indicates $P \leq 0.05$, and ** indicates $P \leq 0.01$ compared to model control group.

Coxsackievirus, classified into enterovirus, is a member of the family Picornaviridae. Based on serotypes, Coxsackieviruses can be divided into two subgroups: Coxsackievirus A (serotypes 1-22 and 24) and Coxsackievirus B (serotypes 1-6). Among them, coxsackievirus $B_3$ ($CVB_3$) has the characteristics of enteric disease and readily replicates in human intestinal cells. After entering the bloodstream, it can reach the central nervous system or other target organs, resulting in a wide range of clinical manifestations that vary widely in severity, with latent and mild infections being the majority. Coxsackievirus is mainly spread via the fecal-oral route (digestive tract route), and may also be spread via secretions from respiratory tract after sneezing or coughing. Coxsackievirus infection can occur in human at any age, and young children are more susceptible than adults. The carrier rate of coxsackievirus among young children reaches 5% to 50%. The course of related diseases generally follows an incubation period of 2-3 days and a peak period of 3-5 days, with most cases self-resolving within about a week. However, a small number of infections can lead to severe consequences, and the pathogenesis after infection is still unclear. According to relevant literature, after coxsackievirus B3 infection, the virus will invade NK cells and $CD4^+$ T cells, leading to immune system imbalance and causing a decrease in the number of NK cells and the ratio of $CD4^+/CD8^+$ T lymphocytes. Moreover, the T lymphocyte-mediated cellular immune response triggered by the viral invasion will further increase the number of $CD8^+$ T cells, causing a further reduction in the $CD4^+/CD8^+$ ratio. The significant increase in the level of $CD8^+$ T cell will aggregate and activate macrophages, which are polarized to secrete large amounts of tumor necrosis factor and causing interferon release from NK cells, thereby killing infected cells and inhibiting viral replication and reproduction.

This study used the young SD rat model established by intraperitoneal injection of $CVB_3$ into the body, and the results showed that the viral replication caused oedema and inflammatory cell infiltration in the submucosa of the colon, cecum, and rectum of young rats in the model control group after coxsackievirus infection, leading to an increase in the proportion of $CD8^+$ lymphocytes in the blood and a decrease in the ratio of $CD4^+/CD8^+$ lymphocytes, resulting in high expression of TNF-$\alpha$ and low expression of IFN-$\gamma$ in the large intestine tissue. After the young rates were infected with $CVB_3$, the changes in immune cells and related cytokines (TNF-$\alpha$ and IFN-$\gamma$) in young rats were basically consistent with those reported in the literature.

After intervention with different doses of the pharmaceutical composition of the present invention via gavage, 3 days after the administration, the result showed that the medium- and high- dose (4.0 and 8.0 g crude drug/kg) of the pharmaceutical composition of the present invention can significantly inhibit the replication of coxsackievirus in the large intestine tissues. For the pharmaceutical composition of the present invention, the medium- and high- dose (4.0 and 8.0 g crude drug/kg) can significantly reduce the degree of colonic lesions in the model animals, the low- and medium- dose (2.0 and 4.0 g crude drug/kg) can significantly increase the ratio of $CD4^+/CD8^+$ lymphocytes in the blood of the model animals, the high-dose (8.0 g of crude drug/kg) can significantly decrease the proportion of $CD8^+$ lymphocytes and increase the ratio of $CD4^+/CD8^+$ lymphocytes in the blood of the model animals, and the low-, medium-, and high-dose (2.0, 4.0, and 8.0 g of crude drug/kg) can significantly decrease the level of TNF-$\alpha$ in the large intestine tissue of the model animals, and the medium- and high- dose (4.0 and 8.0 g of crude drug/kg) can significantly increase the level of IFN-$\gamma$ in the large intestine tissues of the model animals. On the next day after the last administration, the results showed that for the pharmaceutical composition of the present invention, the high-dose (8.0 g crude drug/kg) can significantly reduce the degree of colonic lesions in the model animals, the medium-dose (4.0 crude drug/kg) can significantly increase the ratio of CD4+/CD8+ lymphocytes in the blood of the model animals, and the low-, medium-, and the high-dose (2.0, 4.0, and 8.0 g crude drug/kg) can significantly reduce the TNF-$\alpha$ level and increase IFN-$\gamma$ level in the large intestine tissue of the model animals. The above results showed that the pharmaceutical composition of the present invention can significantly inhibit coxsackievirus replication in the large intestine tissue and increase the ratio of CD4+/CD8+ lymphocyte and IFN-$\gamma$ content, suggesting that its mechanism of action may be to inhibit viral reproduction and maintain the balance of immune cells. Huo Xiang Zheng Qi capsule is a classic antiviral traditional Chinese medicine, in which various components act cooperatively. Therefore it can inhibit viral reproduction mainly through a variety of pathways, and improve the immunity to achieve the therapeutic purpose. The results show that Huo Xiang Zheng Qi capsule can inhibit coxsackievirus replication in the large intestine tissues, and can increase the ratio of CD4[+]/CD8[+] lymphocytes, suggesting that its mechanism of action may be similar to the pharmaceutical composition of the present invention. The main antiviral mechanism of ribavirin is to inhibit the replication and reproduction of the virus[12]. As can be seen, 3 days after the administration of ribavirin, this group showed a significant decrease in viral load in the large intestine tissue and an effective recovery of CD4[+] lymphocytes due to the inhibition effect on viral replication, and the ratio of CD4[+]/CD8[+] lymphocytes was increased, the inflammation reactions were alleviated, and the symptoms were relieved.

In summary, the pharmaceutical composition of the present invention has the effect of inhibiting the replication of coxsackievirus $B_3$ in the large intestine tissue of young rats, regulating the balance of immune cells, and reducing local tissue inflammation.

Test Example 3

Test Purpose

Evaluation of effect of the pharmaceutical composition of the present invention on intestinal propulsion in normal mice

1 Experimental materials

1.1 Test products

1.1.1 Name: The pharmaceutical composition of the present invention (test product number: GLPTN-2108)

1.1.2 Characteristics: The product is a yellowish-brown powder mixed with white inclusion substances and is slightly fragrant.

1.1.3 Functions and Indications: It is mainly used for the syndrome of summer-heat-dampness due to cold and the syndrome of stagnant and jamming dampness-heat due to influenza.

1.1.4 Clinical dosage and administration route: 0.5 g of crude drug/kg body weight, oral administration.

1.1.5 Content: 4.5 g of crude drug/g drug powder.

1.1.6 Source: Shijiazhuang Yiling Pharmaceutical Co., Ltd. Batch number: 211101.

1.1.7 Storage condition: sealed.

1.1.8 Expiration date: tentatively set until November 09, 2023.

1.2 Positive drugs and main reagents

1.2.1 Positive drugs: Huo Xiang Zheng Qi Capsule: releasing the exterior and transforming dampness, regulating qi and harmonizing the middle jiao. It is used for cold due to external wind invasion and internal dampness stagnation, manifested as headache and dizziness, chest congestion, fullness in the chest and diaphragm, abdominal distension and pain, vomiting, and diarrhea. Each capsule contains 0.3 g. Oral administration of 4 capsules twice daily. Manufacturer: Jiangxi Yuanjian Pharmaceutical Co. Ltd. Batch Number: 210408. Expiration date: March 2023.

1.2.2 Main reagents

1.2.2.1 Sterile water for injection: Shijiazhuang No. 4 Pharmaceutical Co., Ltd. Batch Number: 2009053201. Expiration date: 2022.09.04.

1.2.2.2 Dextran Blue 2000 (DB-2000): Solarbio. Batch NO. 528E053. Expiration date: 2026.10.30.

1.2.2.3 Carboxymethyl cellulose sodium (CMC-Na): Tianjin Damao Chemical Reagent Factory. Batch Number: 20200525. Expiration date: 2022.05.24.

1.2.2.4 Distilled water: Guangzhou Watson's Food & Beverage Co., Ltd. Batch Number: 20210417. Expiration date: 2022.10.16.

1.3 Experimental system

1.3.1 Animal Strain: ICR mice.

1.3.2 Animal Grade: SPF grade.

1.3.3 Animal Gender and Quantity: A total of 60 animals were purchased, including 30 males and 30 females. All 60 animals (30 males and 30 females) were used in the test.

1.3.4 Animal Age: Approximately 3-4 weeks old at the time of receipt.

1.3.5 Animal Weight: The intended weight range of animals was 18-22 g. The actual weight range of animals at the time of receipt was 17.0-22.0 g, and the weight range of animals after bred was 21.5-30.6 g when administered.

1.3.6 Animal Source: Purchased from Beijing Vital River Laboratory Animal Technology Co., Ltd. Certificate numbers: 110011210114499974, 110011210114500043. License number: SCXK (Beijing) 2021-0006. Receipt date: December 8, 2021.

1.3.7 Feeding Conditions: Mice were reared in cages at the Innovative Drug Evaluation Center of Hebei Research Institute of Integrated Traditional Chinese and Western Medicine. License Number: SYXK (Hebei) 2020-006. Lighting: 12 h/day. Temperature: 20-26°C. Relative Humidity: 40%-70%.

1.3.8 Quarantine Process: Animals were subjected to adaptive feeding for 5 days, during which body weight was measured daily. During this period, the animals had normal water and food intake, and good health status, with no signs of illness or mortality.

1.3.9 Feed: Rat & mice maintenance feed (SPF-grade) provided by Beijing Keao Xieli Feed Co., Ltd. Feed Production License Number: Beijing Feed Certificate (2018) 06073. Batch Number: 21103213. Certificate Number: 1112622100055093. Production Date: October 11, 2021, Shelf Life: 6 months. Experimental Animal Production License Number: SCXK (Beijing) 2019-0003.

1.3.10 Water: Clean water prepared by the ROB-B drinking water system for laboratory animal was provided, and filled in water bottles for free drinking. The drinking water was detected annually for all indicators, including mainly microbiological indicators, toxicological indicators, sensory characteristics, and general chemical parameters, commissioned to a qualified institution for detection. Microbial limit detection was performed every 2 months, commissioned to the Quality Control Department of Shijiazhuang Yiling Pharmaceutical Co., Ltd. The detection reports were stored by the animal department and archived annually.

1.3.11 Bedding: Corn cob bedding (common grade) provided by Beijing Keao Xieli Feed Co., Ltd., was sterilized by high temperature and high pressure before use. Batch Number: 21109811, Production Date: October 12, 2021. Shelf Life: 6 months. Experimental Animal Production License Number: SCXK (Beijing) 2020-0010.

1.3.12 Marking: Mice were marked with ear tags, provided by Globalebio (Beijing) Technology Co., Ltd. Product number: GERM-02.

2 Test methods

2.1 Test design basis

2.1.1 Adopted standards were determined according to the *Registration, Classification and Submission Data Requirements for Chinese Traditional Medicine and Natural Drugs* issued by the State Food and Drug Administration, *Good Laboratory Practice for non-clinical laboratory studies, Experimental Methodology of*

*Pharmacology* (3rd edition) and related literatures.

2.1.2 Description of selected experimental system: By gastric emptying and intestinal propulsion rate tests, the effect of drugs on the gastrointestinal motor function can be observed. Dextran blue 2000 (DB-2000), which has a low absorption rate in the gastrointestinal tract and a high recovery rate, was selected as the gastrointestinal tract indicator.

2.2 Dosage and grouping

Sixty ICR mice were randomly grouped into five groups according to their body weight, including the model group, the Huo Xiang Zheng Qi group, and the low-, the medium-, and the high- dose groups of the pharmaceutical composition of the present invention. Based on that the clinical dose of the pharmaceutical composition of the present invention was 0.5 g of crude drug/kg body weight, the medium-dose of the pharmaceutical composition of the present invention for mice was set as 10 times the clinical dose, i.e., 5.00 g of crude drug/kg, which was considered as the clinically equivalent amount. The dose interval was set at 2 times, and therefore the low- and high- doses of the pharmaceutical composition of the present invention for mice were 2.50 and 10.00 g of crude drug/kg, respectively. The content of the pharmaceutical composition of the present invention in drug powder was 4.5 g of crude drug/g drug powder. Thus the low-, medium- and high- dose of the pharmaceutical composition of the present invention were 0.56, 1.11 and 2.22 g of drug powder/kg, respectively. Referring to the previous studies in our laboratory and relevant literature, the dose of Huo Xiang Zheng Qi drug administered to mice was set at 0.40 g/kg, as shown in Table 17.

Table 17 Grouping and dose setting in test studying effect of the pharmaceutical composition of the present invention on intestinal propulsion in mice

| Group | Numbe r of animals | Dose (g crude drug/kg) | Dose (g drug powder/kg) | Concentration (g drug powder/ml) |
|---|---|---|---|---|
| Model group | 12 | - | - | - |
| Huo Xiang Zheng Qi group | 12 | - | 0.40 | 0.040 |
| Low-dose group of the pharmaceutical composition of the present invention | 12 | 2.50 | 0.56 | 0.056 |
| Medium-dose group of the pharmaceutical composition of the present invention | 12 | 5.00 | 1.11 | 0.111 |
| High-dose group of the pharmaceutical composition of the present invention | 12 | 10.00 | 2.22 | 0.222 |

2.3 Administration method

The drug was administered via gavage, consistent with the clinical administration route.

2.4 Preparation and storage of test product

Approximately 0.5600g (low dose), 1.1100g (medium dose), and 2.2200g (high dose) of the pharmaceutical composition of the present invention were accurately weighed and ground with sterile water for injection, respectively. The volume was adjusted to a final volume of 10 ml. For Huo Xiang Zheng Qi capsules, one capsule was taken, ground with sterile water for injection, and adjusted to a final volume of 7.5 ml. All test products were prepared just before use.

2.5 Administration of test product

A disposable syringe was used to administer the drug at a dosing volume of 10 ml/kg. Animals in the model group were administered with an equivalent volume of the solvent. This dosing was performed continuously for 5 days.

2.6 Steps and observation indicators

Indicator Preparation: A 4% DB-2000 mixture was prepared using 2% CMC-Na solution.
Newly received experimental animals were numbered, adaptively fed for at least 5 days, and grouped as described previously. Before the final dosing, food fasting (not water) was carried out for at least 16 h. About 60 min after the final dosing, each animal in all groups was administered with 0.2 ml of DB-2000 via gavage. About 30 min later, the animals were scarified by cervical dislocation. The abdominal cavity was immediately opened to separate the mesentery, and the intestinal tract from the upper end (pylorus) to the lower end (ileocecal junction) was taken by cutting and placed on a tray. The small intestine was gently straightened, and two distances, from the pylorus to the leading edge of the pigment and from the pylorus to the ileocecal junction, were measured. The ratio of these two distances was calculated as the propulsion rate of small intestine. The whole stomach was quickly excised by cutting, and the stomach was open by cutting along the greater curvature. The residual DB-2000 inside the stomach was thoroughly washed into 4ml of deionized water. The resulting mixture was centrifuged at 3500 rpm for 15 min, and the supernatant was collected. The absorbance was measured at 620 nm using a microplate reader.

Pigment residue level in stomach was assessed by comparing absorbance values of each group.

Propulsion rate of small intestine (%) = (propulsion distance of indicator/ the full length of small intestine) $\times$ 100%

Evaluation Criteria: A reduction in pigment residue and an increase in propulsion rate indicated that the test product has a promoting effect on gastric emptying and intestinal motility, conversely, an inhibitory effect on gastric emptying and intestinal motility was indicated.

2.7 Notification of relevant staff

The animal department should be notified when animals were purchased. The abnormal event of animals should be reported to the pathology department for processing.

2.8 Instrument system

JJ2000 electronic balance from Changshu Shuangjie Testing Instrument Factory

NVT2201ZH electronic balance (OHAUS)

YP-20002 electronic balance (Shanghai Yue Ping)

BSA2201 Electronic Balance (Sartorius)

SYNERGY neo2 microplate reader (BioTeK)

BT224S precision analytical balance (Sartorius)

High Speed Freezing Centrifuge 1580R (Genespeed)

2.9 Statistical methods

The experimental data were analyzed and processed using statistical software SPSS 20.0. The results were presented as mean $\pm$ standard deviation ($\pm$s). A normality test was performed firstly. For data that followed a normal distribution, means were compared by one-way ANOVA. If the variances were homogeneous, pairwise comparisons were performed using the Least Significant Difference (LSD) method. If the variances were not homogeneous, Dunnett's T3 test was used for pairwise comparisons. For data that did not follow a normal distribution, non-parametric tests were used.

3 Results

As shown in Table 18, compared to the model group, the propulsion rate of small intestine in the medium dose group of the pharmaceutical composition of the present invention was significantly decreased (P<0.05), and the pigment residue level in stomach (absorbance) in this group was significantly increased (P<0.05). Compared to the model group, the propulsion rate of small intestine in the high dose group of the pharmaceutical composition of the present invention was significantly decreased (P<0.05), and the pigment residue level in stomach (absorbance) in this group was significantly increased (P<0.05). Compared to the model group, the propulsion rates of small intestine in the Huo Xiang Zheng Qi group and the low dose group of the pharmaceutical composition of the present invention were decreased, and the pigment residue levels in stomach (absorbance) in the two groups were increased, but no significant differences were observed (P>0.05).

Table 18 Results of test studying effect of the pharmaceutical composition of the present invention on the propulsion rate of small intestine ($\bar{x}\pm s$)

| Group | Number of animals | % Propulsion rate of small intestine | Absorbance |
|---|---|---|---|
| Model group | 12 | 57.66±7.63 | 0.04±0.03 |
| Huo Xiang Zheng Qi group | 12 | 49.67±12.96 | 0.07±0.05 |
| Low-dose group of the pharmaceutical composition of the present invention | 12 | 53.60±10.69 | 0.11±0.10 |
| Medium-dose group of the pharmaceutical composition of the present invention | 12 | 49.46±4.73* | 0.07±0.05* |
| High-dose group of the pharmaceutical composition of the present invention | 12 | 43.78±10.27* | 0.15±0.13* |

Note: * indicates P<0.05 compared to the model group.

4 Conclusion

The pharmaceutical composition of the present invention had the effect of inhibiting gastric emptying and intestinal motility under the conditions of this test.

5 Discussion

Currently, indicators such as Dextran Blue 2000 (DB-2000), Evans Blue, and ink are used to assess the propulsion of small intestine in mice. Dextran Blue 2000 (DB-2000) was selected as the indicator in the gastrointestinal tract due to its low absorption rate in the gastrointestinal tract and high recovery rate.

The basic principle of this experiment involves administering a certain volume and concentration of Dextran Blue 2000 (DB-2000) into the stomachs of normal mice via gavage. After 30 min, the pigment residue level of Dextran Blue 2000 (DB-2000) in the stomach was assessed by measuring absorbance at 620 nm using a microplate reader and the propulsion rate of small intestine was calculated according to its propulsion distance in the small intestine.

Under the conditions of this test, the pharmaceutical composition of the present invention significantly increased the pigment residue level (absorbance) in stomach and decreased the propulsion rate of small intestine, inhibiting gastric emptying and intestinal motility in normal mice.

Test Example 4

Test Purpose

Evaluation of effect of the pharmaceutical composition of the present invention on diarrhea model of mice induced by gavage of *sennae folium* decoction

1 Experimental material

1.1 Test products

1.1.1 Name: The pharmaceutical composition of the present invention (test product number: GLPTN-2108)

1.1.2 Characteristics: The product is a yellowish-brown powder mixed with white inclusion substances and is slightly fragrant.

1.1.3 Functions and Indications: It is mainly used for the syndrome of summer-heat-dampness due to cold and the syndrome of stagnant and jamming dampness-heat due to influenza.

1.1.4 Clinical Dosage and Administration Route: 0.5 g of crude drug/kg body weight, oral administration.

1.1.5 Content: 4.5 g of crude drug/g drug powder.

1.1.6 Source: Shijiazhuang Yiling Pharmaceutical Co., Ltd. Batch number: 211101.

1.1.7 Storage condition: sealed.

1.1.8 Expiration date: tentatively set until November 09, 2023.

1.2 Positive drugs and main reagents

1.2.1 Positive drugs: Huo Xiang Zheng Qi Capsule: releasing the exterior and transforming dampness, regulating qi and harmonizing the middle jiao. It is used for cold due to external wind invasion and internal dampness stagnation, manifested as headache and dizziness, chest congestion, fullness in the epigastrium, abdominal distension and pain, vomiting, and diarrhea. Each capsule contains 0.3 g. 4 capsules for oral administration twice daily. Manufacturer: Jiangxi Yuanjian Pharmaceutical Co. Ltd. Batch Number: 210408. Expiration date: March 2023.

1.2.2 Main reagents

1.2.2.1 Sterile water for injection: Shijiazhuang No. 4 Pharmaceutical Co., Ltd. Batch Number: 2009053201. Production date: 2022.09.05. Expiration date: 2022.09.04.

1.2.2.2 Distilled water: Guangzhou Watson's Food & Beverage Co., Ltd. Batch Number: 20210731. Production date: 2021.07.31. Expiration date: 2023.01.30.

1.2.2.3 *Sennae folium*: Hebei Linshishengtai Pharmaceutical Co. Ltd. Batch Number: 2009010841. Production date: 2020.09.19. Expiration date: 2023.09.18.

1.3 Experimental system

1.3.1 Animal Strain: ICR mice.

1.3.2 Animal Grade: SPF grade.

1.3.3 Animal Gender and Quantity: A total of 80 animals were purchased, including 40 males and 40 females. All 72 animals (36 males and 36 females) were used in the test.

1.3.4 Animal Age: Approximately 3-4 weeks old at the time of receipt.

1.3.5 Animal Weight: The intended weight range for animals to be purchased was 18-22 g. The actual weight range of animals at the time of receipt was 18.3-22.4 g, and the weight range of animals after breeding was 21.4-31.0 g when administered.

1.3.6 Animal Source: The animals were purchased from Beijing Vital River Laboratory Animal Technology Co., Ltd. Certificate numbers: 110011221104102927, 110011221104103022. License number: SCXK (Beijing) 2021-0006. Receipt date: April 20, 2022.

1.3.7 Feeding Conditions: Mice were reared in cages at the Innovative Drug Evaluation Center of Hebei Research Institute of Integrated Traditional Chinese and Western Medicine. License Number: SYXK (Hebei) 2020-006. Lighting: 12 h/day. Temperature: 20-26°C. Relative Humidity: 40%-70%.

1.3.8 Quarantine Process: Animals were subjected to adaptive feeding for 5 days, during which body weight was measured daily. During this period, the animals had normal water and food intake, and good health status, with no signs of illness or mortality.

1.3.9 Feed: Rat & mice maintenance feed (SPF-grade) was provided by Beijing Keao Xieli Feed Co., Ltd. Feed Production License Number: Beijing Feed Certificate (2018) 06073. Batch Number: 22013213. Certificate Number: 1112622200080198. Production Date: 2022.01.02. Shelf Life: 6 months. Experimental

**EP 4 653 008 A1**

Animal Production License Number: SCXK (Beijing) 2019-0003.

1.3.10 Water: Clean water for drinking was prepared using the ROB-B drinking water system for laboratory animal, and filled in water bottles for free drinking. The drinking water was detected annually for all indicators, including mainly microbiological indicators, toxicological indicators, sensory characteristics, and general chemical parameters, commissioned to a qualified institution for detection. Microbial limit detection was performed every 2 months, commissioned to the Quality Control Department of Shijiazhuang Yiling Pharmaceutical Co., Ltd. The detection reports were stored by the animal department and archived annually.

1.3.11 Bedding: Corn cob bedding (common grade) provided by Beijing Keao Xieli Feed Co., Ltd. The bedding was sterilized by high temperature and high pressure before use. Batch Number: 22019811. Production Date: 2022.01.04. Shelf Life: 6 months. Experimental Animal Production License Number: SCXK (Beijing) 2020-0010.

1.3.12 Marking: Mice were marked with the animal marking pen (EPS, Japan). Product number: AM1.

2 Test methods

2.1 Test design basis

2.1.1 Adopted standards were determined according to the *Registration, Classification and Submission Data Requirements for Chinese Traditional Medicine and Natural Drug* issued by the State Food and Drug Administration, *Good Laboratory Practice for non-clinical laboratory studies, Experimental Methodology of Pharmacology* (3rd edition) and related literatures.

2.1.2 Description of selected experimental system: *Sennae folium* is a mild laxative that acts on the large intestine, causing moderate diarrhea with loose stools that have no abnormal odor. This laxative achieves optimal effects while offering advantages such as low residue and high safety. Therefore, it is often used as one of the experimental methods for evaluating antidiarrheal drugs.

2.2 Dosage and Grouping

72 ICR mice were randomly grouped into 6 groups according to their body weight, including the normal group, the model group, the Huo Xiang Zheng Qi group, and the low-, the medium-, and the high- dose groups of the pharmaceutical composition of the present invention. Based on that the clinical dose of the pharmaceutical composition of the present invention was 0.5 g of crude drug/kg body weight, the medium-dose of the pharmaceutical composition of the present invention for mice was set as 10 times the clinical dose, i.e., 5.00 g of crude drug/kg, which was considered as the clinically equivalent amount. The dose interval was set at 2 times, and therefore the low- and high- doses of the pharmaceutical composition of the present invention for mice were 2.50 and 10.00 g of crude drug/kg, respectively. The content of the pharmaceutical composition of the present invention in drug powder was 4.5 g of crude drug/g drug powder. The low-, medium- and high- dose of the pharmaceutical composition of the present invention were 0.56, 1.11 and 2.22 g of drug powder/kg, respectively. Referring to the previous studies in our laboratory and specification, the dose of Huo Xiang Zheng Qi drug administered to mice was set at 0.40 g/kg, as shown in Table 19.

Table 19 Grouping and dose setting in test studying effect of the pharmaceutical composition of the present invention on diarrhea in mice caused by *sennae folium*

| Group | Number of animals | Dose (g crude drug/kg) | Dose (g drug powder/kg) | Concentration (g drug powder/ml) |
|---|---|---|---|---|
| Normal group | 12 | - | - | - |
| Model group | 12 | - | - | - |
| Huo Xiang Zheng Qi group | 12 | - | 0.40 | 0.040 |
| Low-dose group of the pharmaceutical composition of the present invention | 12 | 2.50 | 0.56 | 0.056 |
| Medium-dose group of the pharmaceutical composition of the present invention | 12 | 5.00 | 1.11 | 0.111 |

(continued)

| Group | Number of animals | Dose (g crude drug/kg) | Dose (g drug powder/kg) | Concentration (g drug powder/ml) |
|---|---|---|---|---|
| High-dose group of the pharmaceutical composition of the present invention | 12 | 10.00 | 2.22 | 0.222 |

2.3 Administration Method

The drug was administered via gavage, consistent with the clinical administration route.

2.4 Preparation and storage of test product

Approximately 0.5600 g (low dose), 1.1100 g (medium dose), and 2.2200 g (high dose) of the pharmaceutical composition of the present invention were accurately weighed and ground with sterile water for injection, respectively. The volume was adjusted to a final volume of 10 ml. For Huo Xiang Zheng Qi capsules, two capsules were taken, ground with sterile water for injection, and adjusted to a final volume of 15 ml. All test products were prepared just before use.

2.5 Administration of test product

A disposable syringe was used to administer the drug at a dosing volume of 10 ml/kg. Animals in the normal group and the model group were administered with a corresponding volume of the solvent. This dosing was performed continuously for 5 days.

2.6 Steps and observation indicators

Preparation of modeling drug: 40 g of dried *sennae folium* was weighed. It was soaked in distilled water (100°C) at a 1:10 material-liquid ratio for 20 min and filtered. This process was repeated 3 times. The 3 filtrates were combined, concentrated under reduced pressure to a certain volume using a rotary evaporator, and added with cold distilled water to prepare the *sennae folium* decoction (containing 1 g of crude drug/mL), for later use.

Newly received animals were numbered, adaptively fed for at least 5 days and grouped as previously described. Sixty min after the last administration, except for the normal group, animals in each group were given the *sennae folium* decoction (15 ml/kg) via gavage to establish the model. Animals in each group were observed and recorded for the stool amounts (number of dry stools and number of loose stools), whether occurring diarrhea (indicated by the occurrence of unshaped loose stools), and onset time of diarrhea within 6 h. After 6 h, the diarrhea rate and the diarrhea score were calculated.

Diarrhea rate = number of animals with diarrhea/total number of animals in the group $\times$ 100%.

The onset time of diarrhea was defined as the time of first appearance of typical unshaped loose stools.

Duration of diarrhea: The continuous duration during which only loose stools were observed without any normal stools.

Diarrhea Score: The criterion was the condition of stools left on filter paper (size 15.5×26.5cm) at each time point (one time point per hour): no stools (0 point), only normal stools (1 point), loose stools accounted for 1/4 or less of the filter paper area (2 points), loose stools accounted for 1/2 or less of the filter paper area (3 points), and loose stools accounted for more than 1/2 of the filter paper area (4 points).

Evaluation Criteria: The lower the diarrhea rate, the lower the diarrhea score, the later the diarrhea occurred, and the shorter the duration of diarrhea, the more it indicated that the drug had an antidiarrheal effect. On the contrary, it did not have an antidiarrheal effect.

2.7 Notification of relevant staff

The animal department should be notified when animals were purchased. The abnormal event of animals should be reported to the pathology department for processing.

2.8 Instrument system

NVT2201ZH electronic balance (OHAUS)

BT224S precision analytical balance (Sartorius)

NVL2101B electronic balance (OHAUS)

R-300 rotary evaporator (BUCHI, Switzerland)

2.9 Statistical methods

The experimental data were analyzed and processed using statistical software SPSS 20.0. The results were presented as mean $\pm$ standard deviation ($\pm$s). A normality test was performed firstly. For data that followed a normal distribution, means were compared by one-way ANOVA. If the variances were homogeneous, pairwise comparisons were performed using the Least Significant Difference (LSD) method. If the variances were not homogeneous, Dunnett's T3 test was used for pairwise comparisons. For data that did not follow a normal distribution, non-parametric tests were used.

3 Results

As can be seen from Table 20, animals in the normal group had no diarrhea. The diarrhea incidence of animals in the model group was 100%, compared to the normal group, the onset time of diarrhea was significantly earlier, the diarrhea duration was significantly longer, and the diarrhea score was significantly higher (P<0.01). The diarrhea incidence of animals in the medium-dose group of the pharmaceutical composition of the present invention was 91.67%, compared to the model group, the onset time of diarrhea was significantly later (P<0.05), the diarrhea duration was shorter, and the diarrhea score was lower, with no significant difference (P>0.05). The diarrhea incidence of animals in the high-dose group of the pharmaceutical composition of the present invention was 100%, compared to the model group, the onset time of diarrhea was significantly later (P<0.01), the diarrhea duration was shorter, and the diarrhea score was lower, with no significant difference (P>0.05). For both the Huo Xiang Zheng Qi group and the low-dose group of the pharmaceutical composition of the present invention, the diarrhea incidences of animals were 100%, compared to the model group, the intervals between diarrhea episodes were longer, the diarrhea durations were shorter, and the diarrhea scores were lower, with no significant difference (P>0.05).

Table 20 Test results of effect of the pharmaceutical composition of the present invention on the diarrhea incidence in mice caused by *sennae folium* ($\overline{x}\pm$s)

| Group | Number of animals | % Diarrhea incidence (%) | Onset time of diarrhea (s) | Diarrhea duration (s) | Diarrhea score |
|---|---|---|---|---|---|
| Normal group | 12 | 0.00 | 360.00$\pm$0.00 | 0.00$\pm$0.00 | 3.75$\pm$0.75 |
| Model group | 12 | 100.00 | 72.17$\pm$13.73## | 237.83$\pm$43.14## | 9.92$\pm$1.16## |
| Huo Xiang Zheng Qi group | 12 | 100.00 | 83.17$\pm$15.26 | 221.83$\pm$67.77 | 9.33$\pm$2.10 |
| Low-dose group of the pharmaceutical composition | 12 | 100.00 | 85.33$\pm$24.20 | 189.67$\pm$72.63 | 8.67$\pm$2.35 |
| of the present invention Medium-dose group of the pharmaceutical composition of the present invention | 12 | 91.67 | 111.25$\pm$79.14* | 183.75$\pm$97.05 | 7.58$\pm$2.39 |
| High-dose group of the pharmaceutical composition of the present invention | 12 | 100.00 | 96.00$\pm$13.15** | 199.00$\pm$68.52 | 8.50$\pm$1.88 |

Note: ## indicates P<0.01 in the model group compared to the normal group, * indicates P<0.05 and ** indicates P<0.01 in the administration group compared to the model group.

4 Conclusion

Under the conditions of this test, the pharmaceutical composition of the present invention had an antidiarrheal effect on diarrhea of mice induced by gavage of *sennae folium* decoction.

5 Discussion

*Sennae folium* is a mild laxative that acts on the large intestine, causing moderate diarrhea with loose stools that have no abnormal odor. This laxative achieves optimal effects while offering advantages such as low residue and high safety. Therefore, it is often used as one of the experimental methods for evaluating antidiarrheal drugs. Under the conditions of this test, the pharmaceutical composition of the present invention had an antidiarrheal effect on diarrhea of mice induced by gavage of *sennae folium* decoction.

Test Example 5

Test Purpose

The test purpose was to evaluate the antipyretic effect of the pharmaceutical composition of the invention on rats with fever induced by intraperitoneal injection of lipopolysaccharide (LPS)

1 Experimental material

1.1 Test products

1.1.1 Name: The pharmaceutical composition of the present invention (test product number: GLPTN-2108)

1.1.2 Characteristics: The product is a yellowish-brown powder mixed with white inclusion substances and is slightly fragrant.

1.1.3 Functions and Indications: It is mainly used for the syndrome of summer-heat-dampness due to cold and the syndrome of stagnant and jamming dampness-heat due to influenza.

1.1.4 Clinical Dosage and Administration Route: 0.5g crude drug/kg body weight, oral administration.

1.1.5 Content: 4.5 g of crude drug/g drug powder.

1.1.6 Source: Shijiazhuang Yiling Pharmaceutical Co., Ltd. Batch number: 211101.

1.1.7 Storage condition: sealed.

1.1.8 Expiration date: tentatively set until November 09, 2023.

1.2 Positive drugs and main reagents

1.2.1 Positive drug: Ibuprofen granules are useful in relieving mild to moderate pain such as headaches, joint pain, migraines, toothaches, muscle pain, neuralgia, and dysmenorrhea. They are also useful in fever caused by the common cold or influenza. Each sachet contains 0.2 g. It should be dissolved in warm boiled water and taken as follows: 0.5 sachet for children aged 4-8 years, 1 sachet for children aged ≥8 years and adults. If pain or fever persists, the dose may be repeated every 4-6 h, with no more than 4 doses within 24 h. Manufacturer: CSPC Ouyi Pharmaceutical Co., Ltd. Batch number: 363211001. Production date: 2021.10.22. Expiration date: 2023.10.21.

1.2.2 Main reagents

1.2.2.1 Sterile water for injection: Shijiazhuang No. 4 Pharmaceutical Co., Ltd. Batch Number: 2009053201. Production date: 2022.09.05. Expiration date: 2022.09.04.

1.2.2.2 LPS: Solarbio. Batch NO. 820F036. Expiration date: 2024.11.27.

1.2.2.3 Physiological saline: Shijiazhuang No. 4 Pharmaceutical Co., Ltd. Batch Number: 2102283202. Production date: 2021.02.28. Expiration date: 2023.02.27.

1.3 Experimental system

1.3.1 Animal Strain: SD rats.

1.3.2 Animal Grade: SPF grade.

1.3.3 Animal Gender and Quantity: A total of 100 male animals were purchased. These animals were divided into two batches of 50 animals each, from which 40 animals with a moderate basal body temperature were selected for the formal test.

1.3.4 Animal Age: Approximately 5 weeks old at the time of receipt.

1.3.5 Animal Weight: The intended weight range for purchased animals was 130-150 g. The actual weight range at the time of receipt was 133.0-154.5 g, and the weight range after breeding was 194.2-248.1 g when administered.

1.3.6 Animal Source: The animals were purchased from Beijing Vital River Laboratory Animal Technology Co., Ltd. Certificate numbers: 110011220100035061. License number: SCXK (Beijing) 2021-0011. Receipt date: January 5, 2022.

1.3.7 Feeding Conditions: Rats were reared in cages at the Innovative Drug Evaluation Center of Hebei Research Institute of Integrated Traditional Chinese and Western Medicine. License Number: SYXK (Hebei) 2020-006. Lighting: 12 h/day. Temperature: 20-26°C. Relative Humidity: 40%-70%.

1.3.8 Quarantine Process: Animals were subjected to adaptive feeding for 5 days, during which body weight was measured daily. During this period, the animals had normal water and food intake, and good health status, with no signs of illness or mortality.

1.3.9 Feed: Rat & mice maintenance feed (SPF-grade) was provided by Beijing Keao Xieli Feed Co., Ltd. Feed Production License Number: Beijing Feed Certificate (2018) 06073, Batch Number: 21103213. Certificate Number: 1112622100055093. Production Date: 2021.10.11. Shelf Life: 6 months. Experimental Animal Production License Number: SCXK (Beijing) 2019-0003.

1.3.10 Water: Clean water for drinking was prepared using the ROB-B drinking water system for laboratory animal, and filled in water bottles for free drinking. The drinking water was detected annually for all indicators, including mainly microbiological indicators, toxicological indicators, sensory characteristics, and general chemical parameters, commissioned to a qualified institution for detection. Microbial limit detection was performed every 2 months, commissioned to the Quality Control Department of Shijiazhuang Yiling Pharmaceutical Co., Ltd. The detection reports were stored by the animal department and archived annually.

1.3.11 Bedding: Corn cob bedding (common grade) provided by Beijing Keao Xieli Feed Co., Ltd. The bedding was sterilized by high temperature and high pressure before use. Batch Number: 21109811. Production Date: 2021.10.12. Shelf Life: 6 months. Experimental Animal Production License Number: SCXK (Beijing) 2020-0010.

1.3.12 Marking: Rats were marked with the animal marking pen (Manufacturer: EPS, Japan). Product number: AM15.

2 Test methods

2.1 Test design basis

2.1.1 Adopted standards were determined according to the *Registration, Classification and Submission Data Requirements for Chinese Traditional Medicine and Natural Drugs* issued by the State Food and Drug Administration, *Good Laboratory Practice for non-clinical laboratory studies* and related literatures.

2.1.2 Description of selected experimental system: Lipopolysaccharide (LPS) is the active component of endotoxin (ET) found in Gram-negative bacteria. It induces fever primarily by activating inflammatory cells (mostly monocytes and macrophages) to produce endogenous pyrogen (EP). The LPS-induced fever rats model is widely used for evaluating the antipyretic effects of traditional Chinese medicines. Since pharmacological doses of estrogen have anti-inflammatory and immunosuppressive effects, male animals are generally selected for these studies.

### 2.2 Dosage and Grouping

Rats were randomly grouped into 5 groups according to their basal body temperatures, including the model group, ibuprofen group, and low-, medium-, and high- dose groups of the pharmaceutical composition of the present invention. Based on that the clinical dose of the pharmaceutical composition of the present invention was 0.5 g of crude drug/kg body weight, the medium-dose of the pharmaceutical composition of the present invention for rats was set as 8 times the clinical dose, i.e., 4.00 g of crude drug/kg, which was considered as the clinically equivalent amount. The dose interval was set at 2 times, and therefore the low- and high- doses of the pharmaceutical composition of the present invention for rats were 2.00 and 8.00 g of crude drug/kg, respectively. The content of the pharmaceutical composition of the present invention in drug powder was 4.5 g of crude drug/g drug powder. The low-, medium- and high- doses of the pharmaceutical composition of the present invention for rats were 0.44, 0.89 and 1.78 g of drug powder/kg, respectively. Using the ibuprofen specification, the daily clinical dose of ibuprofen for human was set at 200 mg. Calculated on the basis of 60 kg/human, the clinical dose was 3.33 mg/kg. The equivalent dose for rats was set at 8 times the clinical dose, i.e. 26.64 mg/kg. See Table 21 for details.

Table 21 Grouping and dose setting in test studying the effect of the pharmaceutical composition of the present invention on fever in rats caused by LSP

| Group | Numb er of animals | Dose (g crude drug/kg) | Dose (g drug powder/kg) | Concentration (g drug powder/ml) |
| --- | --- | --- | --- | --- |
| Model group | 16 | - | - | - |
| Ibuprofen group | 16 | - | 26.64 mg/kg | 2.664 mg/ml |
| Low-dose group of the pharmaceutical composition of the present invention | 16 | 2.00 | 0.44 | 0.044 |
| Medium-dose group of the pharmaceutical composition of the present invention | 16 | 4.00 | 0.89 | 0.089 |
| High-dose group of the pharmaceutical composition of the present invention | 16 | 8.00 | 1.78 | 0.178 |

### 2.3 Administration method

The drug was administered via gavage, consistent with the clinical administration route.

### 2.4 Preparation and storage of test product

Approximately 0.8800 g (low dose), 1.7800 g (medium dose), and 3.5600 g (high dose) of the pharmaceutical composition of the present invention were accurately weighed and ground with sterile water for injection, respectively. The volume was adjusted to a final volume of 20 ml. For Ibuprofen granules, one sachet was taken, ground with sterile water for injection, and adjusted to a final volume of 75 ml. All test products were prepared just before use.

### 2.5 Administration of test product

A disposable syringe was used to administer the drug at a dosing volume of 10 ml/kg. Animals in the model group were administered with a corresponding volume of the solvent. A single dose was administered 30 min before modeling.

### 2.6 Steps and observation indicators

After receiving new experimental animals, they were subjected to an adaptive feeding for at least 5 days, followed by rectal temperature measurement and gavage operation adaptation. The insertion depth of the electronic thermometer was controlled at 4 cm. This operation was carried out for at least 3 consecutive days to minimize changes in body temperature due to the operation. During which, the body weight was monitored. Before the formal test, the basal body temperature was measured and recorded as T0. Based on the basal body temperature, rats were screened, grouped and administered. 30 min after the administration, the rats were injected intraperitoneally with LPS (prepared with physiological saline) at 20 $\mu$g/kg and an injection volume of 5 ml/kg. After modeling, the rectal temperature was measured every 1 hour and recorded as Tx within 5 h. $\Delta$T was calculated according to $\Delta$T =Tx - T0.

2.7 Notification of relevant staff

The animal department should be notified when animals were purchased. The abnormal event of animals should be reported to the pathology department for processing.

2.8 Instrument system

BT224S precision analytical balance (Sartorius)

EX225D/AD analytical balance (OHAUS)

BSA2201 electronic balance (Sartorius)

Electronic thermometer (Omron)

2.9 Statistical methods

The experimental data were analyzed and processed using statistical software SPSS 20.0. The results were presented as mean $\pm$ standard deviation ($\pm$s). A normality test was performed firstly. For data that followed a normal distribution, means were compared by one-way ANOVA. If the variances were homogeneous, pairwise comparisons were performed using the Least Significant Difference (LSD) method. If the variances were not homogeneous, Dunnett's T3 test was used for pairwise comparisons. For data that did not follow a normal distribution, non-parametric tests were used.

3 Results

As shown in Table 22, the temperature difference of rats in the model group 1-5 h after modeling by intraperitoneal injection of LPS showed a significant increasing trend, with the differences of 0.39 $\pm$ 0.38 (1h), 0.23 $\pm$ 0.45 (2h), 0.66 $\pm$ 0.32 (3h), 0.89 $\pm$ 0.50 (4h), and 1.45 $\pm$ 0.46 (5h). Compared to the model group, the temperature difference of rats in the ibuprofen (positive drug) group was significantly decreased 3 h after modeling (P < 0.05), with the difference of 0.28 $\pm$ 0.48 (3h); the temperature difference of rats in low-dose group of the pharmaceutical composition of the present invention was significantly decreased 5 h after modeling (P < 0.01), with the difference of 0.91 $\pm$ 0.50 (5h); the temperature difference of rats in medium-dose group of the pharmaceutical composition of the present invention was significantly decreased 5 h after modeling (P < 0.05), with the difference of 1.01 $\pm$ 0.55 (5h); and the temperature difference of rats in high-dose group of the pharmaceutical composition of the present invention was significantly decreased 1 h after modeling (P < 0.05), with the difference of 0.08 $\pm$ 0.36 (1h).

Table 22 Results of test studying effect of the pharmaceutical composition of the present invention on fever in rats caused by LSP ($\bar{x} \pm s$)

| Group | N | $\Delta$T ($\Delta$T =Tx-T$_0$) °C | | | | |
|---|---|---|---|---|---|---|
| | | 1h | 2h | 3h | 4h | 5h |
| Model group | 16 | 0.39$\pm$0.3 8 | 0.23$\pm$0.4 5 | 0.66$\pm$0. 32 | 0.89$\pm$0 .50 | 1.45$\pm$0.4 6 |
| Ibuprofen group | 16 | 0.62$\pm$0.5 2 | 0.11$\pm$0.5 0 | 0.28$\pm$0. 48* | 0.92$\pm$0 .77 | 1.24$\pm$0.6 9 |
| Low-dose group of the pharmaceutical composition of the present invention | 16 | 0.34$\pm$0.3 6 | 0.32$\pm$0.4 6 | 0.75$\pm$0. 51 | 0.82$\pm$0 .61 | 0.91$\pm$0.5 0** |

(continued)

| Group | N | ΔT (ΔT =Tx-T$_0$) °C | | | | |
|---|---|---|---|---|---|---|
| | | 1h | 2h | 3h | 4h | 5h |
| Medium-dose group of the pharmaceutical composition of the present invention | 16 | 0.23±0.4 8 | 0.37±0.5 7 | 0.78±0. 50 | 0.92±0 .51 | 1.01±0.5 5* |
| High-dose group of the pharmaceutical composition of the present invention | 16 | 0.08±0.3 6* | 0.24±0.6 2 | 0.67±0. 44 | 0.90±0 .56 | 1.19±0.5 3 |

Note: * indicates P<0.05, and ** indicates P<0.01 compared to the model group.

4 Conclusion

The pharmaceutical composition of the present invention had the effect of inhibiting the fever in rats caused by intraperitoneal injection of LPS under the conditions of this test.

5 Discussion

Currently, the rat fever model is mainly established by injecting pyrogens into animals to cause fever of the body. Common pyrogens include lipopolysaccharide (LPS), dry yeast, 2,4-dinitrophenol, carrageenan, and the like. Among them, yeast and lipopolysaccharide are both microbial pyrogens, which can activate pyrogen-producing cells to produce and release pyrogenic cytokines. The symptoms observed during the development of fever in the LPS-induced fever animal model are similar to those caused by inflammation due to infections in clinical. Therefore, this model is often used for screening antipyretic drugs. However, the dose range for inducing fever model varies significantly, ranging from 10 to 250 μg/kg, with the commonly used dose being 10, 20, and 100 μg/kg. In the exploration of the optimal dose of LPS for inducing fever in rats, some studies had found that the LPS dose of 20 μg/kg causes a high fever intensity and long duration. Due to the self-limiting and tolerance characteristics of this model, most of the efficacy studies were conducted following preventing the LPS attack after administration. Therefore, in this test, the administration was performed 30 min before intraperitoneal injection of LPS.

Under the conditions of this test, the temperature difference of the rats in the model group showed a significant increasing trend after intraperitoneal injection of LPS for modeling. The temperature difference of rats in high-dose group of the pharmaceutical composition of the present invention was significantly decreased 1 h after modeling. The temperature differences of rats in low- and medium- dose group of the pharmaceutical composition of the present invention was significantly decreased 5 h after modeling. These results showed that the pharmaceutical composition of the present invention had the effect of inhibiting the fever in rats induced by intraperitoneal injection of LPS.

Test Example 6

Test Purpose

The test purpose was to evaluate the effect of the pharmaceutical composition of the invention on pain in mice through their writhing response caused by intraperitoneal injection of 0.7% acetic acid.

    1 Experimental materials

        1.1 Test products

            1.1.1 Name: The pharmaceutical composition of the present invention (test product number: GLPTN-2108)

            1.1.2 Characteristics: The product is a yellowish-brown powder mixed with white inclusion substances and is slightly fragrant.

            1.1.3 Functions and Indications: It is mainly used for the syndrome of summer-heat-dampness due to cold and the syndrome of stagnant and jamming dampness-heat due to influenza.

            1.1.4 Clinical Dosage and Administration Route: 0.5g crude drug/kg body weight, oral administration.

1.1.5 Content: 4.5 g of crude drug/g drug powder.

1.1.6 Source: Shijiazhuang Yiling Pharmaceutical Co., Ltd. Batch number: 211101.

1.1.7 Storage condition: sealed.

1.1.8 Expiration date: tentatively set until November 09, 2023.

1.2 Positive drugs and major reagents

1.2.1 Positive drug: Indomethacin capsules. The main active ingredient of this product is indomethacin, which is useful in: (1) relieving pain and swell of arthritis, (2) soft tissue injuries and inflammation, (3) reducing fever, and (4) treating migraines, dysmenorrhea, postoperative pain, post-traumatic pain and the like (other effects). Administration route: Oral administration. Dosage for adult is as follows. (1) For rheumatism: 25-50 mg as an initial dose, 2-3 times a day, the maximum daily dose not exceeding 150 mg. (2) For pain, 25-50 mg (first dose), 25 mg (second dose), 3 times a day, drug can be discontinued until pain is relieved. (3) For fever, 6.25-12.5 mg, no more than 3 times a day. The dosage for children is 1.5-2.5 mg/kg body weight in 3-4 doses daily, and the dosage is reduced to the minimum dosage after drug acting. Manufacturer: Hebei Yongfeng Pharmaceutical Co., Ltd. Batch Number: 07420020143. Production date: 2020.02.29. Expiration Date: January 2023.

1.2.2 Main reagents

1.2.2.1 Sterile water for injection: Shijiazhuang No. 4 Pharmaceutical Co., Ltd. Batch Number: 2009053201. Production date: 2022.09.05. Expiration date: 2022.09.04.

1.2.2.2 Glacial acetic acid (acetic acid) provided by Tianjin Huihang Chemical Science and Technology Co. Ltd. Batch number: September 11, 2021. Production date: 2021.09.11. Expiration date: 2024.09.10.

1.3 Experimental system

1.3.1 Animal Strain: ICR mice.

1.3.2 Animal Grade: SPF grade.

1.3.3 Animal Gender and Quantity: A total of 70 animals were purchased, including 35 males and 35 females. All 60 animals (30 males and 30 females) were used in the test.

1.3.4 Animal Age: Approximately 3-4 weeks old at the time of receipt.

1.3.5 Animal Weight: The intended weight range for animals to be purchased was 18-22 g. The actual weight range of animals at the time of receipt was 17.8-22.6 g, and the weight range of animals after breeding was 22.7-30.0 g when administered.

1.3.6 Animal Source: The animals were purchased from Beijing Vital River Laboratory Animal Technology Co., Ltd. Certificate numbers: 110011220101108146, 110011220101108068. License number: SCXK (Beijing) 2021-0006. Receipt date: February 9, 2022.

1.3.7 Feeding Conditions: Mice were reared in cages at the Innovative Drug Evaluation Center of Hebei Research Institute of Integrated Traditional Chinese and Western Medicine. License Number: SYXK (Hebei) 2020-006. Lighting: 12 h/day. Temperature: 20-26°C. Relative Humidity: 40%-70%.

1.3.8 Quarantine Process: Animals were subjected to adaptive feeding for 5 days, during which body weight was measured daily. During this period, the animals had normal water and food intake, and good health status, with no signs of illness or mortality.

1.3.9 Feed: Rat & mice maintenance feed (SPF-grade) was provided by Beijing Keao Xieli Feed Co., Ltd. Feed Production License Number: Beijing Feed Certificate (2018) 06073. Batch Number: 21113213.

Certificate Number: 1112622200078056. Production Date: 2021.11.01. Shelf Life: 6 months. Experimental Animal Production License Number: SCXK (Beijing) 2019-0003.

1.3.10 Water: Clean water was prepared using the ROB-B drinking water system for laboratory animal, and filled in water bottles for free drinking. The drinking water was detected annually for all indicators, including mainly microbiological indicators, toxicological indicators, sensory characteristics, and general chemical parameters, commissioned to a qualified institution for detection. Microbial limit detection was performed every 2 months, commissioned to the Quality Control Department of Shijiazhuang Yiling Pharmaceutical Co., Ltd. The detection reports were stored by the animal department and archived annually.

1.3.11 Bedding: Corn cob bedding (common grade) provided by Beijing Keao Xieli Feed Co., Ltd. The bedding was sterilized by high temperature and high pressure before use. Batch Number: 22019811. Production Date: 2022.01.04. Shelf Life: 6 months. Experimental Animal Production License Number: SCXK (Beijing) 2020-0010.

1.3.12 Marking: Mice were marked with the animal marking pen (EPS, Japan). Product number: AM15.

2 Test methods

2.1 Test design basis

2.1.1 Adopted standards were determined according to the *Registration, Classification and Submission Data Requirements for Chinese Traditional Medicine and Natural Drugs* issued by the State Food and Drug Administration, *Good Laboratory Practice for non-clinical laboratory studies, Experimental Methodology of Pharmacology* (3rd edition) and related literatures.

2.1.2 Description of selected experimental system: The basic principle of this experiment is to inject a certain volume and concentration of a chemical irritant (acetic acid) into the abdominal cavity of mice to stimulate the visceral and parietal peritoneum, leading to a deep, widespread, and long-time pain. The mice have a writhing response on pain, manifested as behaviors such as abdominal retraction, stretching of the trunk and hind limbs, and arching of the buttocks. The writhing response occurs frequently within 15 min after the injection. Therefore, the number of writhing incidents or the number of mice showing this response within 15 min after injection is used as a quantitative indicator of pain.

2.2 Dosage and Grouping

60 ICR mice were randomly grouped into 5 groups according to their body weight, including the model group, the indomethacin group, and the low-, the medium-, and the high-dose groups of the pharmaceutical composition of the present invention. Based on that the clinical dose of the pharmaceutical composition of the present invention was 0.5 g of crude drug/kg body weight, the medium-dose of the pharmaceutical composition of the present invention for mice was set as 10 times the clinical dose, i.e., 5.00 g of crude drug/kg, which was considered as the clinically equivalent amount. The dose interval was set at 2 times, and therefore the low- and high- doses of the pharmaceutical composition of the present invention for mice were 2.50 and 10.00 g of crude drug/kg, respectively. The content of the pharmaceutical composition of the present invention in drug powder was 4.5 g of crude drug/g drug powder. The low-, medium- and high-doses of the pharmaceutical composition of the present invention were 0.56, 1.11 and 2.22 g of drug powder/kg, respectively. Referring to the previous studies in our laboratory and relevant literatures, the dose of indomethacin drug administered to mice was set at 5.00 mg/kg, as shown in Table 23.

Table 23 Grouping and dose setting in test studying effect of the pharmaceutical composition of the present invention on writhing in mice caused by acetic acid

| Group | Number of animals | Dose (g crude drug/kg) | Dose (g drug powder/kg) | Concentration (g drug powder/ml) |
|---|---|---|---|---|
| Model group | 12 | - | - | - |
| Indomethacin group | 12 | - | 5.00 mg/kg | 0.500 mg/ml |
| Low-dose group of the pharmaceutical composition of the present invention | 12 | 2.50 | 0.56 | 0.056 |

(continued)

| Group | Number of animals | Dose (g crude drug/kg) | Dose (g drug powder/kg) | Concentration (g drug powder/ml) |
|---|---|---|---|---|
| Medium-dose group of the pharmaceutical composition of the present invention | 12 | 5.00 | 1.11 | 0.111 |
| High-dose group of the pharmaceutical composition of the present invention | 12 | 10.00 | 2.22 | 0.222 |

2.3 Administration method

The drug was administered via gavage, consistent with the clinical administration route.

2.4 Preparation and storage of test product

Approximately 0.5600 g (low dose), 1.1100 g (medium dose), and 2.2200 g (high dose) of the pharmaceutical composition of the present invention were accurately weighed and ground with sterile water for injection, respectively. The volume was adjusted to a final volume of 10 ml. For indomethacin capsules, one capsule was taken, ground with sterile water for injection, and adjusted to a final volume of 50 ml. All test products were prepared just before use.

2.5 Administration of test product

A disposable syringe was used to administer the drug at a dosing volume of 10 ml/kg. Animals in the model group were administered with a corresponding volume of the solvent. The administration was carried out for 5 days.

2.6 Steps and observation indicators

After receiving new experimental animals, they were numbered, subjected to an adaptive feeding for 5 days and grouped as described above. All animals were fasted for more than 12 h before the last administration, and 1 h after the last administration, 0.7% acetic acid was injected into the abdominal cavity of mice at a dose of 10ml/kg to stimulate the visceral and parietal peritoneum, leading to a deep, widespread, and long-time pain. As a result, the mice had a writhing response on pain, manifested as behaviors such as abdominal retraction, stretching of the trunk and hind limbs, and arching of the buttocks. The latency period to the onset of writhing and the number of writhing incidents within 15 min were recorded as quantitative indicators of pain.
Evaluation Criteria: A decrease in the number of writhing incidents and a prolonging in the latency period indicate that the drug inhibits response to pain. Conversely, it does not inhibit response to pain.

2.7 Notification of relevant staff

The animal department should be notified when animals were purchased. The abnormal event of animals should be reported to the pathology department for processing.

2.8 Instrument system

JJ2000 electronic balance from Changshu Shuangjie Testing Instrument Factory

NVT2201ZH electronic balance (OHAUS)

BT224S precision analytical balance (Sartorius)

Research plus (Eppendorf)

2.9 Statistical methods

The experimental data were analyzed and processed using statistical software SPSS 20.0. The results were presented as mean ± standard deviation (±s). A normality test was performed firstly. For data that followed a normal distribution, means were compared by one-way ANOVA. If the variances were homogeneous, pairwise comparisons were performed using the Least Significant Difference (LSD) method. If the variances were not homogeneous, Dunnett's T3 test was used for pairwise comparisons. For data that did not follow a normal distribution, non-parametric tests were used.

3 Results

As shown in Table 24, for mice in the model group, the latency period to the onset of writhing was 377.83±208.68 seconds and the number of writhing incidents with 15 min was 14.92±10.56, respectively. Compared to the model group, the latency period to the onset of writhing was significantly prolonged (P<0.01) and the number of writhing incidents was significantly decreased (P<0.01) in the indomethacin group; the latency period to the onset of writhing was significantly prolonged (P<0.05) in the low- and medium- dose groups of the pharmaceutical composition of the present invention; and the latency period to the onset of writhing was significantly prolonged (P<0.01) and the number of writhing incidents was significantly decreased (P<0.01) in the high-dose group of the pharmaceutical composition of the present invention. The numbers of writhing incidents were decreased in the low- and medium-dose groups of the pharmaceutical composition of the present invention, but there was no significant difference (P>0.05).

Table 24 Results of test studying effect of the pharmaceutical composition of the present invention on writhing in mice caused by acetic acid ($\overline{x}$ ± s)

| Group | Number of animals | Latency period (s) | Number of writhing incident |
|---|---|---|---|
| Model group | 12 | 377.83±208.68 | 14.92±10.56 |
| Indomethacin group | 12 | 736.83±258.16** | 2.17±4.53** |
| Low-dose group of the pharmaceutical composition of the present invention | 12 | 620.17±286.38* | 8.08±12.85 |
| Medium-dose group of the pharmaceutical composition of the present invention | 12 | 636.50±326.45* | 8.08±13.08 |
| High-dose group of the pharmaceutical composition of the present invention | 12 | 728.33±254.76** | 1.83±4.30** |

Note: * indicates P<0.05, and ** indicates P<0.01 compared to the model group.

4 Conclusion

Under the conditions of this test, the pharmaceutical composition of the present invention can significantly inhibit the response to pain in mice caused by stimulating the peritoneum with acetic acid.

5 Discussion

The basic principle of this experiment is to inject a certain volume and concentration of acetic acid into the abdominal cavity of mice. This stimulates the visceral and parietal peritoneum, leading to a deep, widespread, and long-time inflammatory pain. The mice have a writhing response on pain, manifested as behaviors such as abdominal retraction, stretching of the trunk and hind limbs, and arching of the buttocks. The writhing response occurs frequently within 15 min after injection. Therefore, the writhing response occurred within 15 min after injection was used as a quantitative indicator of pain. The model is susceptible to the influence of acetic acid concentration and ambient temperature. After literature review, the writhing response was obvious in the conditions of intraperitoneal injection of 0.7% acetic acid and the ambient temperature controlled between 20-26°C. Under the conditions of this test, the pharmaceutical composition of the present invention significantly prolonged the latency period to the onset of writhing, decreased the number of writhing times, and inhibited the response to pain caused by stimulating the peritoneum with acetic acid in mice.

Test Example 7

Test Purpose

The ear-swelling model of mice was established by applying xylene as a chemical proinflammatory substance to one side of the ear. The degree of swelling was detected by measuring the weight difference between the two ears to evaluate the effect of the pharmaceutical composition of the present invention on xylene-induced inflammation.

1 Experimental materials

1.1 Test products

1.1.1 Name: The pharmaceutical composition of the present invention (test product number: GLPTN-2108)

1.1.2 Characteristics: The product is a yellowish-brown powder mixed with white inclusion substances and is slightly fragrant.

1.1.3 Functions and Indications: It is mainly used for the syndrome of summer-heat-dampness due to cold and the syndrome of stagnant and jamming dampness-heat due to influenza.

1.1.4 Clinical Dosage and Administration Route: 0.5g crude drug/kg body weight, oral administration.

1.1.5 Content: 4.5 g of crude drug/g drug powder.

1.1.6 Source: Shijiazhuang Yiling Pharmaceutical Co., Ltd. Batch number: 211101.

1.1.7 Storage condition: sealed.

1.1.8 Expiration date: tentatively set until November 09, 2023.

1.2 Positive drugs and major reagents

1.2.1 The positive drug was aspirin enteric-coated tablets. The main ingredient of this product is aspirin, which can be used for chronic and acute inflammation, such as rheumatoid arthritis, osteoarthritis, and ankylosing spondylitis. Administration and Dosage: Oral administration, 4-8 g a day. Manufacturer: Bayer S.P.A. Batch Number: BJ55889. Production Date: 2020.07.23. Expiration date: 2023.07.22.

1.2.2 Main reagents

1.2.2.1 Sterile water for injection: Shijiazhuang No. 4 Pharmaceutical Co., Ltd. Batch Number: 2009053201. Production date: 2020.09.05. Expiration date: 2022.09.04.

1.2.2.2 Xylene: Tianjin Oubokai Chemical and Industrial Co. Ltd. Batch number: August 3, 2021. Production date: 2021.08.03. Expiration date: 2022.08.02.

1.3 Experimental system

1.3.1 Animal Strain: ICR mice.

1.3.2 Animal Grade: SPF grade.

1.3.3 Animal Gender and Quantity: A total of 70 animals were purchased, including 35 males and 35 females. 60 animals (30 males and 30 females) were used in the test.

1.3.4 Animal Age: Approximately 3-4 weeks old at the time of receipt.

1.3.5 Animal Weight: The intended weight range for animals to be purchased was 18-22 g. The actual weight range of animals at the time of receipt was 19.1-23.2 g, and the weight range of animals after breeding was 23.3-31.6 g when administered.

1.3.6 Animal Source: The animals were purchased from Beijing Vital River Laboratory Animal Technology Co., Ltd. Certificate numbers: 110011220102050423, 110011220102050373. License number: SCXK (Beijing) 2021-0006. Receipt date: March 2, 2022.

1.3.7 Feeding Conditions: Mice were reared in cages at the Innovative Drug Evaluation Center of Hebei Research Institute of Integrated Traditional Chinese and Western Medicine. License Number: SYXK (Hebei) 2020-006. Lighting: 12 h/day. Temperature: 20-26°C. Relative Humidity: 40%-70%.

1.3.8 Quarantine Process: Animals were subjected to adaptive feeding for 5 days, during which body weight was measured daily. During this period, the animals had normal water and food intake, and good health status, with no signs of illness or mortality.

1.3.9 Feed: Rat & mice maintenance feed (SPF-grade) was provided by Beijing Keao Xieli Feed Co., Ltd. Feed Production License Number: Beijing Feed Certificate (2018) 06073. Batch Number: 21113213. Certificate Number: 1112622200078056. Production Date: 2021.11.01. Shelf Life: 6 months. Experimental Animal Production License Number: SCXK (Beijing) 2019-0003.

1.3.10 Water: Drinking clean water was prepared using the ROB-B drinking water system for laboratory animal, and filled in water bottles for free drinking. The drinking water was detected annually for all indicators, including mainly microbiological indicators, toxicological indicators, sensory characteristics, and general chemical parameters, commissioned to a qualified institution for detection. Microbial limit detection was performed every 2 months, commissioned to the Quality Control Department of Shijiazhuang Yiling Pharmaceutical Co., Ltd. The detection reports were stored by the animal department and archived annually.

1.3.11 Bedding: Corn cob bedding (common grade) provided by Beijing Keao Xieli Feed Co., Ltd. The bedding was sterilized by high temperature and high pressure before use. Batch Number: 22019811. Production Date: 2022.01.04. Shelf Life: 6 months. Experimental Animal Production License Number: SCXK (Beijing) 2020-0010.

1.3.12 Marking: Mice were marked with the animal marking pen (EPS, Japan). Product number: AM15.

2 Test methods

2.1 Test design basis

2.1.1 Adopted standards were determined according to the *Registration, Classification and Submission Data Requirements for Chinese Traditional Medicine and Natural Drugs* issued by the State Food and Drug Administration, *Good Laboratory Practice for non-clinical laboratory studies, Experimental Methodology of Pharmacology* (3rd edition) and related literatures.

2.1.2 Description of selected experimental system: Acute inflammation models commonly use proinflammatory agents such as xylene or croton oil. The xylene is a chemical reagent that, when applied topically or injected into localized areas of an animal (such as the ear or toes), can cause local capillary dilation and increased permeability, leading to inflammatory infiltration. Therefore, it is often used as one of the routine experimental methods for evaluating anti-inflammatory drugs.

2.2 Dosage and Grouping

60 ICR mice were randomly grouped into 5 groups according to their body weight, including the model group, the aspirin group, and the low-, the medium-, and the high- dose groups of the pharmaceutical composition of the present invention. Based on that the clinical dose of the pharmaceutical composition of the present invention was 0.5 g of crude drug/kg body weight, the medium-dose of the pharmaceutical composition of the present invention for mice was set as 10 times the clinical dose, i.e., 5.00 g of crude drug/kg, which was considered as the clinically equivalent amount. The dose interval was set at 2 times, and therefore the low- and high- doses of the pharmaceutical composition of the present invention for mice were 2.50 and 10.00 g of crude drug/kg, respectively. The content of the pharmaceutical composition of the present invention in drug powder was 4.5 g of crude drug/g drug powder. The low-, medium- and high- dose of the pharmaceutical composition of the present invention required 0.56, 1.11 and 2.22 g of drug powder/kg, respectively. Referring to the specification and literature documents, the dose of aspirin drug administered to mice was set at 0.50 g/kg, as shown in Table 25.

Table 25 Grouping and dose setting in test studying effect of the pharmaceutical composition of the present invention on ear-swelling in mice caused by xylene

| Group | Number of animals | Dose (g crude drug/kg) | Dose (g drug powder/kg) | Concentration (g drug powder/ml) |
|---|---|---|---|---|
| Model group | 12 | - | - | - |
| Aspirin group | 12 | - | 0.50g/kg | 0.050g/ml |
| Low-dose group of the pharmaceutical composition of the present invention | 12 | 2.50 | 0.56 | 0.056 |
| Medium-dose group of the pharmaceutical composition of the present invention | 12 | 5.00 | 1.11 | 0.111 |
| High-dose group of the pharmaceutical composition of the present invention | 12 | 10.00 | 2.22 | 0.222 |

2.3 Administration method

The drug was administered via gavage, consistent with the proposed clinical administration route.

2.4 Preparation and storage of test product

Approximately 0.5600 g (low dose), 1.1100 g (medium dose), and 2.2200 g (high dose) of the pharmaceutical composition of the present invention were accurately weighed and ground with sterile water for injection, respectively. The volume was adjusted to a final volume of 10 ml. For aspirin enteric-coated tablets, 5 tablets were taken, ground with sterile water for injection, and adjusted to a final volume of 10 ml. All test products were prepared just before use.

2.5 Administration of test product

A disposable syringe was used to administer the drug at a dosing volume of 10 ml/kg. Animals in the model group were administered with a corresponding volume of the solvent. The administration was carried out for 5 days.

2.6 Steps and observation indicators

After receiving new experimental animals, they were numbered and subjected to an adaptive feeding for at least 5 days. The grouping was carried out as described previously. 0.5 h after the last administration, the animal was fixed, and the right ear was exposed. The xylene was applied to the middle area of the right ear of the mice by dropwise addition using a pipette, with each 15 μL of xylene applied to dorsal and ventral sides and spread evenly. The left ear was left untreated. After 40 min, the animal was sacrificed by cervical dislocation. Both ears were then cut along the base of the auricle. A punch (7 mm diameter) was used to obtain symmetrical ear tissues from both ears, which were weighed on a precision analytical balance. The weights were recorded, and the degree of swelling and the swelling inhibition rate were calculated.

$$\text{Swelling degree} = \text{weight of the right ear tissue} - \text{weight of the left ear tissue}$$

Swelling inhibition rate = (average swelling degree of the model group - average swelling degree of the administration group)/average swelling degree of the model group×100%.

Evaluation criteria: A decrease in swelling degree and an increase in swelling inhibition rate indicated that the drug has an anti-inflammatory effect; conversely, it did not have an anti-inflammatory effect.

2.7 Notification of relevant staff

The animal department should be notified when animals were purchased. The abnormal event of animals should be reported to the pathology department for processing.

2.8 Instrument system

NVT2201ZH electronic balance (OHAUS)

AL204 precision analytical balance (Mettler)

BT224S precision analytical balance (Sartorius)

Research plus (eppendorf)

Punch (Shandong Heze Precision Instrument Co. Ltd.)

2.9 Statistical methods

The experimental data were analyzed and processed using statistical software SPSS 20.0. The results were presented as mean $\pm$ standard deviation ($\pm$s). A normality test was performed firstly. For data that followed a normal distribution, means were compared by one-way ANOVA. If the variances were homogeneous, pairwise comparisons were performed using the Least Significant Difference (LSD) method. If the variances were not homogeneous, Dunnett's T3 test was used for pairwise comparisons. For data that did not follow a normal distribution, non-parametric tests were used.

3 Results

As shown in Table 26, the right ear of the animals in the model group showed inflammation after applying xylene, and the ear-swelling degree of the ear was 11.14$\pm$3.60 mg. Compared to the model group, the ear-swelling degree of the mice in the aspirin group was significantly reduced (P<0.01), and the swelling inhibition rate was 50.34%. Compared to the model group, the ear-swelling degrees of the mice in the low-, medium-, and high- dose groups of the pharmaceutical composition of the present invention were significantly reduced (P<0.01), and the swelling inhibition rates were 38.89%, 35.90% and 38.82%, respectively.

Table 26 Results of test studying effect of the pharmaceutical composition of the present invention on ear-swelling in mice caused by xylene ($\bar{x}\pm$s)

| Group | Number of animals | Swelling degree (mg) | Swelling inhibition rate (%) |
|---|---|---|---|
| Model group | 12 | 11.14$\pm$3.60 | - |
| Aspirin group | 12 | 5.53$\pm$2.81** | 50.34 |
| Low-dose group of the pharmaceutical composition of the present invention | 12 | 6.81$\pm$2.52** | 38.89 |
| Medium-dose group of the pharmaceutical composition of the present invention | 12 | 7.14$\pm$2.13** | 35.90 |
| High-dose group of the pharmaceutical composition of the present invention | 12 | 6.82$\pm$3.80** | 38.82 |

Note: ** indicates P<0.01 compared to the model group.

4 Conclusion

Under the present test conditions, the pharmaceutical composition of the present invention significantly inhibited xylene-induced ear swelling in mice, and had an anti-xylene-induced local inflammatory effect.

5 Discussion

Xylene, as a chemical proinflammatory agent, is often selected for modeling due to its ease of operation in pharmacological tests to evaluate whether a drug has an anti-inflammatory effect. Literature has reported that the ear-swelling degree of mice is closely related to the action duration of xylene, and the optimal action duration is about 40 min. In this test, the action duration of xylene was selected to be 40 min. Under the conditions of this test, the ear-swelling degrees of mice in the low-, medium- and high- dose groups of the pharmaceutical composition of the present invention were significantly reduced, and the swelling inhibition rate was 38.89%, 35.90% and 38.82%, respectively, indicating that the pharmaceutical composition of the present invention has a significant reliving effect on the inflammation caused by xylene.

Test Example 8

This study systematically evaluated the clinical efficacy of the pharmaceutical composition of the present invention in treating patients with the cold identified as the pattern of wind attacking the exterior and dampness obstruction in the middle jiao. A randomized controlled trial was conducted, enrolling 90 patients with cold diagnosed with this syndrome pattern. Patients were randomly grouped into two groups: the prescription group of the pharmaceutical composition of the present invention (the traditional Chinese medicine composition of the present invention was given) and the blank control group (general symptomatic treatment was given). The average course of treatment was approximately 5-7 days. The measurements of the following observation indicators were used to evaluate the clinical efficacy and safety of the pharmaceutical composition of the present invention, including the clinical cure rate/clinical time-to-cure (cure is defined as the disappearance of all major symptoms), the effective rate (a decrease rate of ≥50% in the total symptom score for the cold), the disappearance rate of single-item symptom, and the effective rate on traditional Chinese medicine syndrome.

The results showed that for patients treated with the pharmaceutical composition of the present invention, the clinical cure rate was significantly higher compared to the control group, and the median clinical time-to-cure was 3 days, significantly shorter than the 5 days observed in the control group. For the effective rate on symptoms of cold and the effective rate on traditional Chinese medicine syndrome, the prescription group of the pharmaceutical composition of the present invention showed a better trend compared to the control group. For the disappearance rate of single symptom (such as fever with aversion to wind, nasal congestion and runny nose, headache and dizziness, nausea and vomiting, diarrhea, limb fatigue and heaviness, abdominal distention, low food intake and poor appetite, dry mouth, and thirst), the prescription group of the pharmaceutical composition of the present invention showed good therapeutic trends compared to the control group. During the study period, the use of the prescription of the pharmaceutical composition of the present invention was found to be safe, with no adverse reactions. These above clinical observation results showed that the pharmaceutical composition of the present invention had good efficacy and good safety in treating patients with cold identified as the pattern of wind attacking the exterior and dampness obstruction in the middle jiao.

Typical case 1: Jia (female), 58 years old, consulted in July 2021, with the chief complaint of fever with aversion to wind for 6 days and occasional cough, accompanied by headache and dizziness, limb fatigue and heaviness, general fatigue, low food intake and poor appetite, a yellow and greasy tongue coating, a floating, slippery, and rapid pulse. The patient was administered with the original prescription of the pharmaceutical composition of the present invention, one dose daily. After taking 3 doses, the patient had good spirits, no fever, significant improvement in symptoms such as a heavy-headed sensation, limb fatigue and heaviness, general fatigue, low food intake and poor appetite. There were no symptoms of nausea and vomiting, abdominal pain, and diarrhea. The patient continued with 2 doses, after which all symptoms were resolved.

Typical case 2: Yang (female), 76 years old, consulted in April 2021, with the chief complaint of fever for 2 days and worsening for 1 day. The symptoms included poor spirit, fever with aversion to wind, cough, and occasional expectoration, accompanied by diarrhea, nasal congestion and runny nose, abdominal distension, low food intake and poor appetite, dry mouth, light red tongue with a white greasy coating, and a slippery and rapid pulse. The patient was administered with the original prescription of the pharmaceutical composition of the present invention, one dose daily. After taking 5 doses, the patient had improved spirits, occasional cough and expectoration, no fever, no nausea and vomiting, no nasal congestion and runny nose, improved abdominal distension, improved low food intake and poor appetite, normal stool and urine. The patient continued with 2 doses, after which the patient had good spirits, disappearance of cough and expectoration, no fever, no nasal congestion and runny nose, no abdominal distension, no low food intake and poor appetite, normal stool and urine, light red tongue with a white greasy coating, and a slippery pulse.

The above is only preferred examples of the present invention, and is not intended to limit the present invention. Any modifications, equivalent substitutions and improvements made within the spirit and principles of the present invention shall be included in the scope of protection of the present invention.

**Claims**

1.   A traditional Chinese medicine composition for use in the treatment of a cold, comprising the following components in parts by weight: 230 to 340 parts of *Perillae folium,* 230 to 340 parts of *Pogostemonis cablin,* 230 to 340 parts of *Moslae herba,* 230 to 340 parts of *Magnoliae officinalis cortex,* 230 to 340 parts of *Scutellariae radix,* 170 to 260 parts of *Pinelliae rhizoma praeparatum cum alumine,* 285 to 430 parts of *Lonicerae flos,* 230 to 340 parts of *Forsythiae fructus,* 230 to 340 parts of *Poria,* 230 to 340 parts of *Citri reticulatae pericarpium,* and 115 to 170 parts of *Zingiberis rhizoma recens.*

2. The traditional Chinese medicine composition according to claim 1, wherein the traditional Chinese medicine composition comprises the following components in parts by weight: 230 parts of *Perillae folium,* 340 parts of *Pogostemonis cablin,* 230 parts of *Moslae herba,* 340 parts of *Magnoliae officinalis cortex,* 230 parts of *Scutellariae radix,* 260 parts of *Pinelliae rhizoma praeparatum cum alumine,* 285 parts of *Lonicerae flos,* 340 parts of *Forsythiae fructus,* 230 parts of *Poria,* 340 parts of *Citri reticulatae pericarpium,* and 115 parts of *Zingiberis rhizoma recens.*

3. The traditional Chinese medicine composition according to claim 1, wherein the traditional Chinese medicine composition comprises the following components in parts by weight: 340 parts of *Perillae folium,* 230 parts of *Pogostemonis cablin,* 340 parts of *Moslae herba,* 230 parts of *Magnoliae officinalis cortex,* 340 parts of *Scutellariae radix,* 170 parts of *Pinelliae rhizoma praeparatum cum alumine,* 430 parts of *Lonicerae flos,* 230 parts of *Forsythiae fructus,* 340 parts of *Poria,* 230 parts of *Citri reticulatae pericarpium,* and 170 parts of *Zingiberis rhizoma recens.*

4. The traditional Chinese medicine composition according to claim 1, wherein the traditional Chinese medicine composition comprises the following components in parts by weight: 300 parts of *Perillae folium,* 300 parts of *Pogostemonis cablin,* 300 parts of *Moslae herba,* 300 parts of *Magnoliae officinalis cortex,* 300 parts of *Scutellariae radix,* 230 parts of *Pinelliae rhizoma praeparatum cum alumine,* 380 parts of *Lonicerae flos,* 300 parts of *Forsythiae fructus,* 300 parts of *Poria,* 300 parts of *Citri reticulatae pericarpium,* and 150 parts of *Zingiberis rhizoma recens.*

5. The traditional Chinese medicine composition according to claim 1, wherein the traditional Chinese medicine composition comprises the following components in parts by weight: 286 parts of *Perillae folium,* 286 parts of *Pogostemonis cablin,* 286 parts of *Moslae herba,* 286 parts of *Magnoliae officinalis cortex,* 286 parts of *Scutellariae radix,* 214 parts of *Pinelliae rhizoma praeparatum cum alumine,* 357 parts of *Lonicerae flos,* 286 parts of *Forsythiae fructus,* 286 parts of *Poria,* 286 parts of *Citri reticulatae pericarpium,* and 143 parts of *Zingiberis rhizoma recens.*

6. The traditional Chinese medicine composition according to claim 1, wherein the traditional Chinese medicine composition comprises the following components in parts by weight: 250 parts of *Perillae folium,* 250 parts of *Pogostemonis cablin,* 250 parts of *Moslae herba,* 250 parts of *Magnoliae officinalis cortex,* 250 parts of *Scutellariae radix,* 200 parts of *Pinelliae rhizoma praeparatum cum alumine,* 320 parts of *Lonicerae flos,* 250 parts of *Forsythiae fructus,* 250 parts of *Poria,* 250 parts of *Citri reticulatae pericarpium,* and 125 parts of *Zingiberis rhizoma recens.*

7. Use of the traditional Chinese medicine composition according to any one of claims 1 to 6 in the manufacture of a medicament for treating a cold, wherein the cold is influenza, common cold or gastrointestinal type cold.

8. The traditional Chinese medicine composition according to claim 7, wherein the gastrointestinal type cold is manifested as fever with aversion to wind, headache and dizziness, nausea and vomiting, diarrhea, nasal congestion and runny nose, limb fatigue and heaviness, abdominal distention and pain, or low food intake and poor appetite.

9. Use of the traditional Chinese medicine composition according to any one of claims 1 to 6 in the manufacture of a medicament against H3N2, IBV, RSV, CVB3, EV71, HCoV-229E, PIV or RV virus.

10. Use of the traditional Chinese medicine composition according to any one of claims 1 to 6 in the manufacture of a medicament for inhibiting replication of coxsackievirus B3 in large intestine tissues, regulating balance of immune cells, and reducing inflammation reaction of local tissues.

11. Use of the traditional Chinese medicine composition according to any one of claims 1 to 6 in the manufacture of a medicament for reducing degree of colonic lesions, increasing ratio of lymphocytes CD4+/CD8+ in blood, decreasing level of TNF-$\alpha$ or elevating level of IFN-$\gamma$ in large intestine tissues of model animals

12. Use of the traditional Chinese medicine composition according to any one of claims 1 to 6 in the manufacture of a medicament for inhibiting gastric emptying and intestinal motility effect.

13. Use of the traditional Chinese medicine composition according to any one of claims 1 to 6 in the manufacture of a medicament for treating diarrhea.

14. Use of the traditional Chinese medicine composition according to any one of claims 1 to 6 in the manufacture of a medicament for relieving pain.

15. Use of the traditional Chinese medicine composition according to any one of claims 1 to 6 in the manufacture of a medicament against inflammation.

16. A method for producing the traditional Chinese medicine composition according to any one of claims 1 to 6, comprising producing an active ingredient of the traditional Chinese medicine composition by steps of:

(1) weighing *Perillae folium, Pogostemonis cablin, Moslae herba, Citri reticulatae pericarpium*, *and Zingiberis rhizoma recens* according to a prescribed amount, extracting a volatile oil via distillation by water vapor, collecting the volatile oil for later use, and filtering an aqueous solution obtained after the distillation for later use;
(2) weighing *Pogostemonis cablin, Scutellariae radix, Pinelliae rhizoma praeparatum cum alumine, Lonicerae flos, Forsythiae fructus, and Poria* according to a prescribed amount, adding water, extracting, filtering to obtain a filtrate, concentrating the filtrate and the aqueous solution obtained after the distillation of step (1) under reduced pressure, performing spray drying, and collecting spray powder for later use;

wherein the active ingredient of the traditional Chinese medicine composition comprises the volatile oil obtained in step (1) and the spray powder obtained in step (2).

17. A medicament comprising the traditional Chinese medicine composition according to any one of claims 1 to 6 or the traditional Chinese medicine composition produced by the method according to claim 16.

18. The medicament according to claim 17, wherein the medicament is in a dosage form of capsule, tablet, granule, injection, pill, powder, spray or oral liquid.

19. The medicament according to claim 18, wherein the granule is produced by steps of:

(1) weighing *Perillae folium, Pogostemonis cablin, Moslae herba, Citri reticulatae pericarpium, and Zingiberis rhizoma recens* according to a prescribed amount, extracting a volatile oil via distillation by water vapor, collecting the volatile oil for later use, and filtering an aqueous solution obtained after the distillation to obtain a volatile oil extract for later use;
(2) weighing *Pogostemonis cablin, Scutellariae radix, Pinelliae rhizoma praeparatum cum alumine, Lonicerae flos, Forsythiae fructus, and Poria* according to a prescribed amount, adding water, decocting, filtering to obtain a filtrate, combining the filtrate and the volatile oil extract obtained in step (1), concentrating, filtering and drying to obtain dry paste powder for later use;
(3) encapsulating the volatile oil obtained in step (1) in betacyclodextrin to obtain a volatile oil inclusion substance; and
(4) adding an appropriate amount of an excipient to the dry paste powder obtained in step (2) and the volatile oil inclusion substance obtained in step (3) for granulation, and producing the granule according to a conventional method.

20. The medicament according to claim 19, wherein the granule is produced by steps of:

(1) weighing *Perillae folium, Pogostemonis cablin, Moslae herba, Citri reticulatae pericarpium,* and *Zingiberis rhizoma recens* according to a prescribed amount, adding 8 to 12 times the amount of water, extracting a volatile oil via distillation by water vapor for 6 to 10 h, collecting the volatile oil for later use, and filtering an aqueous solution obtained after the distillation to obtain a volatile oil extract for later use;
(2) weighing *Pogostemonis cablin, Scutellariae radix, Pinelliae rhizoma praeparatum cum alumine, Lonicerae flos, Forsythiae fructus, and Poria* according to a prescribed amount, adding 8 to 12 times the amount of water, decocting twice for 1 to 2 h each time, filtering to obtain a filtrate, combining the filtrate and the volatile oil extract obtained in step (1), concentrating under reduced pressure, and performing spray drying to obtain dry paste powder for later use;
(3) encapsulating the volatile oil obtained in step (1) in betacyclodextrin to obtain a volatile oil inclusion substance, drying, and crushing to obtain dry powder for later use; and
(4) subjecting the dry paste powder obtained in step (2) and the dry powder of the volatile oil inclusion substance obtained in step (3) to dry granulation, and producing the granule according to a conventional method.

21. The medicament according to claim 20, wherein the granule is produced by steps of:

(1) weighing *Perillae folium, Pogostemonis cablin, Moslae herba, Citri reticulatae pericarpium,* and *Zingiberis rhizoma recens* according to a prescribed amount, adding 10 times the amount of water, extracting a volatile oil via distillation by water vapor for 8 h, collecting the volatile oil for later use, and filtering an aqueous solution obtained after the distillation to obtain a volatile oil extract for later use;

(2) weighing *Pogostemonis cablin, Scutellariae radix, Pinelliae rhizoma praeparatum cum alumine, Lonicerae flos, Forsythiae fructus, and Poria* according to a prescribed amount, adding 10 times the amount of water, decocting twice for 1.5 h each time, filtering to obtain a filtrate, combining the filtrate and the volatile oil extract obtained in step (1), concentrating under reduced pressure, and performing spray drying to obtain dry paste powder for later use;

(3) encapsulating the volatile oil obtained in step (1) in betacyclodextrin to obtain a volatile oil inclusion substance, drying, and crushing to obtain dry powder for later use; and

(4) subjecting the dry paste powder obtained in step (2) and the dry powder of the volatile oil inclusion substance obtained in step (3) to dry granulation, and producing the granule according to a conventional method.

22. The medicament according to claim 18, wherein the tablet is produced by steps of:

(1) weighing *Perillae folium, Pogostemonis cablin, Moslae herba, Citri reticulatae pericarpium,* and *Zingiberis rhizoma recens* according to a prescribed amount, adding 10 times the amount of water, extracting a volatile oil via distillation by water vapor for 8 h, collecting the volatile oil for later use, and filtering an aqueous solution obtained after the distillation to obtain a volatile oil extract for later use;

(2) weighing *Pogostemonis cablin, Scutellariae radix, Pinelliae rhizoma praeparatum cum alumine, Lonicerae flos, Forsythiae fructus, and Poria* according to a prescribed amount, adding 10 times the amount of water, decocting twice for 1.5 h each time, filtering to obtain a filtrate, combining the filtrate and the volatile oil extract obtained in step (1), concentrating under reduced pressure, and performing spray drying to obtain dry paste powder for later use;

(3) encapsulating the volatile oil obtained in step (1) in betacyclodextrin to obtain a volatile oil inclusion substance, drying, and crushing to obtain dry powder for later use; and

(4) subjecting the dry paste powder obtained in step (2) and the dry powder of the volatile oil inclusion substance obtained in step (3) to dry granulation, and producing the tablet according to a conventional method.

23. The medicament according to claim 18, wherein the capsule is produced by steps of:

(1) weighing *Perillae folium, Pogostemonis cablin, Moslae herba, Citri reticulatae pericarpium,* and *Zingiberis rhizoma recens* according to a prescribed amount, adding 10 times the amount of water, extracting a volatile oil via distillation by water vapor for 8 h, collecting the volatile oil for later use, and filtering an aqueous solution obtained after the distillation to obtain a volatile oil extract for later use;

(2) weighing *Pogostemonis cablin, Scutellariae radix, Pinelliae rhizoma praeparatum cum alumine, Lonicerae flos, Forsythiae fructus, and Poria* according to a prescribed amount, adding 10 times the amount of water, decocting twice for 1.5 h each time, filtering to obtain a filtrate, combining the filtrate and the volatile oil extract obtained in step (1), concentrating under reduced pressure, and performing spray drying to obtain dry paste powder for later use;

(3) encapsulating the volatile oil obtained in step (1) in betacyclodextrin to obtain a volatile oil inclusion substance, drying, and crushing to obtain dry powder for later use; and

(4) subjecting the dry paste powder obtained in step (2) and the dry powder of the volatile oil inclusion substance obtained in step (3) to dry granulation, and producing the capsule according to a conventional method.

24. A method for treating a disease, comprising administering the traditional Chinese medicine composition according to any one of claims 1 to 6, the traditional Chinese medicine composition produced by the method according to claim 16, or the medicament according to any one of claims 17 to 23.

25. The method according to claim 24, wherein the treating includes at least one of treating a cold, inhibiting gastric emptying and intestinal motility, treating diarrhea, relieving pain and/or anti-inflammation effect.

26. The method according to claim 25, wherein the cold includes influenza, common cold or gastrointestinal type cold.

27. The method according to claim 26, wherein the gastrointestinal type cold is manifested as fever with aversion to wind, headache and dizziness, nausea and vomiting, diarrhea, nasal congestion and runny nose, limb fatigue and heaviness, abdominal distention and pain, or low food intake and poor appetite.

28. The method according to claim 25, wherein the treating a cold includes resisting H3N2, IBV, RSV, CVB3, EV71, HCoV-229E, PIV or RV virus.

29. The method according to claim 28, wherein the treating a cold includes at least one of:

inhibiting replication of coxsackievirus B3 in large intestine tissues, regulating balance of immune cells, and reducing inflammation reaction of local tissues; and
reducing degree of colonic lesions, increasing ratio of lymphocytes CD4+/CD8+ in blood, decreasing level of TNF-$\alpha$ or elevating level of IFN-$\gamma$ in large intestine tissues of model animals.

## INTERNATIONAL SEARCH REPORT

| | International application No. |
|---|---|
| | **PCT/CN2024/073799** |

| | |
|---|---|
| **A.** | **CLASSIFICATION OF SUBJECT MATTER** |

A61K36/9068(2006.01)i; A61P37/02(2006.01)i; A61P31/16(2006.01)i; A61P31/14(2006.01)i; A61P31/12(2006.01)i; A61P29/00(2006.01)i; A61P11/02(2006.01)i; A61P11/00(2006.01)i; A61P1/14(2006.01)i; A61P1/12(2006.01)i; A61P1/08(2006.01)i

According to International Patent Classification (IPC) or to both national classification and IPC

| | |
|---|---|
| **B.** | **FIELDS SEARCHED** |

Minimum documentation searched (classification system followed by classification symbols)

IPC: A61K, A61P

Documentation searched other than minimum documentation to the extent that such documents are included in the fields searched

Electronic data base consulted during the international search (name of data base and, where practicable, search terms used)

CNTXT, CNKI, VEN, CJFD, WPABSC, WPABS, ENTXT, ENTXTC, DWPI, E药全库, E MEDICINE LIBRARY, 中国药物专利数据库, CHINESE PHARMACEUTICAL PATENT DATABASE, 百度学术搜索, Baidu Scholar Search, 超星读秀, CHAOXING DUXIU, PUBMED, 百度, Baidu: 紫苏叶, 广藿香, 香薷, 厚朴, 黄芩, 半夏, 山银花, 连翘, 茯苓, 陈皮, 生姜, 感冒, 流感, 发热, 发烧, 高烧, 高烧, 恶风, 头痛, 头疼, 昏沉, 头昏, 头晕, 恶心, 呕吐, 腹泻, 鼻塞, 流涕, 脘腹胀痛, 肢体困重, 食少纳呆, 病毒, 免疫, 炎症, 抗炎, 胃排空, 肠动力, 疼痛, 止痛, perilla+, agastach+, pogostemon+, elsholtzia+, mosla+, magnolia+, scutellaria+, pinellia+, lonicera+, forsythia+, poria+, citri, citrus, tangerine+, zingberis, cold, influenza, fever, wind, headache, dizziness, nausea, emesis, diarrhea, rhinobyon, rhinorrhea, abdominal distension, limb, eat less and stay less, virus, viral, immun+, +inflamma+, gastric emptying, intestinal motility, pain

| | |
|---|---|
| **C.** | **DOCUMENTS CONSIDERED TO BE RELEVANT** |

| Category* | Citation of document, with indication, where appropriate, of the relevant passages | Relevant to claim No. |
|---|---|---|
| A | CN 101138605 A (YIN, Kehua) 12 March 2008 (2008-03-12)<br>description, page 1 | 1-23 |
| A | CN 102008611 A (XU, Rouyun) 13 April 2011 (2011-04-13)<br>claims 1-4 | 1-23 |
| A | 马启杭 (MA, Qihang). "感冒汤治疗感冒156例观察 (Non-official translation: Observation of Cold Decoction Treatment for 156 Patients with Cold)"<br>基层医学论坛 (The Medical Forum), Vol. 9, No. (12), 31 December 2005 (2005-12-31),<br>pages 1118-1119 | 1-23 |

☑ Further documents are listed in the continuation of Box C.  ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **15 March 2024** | **07 April 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/073799** |

**C.    DOCUMENTS CONSIDERED TO BE RELEVANT**

| Category* | Citation of document, with indication, where appropriate, of the relevant passages | Relevant to claim No. |
| --- | --- | --- |
| A | 王玉珠 (WANG, Yuzhu). "高热治验2则 (Non-official translation: 2 Cases of High Fever Treatment)" <br> 甘肃中医 *(Gansu Journal of TCM)*, Vol. 8, No. (6), 31 December 1995 (1995-12-31), pp. 23-24 | 1-23 |
| A | 张参军等 (ZHANG, Canjun et al.). "清气饮子治疗外感夹湿证39例小结 (Non-official translation: Summary of 39 Cases of Treatment of External Dampness Syndrome with Qingqi Drink)" <br> 甘肃中医 *(Gansu Journal of TCM)*, Vol. 16, No. (12), 31 December 2003 (2003-12-31), pp. 12-13 | 1-23 |

## INTERNATIONAL SEARCH REPORT

International application No.

**PCT/CN2024/073799**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **24-29**
   because they relate to subject matter not required to be searched by this Authority, namely:

   Claims 24-29 relate to a method for treatment of the human or animal body by therapy, and thus do not comply with PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/CN2024/073799**

| Patent document cited in search report | | | Publication date (day/month/year) | Patent family member(s) | Publication date (day/month/year) |
|---|---|---|---|---|---|
| CN | 101138605 | A | 12 March 2008 | None | |
| CN | 102008611 | A | 13 April 2011 | None | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310058976